(19) Europäisches Patentamt
European Patent Office
Office européen des brevets

(11) EP 3 112 337 B1

(12) **EUROPEAN PATENT SPECIFICATION**

(45) Date of publication and mention of the grant of the patent:
**03.04.2019 Bulletin 2019/14**

(21) Application number: **15754666.4**

(22) Date of filing: **04.02.2015**

(51) Int Cl.:
***C07C 25/22** (2006.01)*
***C09K 19/30** (2006.01)*
***C09K 19/34** (2006.01)*
***C09K 19/54** (2006.01)*
***C07C 25/24** (2006.01)*
***C09K 19/12** (2006.01)*
***C07D 309/04** (2006.01)*
***C09K 19/32** (2006.01)*
***C09K 19/38** (2006.01)*
***G02F 1/13** (2006.01)*
***C09K 19/04** (2006.01)*

(86) International application number:
**PCT/JP2015/053070**

(87) International publication number:
**WO 2015/129412 (03.09.2015 Gazette 2015/35)**

# (54) LIQUID CRYSTALLINE COMPOUND CONTAINING TETRAFLUOROFLUORENE, LIQUID CRYSTAL COMPOSITION, AND LIQUID CRYSTAL DISPLAY DEVICE

FLÜSSIGKRISTALLVERBINDUNG MIT TETRAFLUORFLUOREN, FLÜSSIGKRISTALLZUSAMMENSETZUNG UND FLÜSSIGKRISTALLANZEIGEVORRICHTUNG

COMPOSÉ CRISTALLIN LIQUIDE COMPRENANT DU TÉTRAFLUOROFLUORÈNE, COMPOSITION DE CRISTAUX LIQUIDES ET DISPOSITIF D'AFFICHAGE À CRISTAUX LIQUIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2014 JP 2014037952**

(43) Date of publication of application:
**04.01.2017 Bulletin 2017/01**

(73) Proprietors:
- **JNC Corporation**
  **Chiyoda-ku**
  **Tokyo 100-8105 (JP)**
- **JNC Petrochemical Corporation**
  **Tokyo 100-0004 (JP)**

(72) Inventors:
- **TANAKA, Hiroyuki**
  **Ichihara-shi**
  **Chiba 290-8551 (JP)**
- **HIRATA, Kenji**
  **Ichihara-shi**
  **Chiba 290-8551 (JP)**
- **YANO, Masakazu**
  **Ichihara-shi**
  **Chiba 290-8551 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 223 210**
**JP-A- H10 236 992**
**JP-A- 2000 178 211**
**JP-A- 2000 297 051**
**JP-A- 2004 529 867**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## Technical Field

The invention relates to a liquid crystal compound, a liquid crystal composition and a liquid crystal display device. More specifically, the invention relates to a compound having a 3,4,5,6-tetrafluoro-9H-fluorene-2,7-diyl skeleton and a negative dielectric anisotropy, a liquid crystal composition containing the compound and having a nematic phase, and a liquid crystal display device including the composition.

## Background Art

The liquid crystal display device is widely utilized for a display of a personal computer, a television and so forth. The device utilizes optical anisotropy, dielectric anisotropy and so forth of the liquid crystal compound. As an operating mode of the liquid crystal display device, such a mode is known as a phase change (PC) mode, a twisted nematic (TN) mode, a super twisted nematic (STN) mode, a bistable twisted nematic (BTN) mode, an electrically controlled birefringence (ECB) mode, an optically compensated bend (OCB) mode, an in-plane switching (IPS) mode, a vertical alignment (VA) mode, a fringe field switching (FFS) mode and a polymer sustained alignment (PSA) mode.

Among the modes, The IPS mode, the FFS mode and the VA mode are known to be able to improve narrowness of a viewing angle, being a disadvantage of the operating modes such as the TN mode and the STN mode. The liquid crystal composition having negative dielectric anisotropy has been mainly used in the liquid crystal display device having the kind of mode. In order to further improve characteristics of the liquid crystal display device, the liquid crystal compound contained in the composition preferably has physical properties described in (1) to (8) below:

(1) high stability to heat, light and so forth,
(2) a high clearing point,
(3) a low minimum temperature of a liquid crystal phase,
(4) small viscosity ($\eta$),
(5) suitable optical anisotropy ($\Delta n$),
(6) large negative dielectric anisotropy ($\Delta\varepsilon$),
(7) a suitable elastic constant ($K_{33}$: bend elastic constant), and
(8) excellent compatibility with other liquid crystal compounds.

An effect of the physical properties of the liquid crystal compound on the characteristics of the device is as described below. A compound having the high stability to heat, light and so forth as described in (1) increases a voltage holding ratio of the device. Therefore, a service life of the device becomes long. A compound having the high clearing point as described in (2) extends a temperature range in which the device can be used. A compound having the low minimum temperature of the liquid crystal phase such as a nematic phase or a smectic phase, particularly the low minimum temperature of the nematic phase as described in (3) also extends the temperature range in which the device can be used. A compound having the small viscosity as described in (4) shortens response time of the device.

A compound having the suitable optical anisotropy as described in (5) improves contrast of the device. According to a design of the device, a compound having a large optical anisotropy or a small optical anisotropy, more specifically the suitable optical anisotropy is required. When the response time is shortened by decreasing a cell gap of the device, a compound having the large optical anisotropy is suitable. A compound having the large negative dielectric anisotropy as described in (6) decreases a threshold voltage of the device. Therefore, a power consumption of the device becomes small.

With regard to (7), a compound having a large elastic constant shortens the response time of the device. A compound having a small elastic constant decreases the threshold voltage of the device. Accordingly, the suitable elastic constant is needed depending on the characteristics to be improved. A compound having the excellent compatibility with other liquid crystal compounds as described in (8) is preferred. The reason is that the physical properties of the composition are adjusted by mixing the compounds having different physical properties. A melting point and compatibility of the compound are known to be correlated, and a compound having a low melting point is required.

A variety of liquid crystal compounds having the large negative dielectric anisotropy have so far been synthesized (for example, Patent literature No. 1). Patent literature No. 1 discloses compounds (S-1) and (S-2) having a 3,4,5,6-tetrafluoro-9H-fluorene-2,7-diyl skeleton and a $-CH_2O-$bonding group. However, the compounds have a high melting point, and the compatibility with other liquid crystal compounds is not high enough.

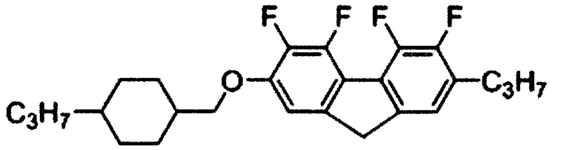

(S-1)

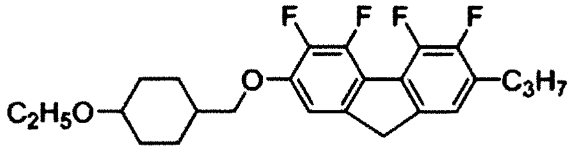

(S-2)

Under such circumstances, a desire has been expressed for developing a compound having excellent physical properties and a suitable balance with regard to the physical properties (1) to (8) above. In particular, a compound having the large negative dielectric anisotropy and being excellent in the compatibility with other compounds has been required.

JP-H10-236992 describes a low viscosity liquid crystal compound having a negative anisotropic value of dielectric constant, optical anisotropy, voltage retention, stability and compatibility and containing a specific structure in a skeleton structure.

WO 02/055463 describes fluorene compounds comprising two fluorine radicals at the fluorene skeleton.

JP 2000 0297051 describes a fluorene compound suitable for a liquid crystal display element, especially for a ferroelectric crystal display element. These compounds comprise oxygen atoms linked to the fluorene ring.

JP 2000 0178211 describes a fluorene-based compound suitable for a ferroelectric liquid crystal display element.

EP 1223210 also describes fluorene compounds which comprise 1 to 3 fluorine atoms at the fluorene skeleton. These compounds have an influence on the dielectric and/or optical anisotropy of the liquid crystal medium.

**Citation List**

**Patent Literature**

Patent literature No. 1: JP H10-236992 A

**Summary of Invention**

**Technical Problem**

A first object of the invention is to provide a liquid crystal compound satisfying at least one of physical properties such as high stability to heat and light, a high clearing point, a low minimum temperature of a liquid crystal phase, small viscosity, suitable optical anisotropy, large negative dielectric anisotropy, a suitable elastic constant and an excellent compatibility with other liquid crystal compounds, in particular, to provide a compound having the large negative dielectric anisotropy and being excellent in compatibility with other liquid crystal compounds. A second object is to provide a composition containing the compound and satisfying at least one of physical properties such as a high maximum temperature of a nematic phase, a low minimum temperature of the nematic phase, small viscosity, suitable optical anisotropy, large negative dielectric anisotropy and a suitable elastic constant. The object is to provide a liquid crystal composition having a suitable balance regarding at least two of the physical properties. A third object is to provide a liquid crystal display device including the composition and satisfying a wide temperature range in which the device can be used, a short response time, a large voltage holding ratio, a low threshold voltage, a large contrast ratio and a long service life.

**Solution to Problem**

The invention relates to a compound represented by formula (1), a liquid crystal composition containing the compound, and a liquid crystal display device including the composition.

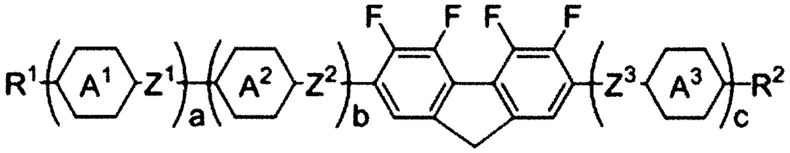

(1)

wherein, in formula (1),

$R^1$ and $R^2$ are independently alkyl having 1 to 15 carbons or alkenyl having 2 to 15 carbons, and in the groups, at least one piece of hydrogen may be replaced by halogen;
ring $A^1$, ring $A^2$ and ring $A^3$ are independently 1, 4-cyclohexylene, 1,4-cyclohexenylene, 1,4-phenylene, 1,4-phenylene in which at least one piece of hydrogen is replaced by halogen, or tetrahydropyran-2,5-diyl;
$Z^1$, $Z^2$ and $Z^3$ are independently a single bond, - $(CH_2)_2$-, -CH=CH-, -C≡C-, -CF=CF-, -$(CH_2)_4$-, -CH=CH-$(CH_2)_2$- or -$(CH_2)_2$-CH=CH-; and
a, b and c are independently 0 or 1, and a sum of a, b and c is 0, 1 or 2.

A compound represented by formula (1) has 3,4,5,6-tetrafluoro-9H-fluorene-2,7-diyl skeleton. Thus, the compound has a large negative dielectric anisotropy. Further, the compound has no group containing an oxygen atom, such as -$CH_2O$- and -$OCH_2$- in the bonding group and no group containing an oxygen atom, such as alkoxy, in a terminal group. Thus, the compound has a low melting point and is excellent in compatibility with other liquid crystal compounds.

## Advantageous Effects of Invention

A first advantage of the invention is to provide a liquid crystal compound satisfying at least one of physical properties such as high stability to heat and light, a high clearing point, a low minimum temperature of a liquid crystal phase, small viscosity, suitable optical anisotropy, large dielectric anisotropy, a suitable elastic constant and excellent compatibility with other liquid crystal compounds. The advantage is particularly to provide a liquid crystal compound having large dielectric anisotropy and excellent compatibility with other liquid crystal compounds. A second advantage is provide a liquid crystal composition containing the compound and satisfying at least one of physical properties such as a high maximum temperature of a nematic phase, a low minimum temperature of the nematic phase, small viscosity, suitable optical anisotropy, large negative dielectric anisotropy and a suitable elastic constant. A third advantage is to provide a liquid crystal display device including the composition and satisfying a wide temperature range in which the device can be used, a short response time, a large voltage holding ratio, a low threshold voltage, a large contrast ratio and a long service life.

## Description of Embodiments

Usage of terms herein is as described below. A liquid crystal compound is a generic term for a compound having a liquid crystal phase such as a nematic phase and a smectic phase, and a compound having no liquid crystal phase but being useful as a component of a liquid crystal composition. "Liquid crystal compound," "liquid crystal composition" and "liquid crystal display device" may be occasionally abbreviated as "compound, " "composition" and "device, " respectively. The Liquid crystal display device is a generic term for a liquid crystal display panel and a liquid crystal display module. A clearing point is a transition temperature between the liquid crystal phase and an isotropic phase in the liquid crystal compound. A minimum temperature of the liquid crystal phase is a transition temperature between a solid and the liquid crystal phase (the smectic phase, the nematic phase or the like) in the liquid crystal compound. A maximum temperature of the nematic phase is a transition temperature between the nematic phase and the isotropic phase in the liquid crystal composition, and may be occasionally abbreviated as "maximum temperature. " A minimum temperature of the nematic phase may be occasionally abbreviated as "minimum temperature." A compound represented by formula (1) may be occasionally abbreviated as "compound (1)." The abbreviation may also applies occasionally to a compound represented by formula (2) or the like. In formulas (1) and (2), a symbol of $A^1$ or $D^1$ surrounded by a hexagonal shape corresponds to ring $A^1$ or ring $D^1$, respectively. A plurality of rings $A^1$ are described as one formula or a different formula. In the compounds, two groups represented by two pieces of arbitrary ring $A^1$ may be identical or different. A same rule also applies to a symbol such as ring $A^2$ and ring $Z^2$. A same rule also applied to ring $A^1$ that becomes two when 1 is 2. An amount of a compound is expressed in terms of weight percent (% by weight) based on the weight of the liquid crystal composition.

An expression "at least one of 'A' may be replaced by 'B'" means that a position of 'A' when the number of 'A' is 1 is arbitrary, and that positions thereof can be selected without restriction when the number of 'A' is 2 or more. An expression "at least one piece of A may be replaced by B, C or D" means a case where at least one piece of A is replaced by B, a case where at least one piece of A is replaced by C, and a case where at least one piece of A is replaced by D, and also a case where a plurality of A are replaced by at least two pieces of B, C and D. For example, alkyl in which at least one piece of -$CH_2$- may be replaced by -O- or -CH=CH- includes alkyl, alkenyl, alkoxy, alkoxyalkyl, alkoxyalkenyl and alkenyloxyalkyl. In addition, a case where replacement of two pieces of successive -$CH_2$-by -O- results in forming -O-O- is not preferred. In the alkyl or the like, a case where replacement of -$CH_2$- of a methyl group (-$CH_2$-H) by -O- results in forming -O-H is not preferred, either.

Then, 2-fluoro-1,4-phenylene means two divalent groups described below. In the chemical formula, fluorine may be leftward (L) or rightward (R). A same rule also applies to an asymmetrical divalent ring such as tetrahydropyran-2,5-diyl.

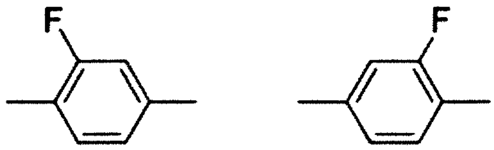

(L) (R)

The invention includes the content described in items 1 to 13 below.

Item 1. A compound represented by formula (1):

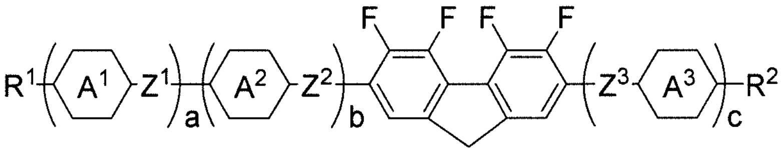

(1)

wherein, in formula (1),

$R^1$ and $R^2$ are independently alkyl having 1 to 15 carbons or alkenyl having 2 to 15 carbons, and in the groups, at least one piece of hydrogen may be replaced by halogen;

ring $A^1$, ring $A^2$ and ring $A^3$ are independently 1, 4-cyclohexylene, 1,4-cyclohexenylene, 1,4-phenylene, 1,4-phenylene in which at least one piece of hydrogen is replaced by halogen, or tetrahydropyran-2,5-diyl;

$Z^1$, $Z^2$ and $Z^3$ are independently a single bond, - $(CH_2)_2$-, -CH=CH-, -C≡C-, -CF=CF-, -$(CH_2)_4$-, -CH=CH-$(CH_2)_2$- or -$(CH_2)_2$-CH=CH-; and

a, b and c are independently 0 or 1, and a sum of a, b and c is 0, 1 or 2.

Item 2. The compound according to item 1, wherein, in the formula (1) described in item 1, $R^1$ and $R^2$ are independently alkyl having 1 to 10 carbons, alkenyl having 2 to 10 carbons, alkyl having 1 to 10 carbons in which at least one piece of hydrogen is replaced by fluorine, or alkenyl having 2 to 10 carbons in which at least one piece of hydrogen is replaced by fluorine, and $Z^1$, $Z^2$ and $Z^3$ are independently a single bond, -$(CH_2)_2$-, -CH=CH-, -$(CH_2)_4$-, -CH=CH-$(CH_2)_2$- or -$(CH_2)_2$-CH=CH-.

Item 3. The compound according to item 1, represented by any one of formulas (1-1) to (1-4):

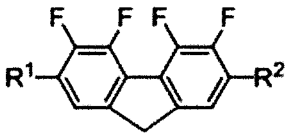

**(1-1)**

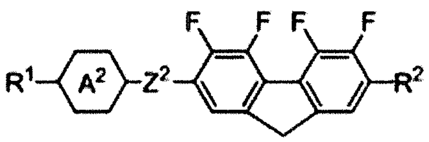

**(1-2)**

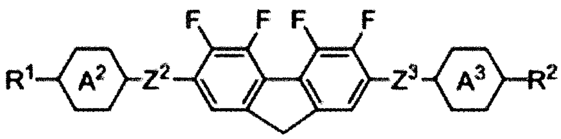

**(1-3)**

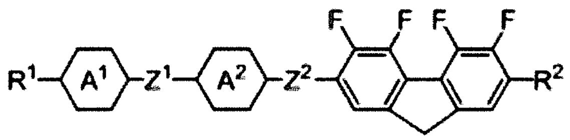

(1-4)

wherein, formulas (1-1) to (1-4),

$R^1$ and $R^2$ are independently alkyl having 1 to 10 carbons, alkenyl having 2 to 10 carbons, or alkyl having 1 to 10 carbons in which at least one piece of hydrogen is replaced by fluorine;
ring $A^1$, ring $A^2$ and ring $A^3$ are independently 1, 4-cyclohexylene, 1,4-cyclohexenylene, 1,4-phenylene, 1,4-phenylene in which at least one piece of hydrogen is replaced by fluorine, or tetrahydropyran-2,5-diyl;
$Z^1$ is a single bond, $-(CH_2)_2-$ or -CH=CH-;
$Z^2$ is a single bond, $-(CH_2)_2-$, $-(CH_2)_4-$ or $-CH=CH-(CH_2)_2-$; and
$Z^3$ is a single bond, $-(CH_2)_2-$, $-(CH_2)_4-$ or $-(CH_2)_2-CH=CH-$.

Item 4. The compound according to item 1, represented by any one of formulas (1-5) to (1-12):

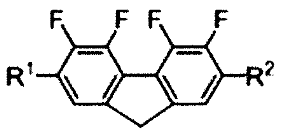

(1-5)

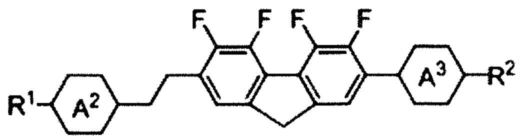

(1-9)

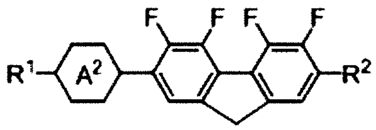

(1-6)

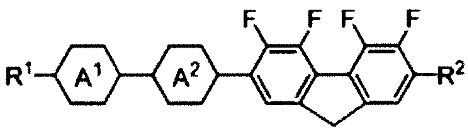

(1-10)

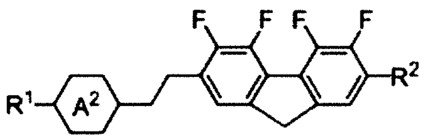

(1-7)

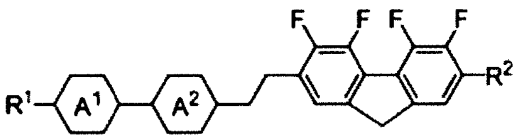

(1-11)

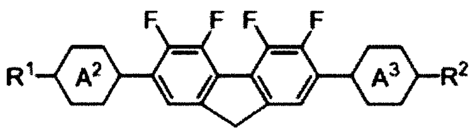

(1-8)

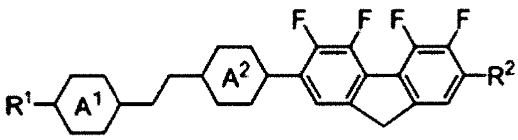

(1-12)

wherein, in formulas (1-5) to (1-12),

$R^1$ and $R^2$ are independently alkyl having 1 to 10 carbons or alkenyl having 2 to 10 carbons; and
ring $A^1$, ring $A^2$ and ring $A^3$ are independently 1, 4-cyclohexylene, 1,4-cyclohexenylene, 1,4-phenylene, 1,4-phenylene in which at least one piece of hydrogen is replaced by fluorine, or tetrahydropyran-2,5-diyl.

Item 5. The compound according to item 1, represented by any one of formulas (1-13) to (1-22):

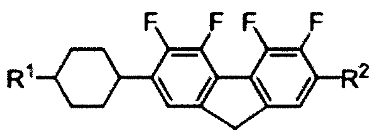

(1-13)

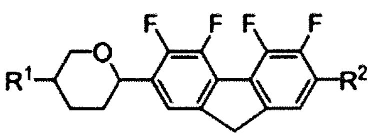

(1-18)

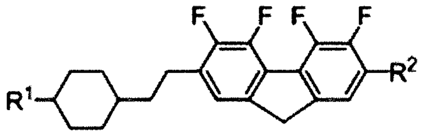
(1-14)

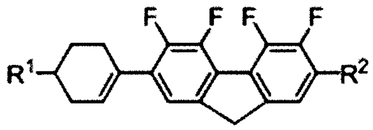
(1-15)

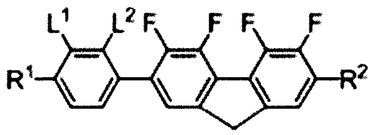
(1-16)

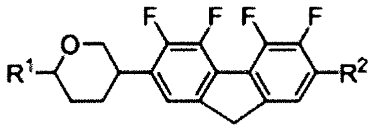
(1-17)

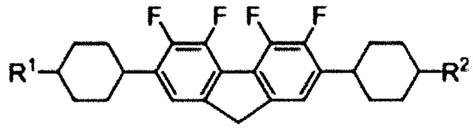
(1-19)

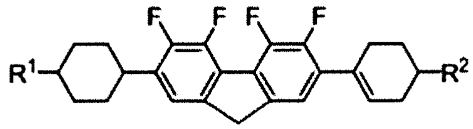
(1-20)

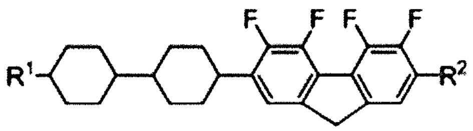
(1-21)

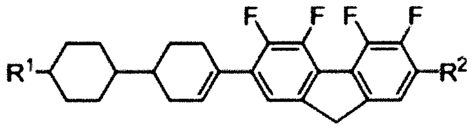
(1-22)

wherein, in formulas (1-13) to (1-22), $R^1$ and $R^2$ are independently alkyl having 1 to 10 carbons or alkenyl having 2 to 10 carbons; and $L^1$ and $L^2$ are independently hydrogen or fluorine.

Item 6. The compound according to item 1, represented by any one of formulas (1-23) to (1-25):

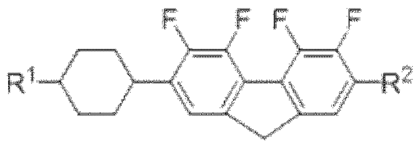
(1-23)

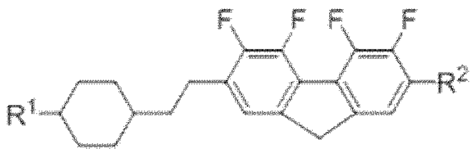
(1-24)

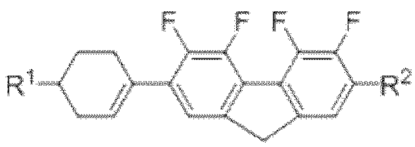
(1-25)

wherein, in formulas (1-23) to (1-25), $R^1$ and $R^2$ are independently alkyl having 1 to 7 carbons or alkenyl having 2 to 7 carbons.

Item 7. A liquid crystal composition, containing at least one of compounds described in item 1.

Item 8. The liquid crystal composition according to item 7, further containing at least one compound selected from the group of compounds represented by formulas (2) to (4):

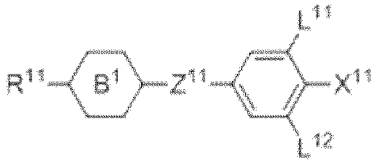
(2)

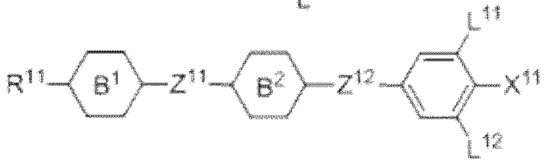
(3)

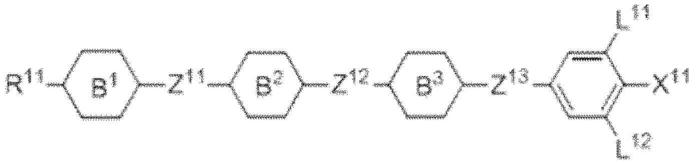
(4)

wherein, in formulas (2) to (4),

$R^{11}$ is alkyl having 1 to 10 carbons or alkenyl having 2 to 10 carbons, and in the alkyl and the alkenyl, at least one piece of hydrogen may be replaced by fluorine, and at least one piece of $-CH_2-$may be replaced by -O-;
$X^{11}$ is fluorine, chlorine, $-OCF_3$, $-OCHF_2$, $-CF_3$, $-CHF_2$, $-CH_2F$, $-OCF_2CHF_2$ or $-OCF_2CHFCF_3$;
ring $B^1$, ring $B^2$ and ring $B^3$ are independently 1, 4-cyclohexylene, 1,4-phenylene, 2-fluoro-1,4-phenylene, 2,5-difluoro-1,4-phenylene, tetrahydropyran-2,5-diyl, 1,3-dioxane-2,5-diyl or pyrimidine-2,5-diyl;
$Z^{11}$, $Z^{12}$ and $Z^{13}$ are independently a single bond, $-CH_2CH_2-$, -CH=CH-, -C≡C-, -COO-, $-CF_2O-$, $-OCF_2-$, $-CH_2O-$ or $-(CH_2)_4-$; and
$L^{11}$ and $L^{12}$ are independently hydrogen or fluorine.

Item 9. The liquid crystal composition according to item 7, further containing at least one compound selected from the group of compounds represented by formula (5):

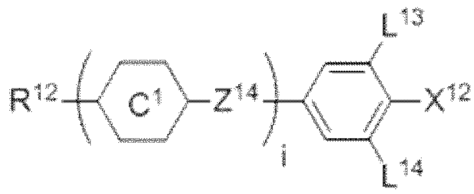
(5)

wherein, in the formula (5),

$R^{12}$ is alkyl having 1 to 10 carbons or alkenyl having 2 to 10 carbons, and in the alkyl and the alkenyl, at least one piece of hydrogen may be replaced by fluorine, and at least one piece of $-CH_2-$may be replaced by -O-;
$X^{12}$ is -C≡N or -C≡C-C≡N;
ring $C^1$ is 1,4-cyclohexylene, 1,4-phenylene, 2-fluoro-1,4-phenylene, 2,5-difluoro-1,4-phenylene, tetrahydropyran-2,5-diyl, 1,3-dioxane-2,5-diyl or pyrimidine-2,5-diyl;
$Z^{14}$ is a single bond, $-CH_2CH_2-$, -C≡C-, -COO-, $-CF_2O-$, $-OCF_2-$ or $-CH_2O-$ ;
$L^{13}$ and $L^{14}$ are independently hydrogen or fluorine; and
i is 1, 2, 3 or 4.

Item 10. The liquid crystal composition according to item 7, further containing at least one compound selected from the group of compounds represented by formulas (6) to (12):

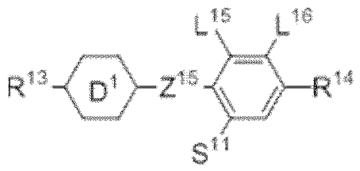

(6)

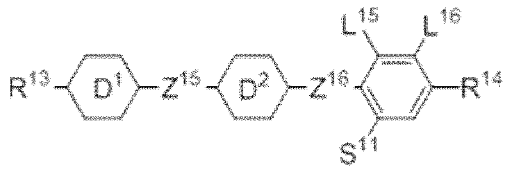

(7)

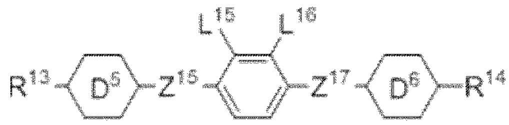

(8)

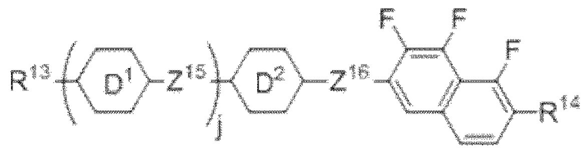

(9)

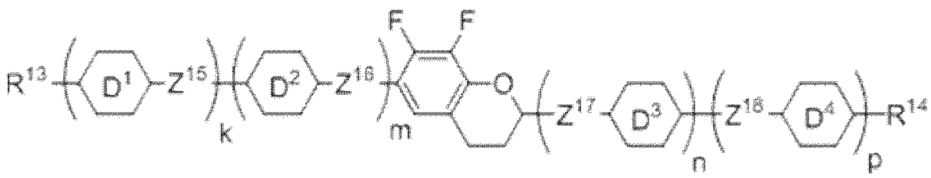

(10)

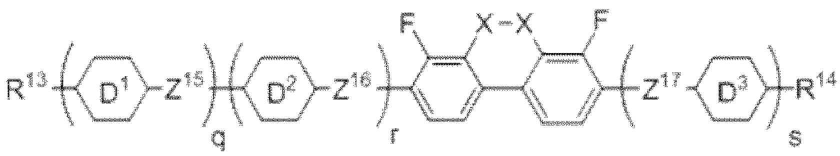

(11)

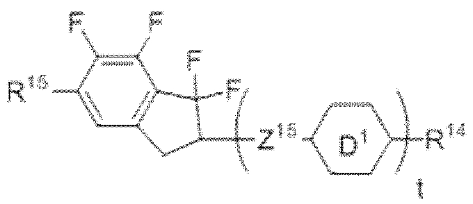

(12)

wherein, in formulas (6) to (12),

$R^{13}$ and $R^{14}$ are independently alkyl having 1 to 10 carbons or alkenyl having 2 to 10 carbons, and in the alkyl and the alkenyl, at least one piece of $-CH_2-$ may be replaced by -O-;

$R^{15}$ is hydrogen, fluorine, alkyl having 1 to 10 carbons or alkenyl having 2 to 10 carbons, and in the alkyl and the alkenyl, at least one piece of $-CH_2-$ may be replaced by -O-, and at least one piece of hydrogen may be replaced by fluorine;

$S^{11}$ is hydrogen or methyl;

X is $-CF_2-$, -O- or -CHF-;

ring $D^1$, ring $D^2$, ring $D^3$ and ring $D^4$ are independently 1,4-cyclohexylene, 1,4-cyclohexenylene, 1,4-phenylene in which at least one piece of hydrogen may be replaced by fluorine, tetrahydropyran-2,5-diyl or decahydronaphthalene-2,6-diyl;

ring $D^5$ and ring $D^6$ are independently 1,4-cyclohexylene, 1,4-cyclohexenylene, 1,4-phenylene, tetrahydropyran-2,5-diyl, or decahydronaphthalene-2,6-diyl;
$Z^{15}$, $Z^{16}$, $Z^{17}$ and $Z^{18}$ are independently a single bond, $-CH_2CH_2-$, -COO-, $-CH_2O-$, $-OCF_2-$ or $-OCF_2CH_2CH_2-$;
$L^{15}$ and $L^{16}$ are independently fluorine or chlorine; and
j, k, m, n, p, q, r and s are independently 0 or 1, a sum of k, m, n and p is 1 or 2, a sum of q, r and s is 0, 1, 2 or 3, and t is 1, 2 or 3.

Item 11. The liquid crystal composition according to item 7, further containing at least one compound selected from the group of compounds represented by formulas (13) to (15):

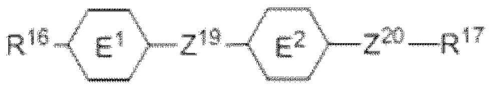
(13)

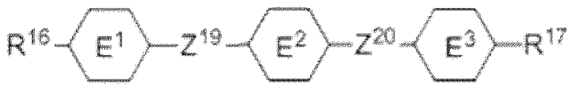
(14)

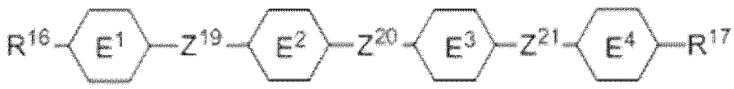
(15)

wherein, in formulas (13) to (15),

$R^{16}$ and $R^{17}$ are independently alkyl having 1 to 10 carbons or alkenyl having 2 to 10 carbons, and in the alkyl or the alkenyl, at least one piece of $-CH_2-$ may be replaced by -O-, and at least one piece of hydrogen may be replaced by fluorine;
ring $E^1$, ring $E^2$, ring $E^3$ and ring $E^4$ are independently 1,4-cyclohexylene, 1,4-phenylene, 2-fluoro-1,4-phenylene, 2,5-difluoro-1,4-phenylene or pyrimidine-2,5-diyl; and
$Z^{19}$, $Z^{20}$ and $Z^{21}$ are independently a single bond, $-CH_2CH_2-$, -CH=CH-, -C≡C- or -COO-.

Item 12. The liquid crystal composition according to item 7, further containing at least one of a polymerizable compound, an optically active compound, an antioxidant, an ultraviolet light absorber, a light stabilizer, a heat stabilizer, and an antifoaming agent.
Item 13. A liquid crystal display device, including the liquid crystal composition described in item 7.
The compound, the liquid crystal composition and the liquid crystal display device according to the invention are described in the order.

1-1. Compound (1)

The compound of the invention will be described. Preferred examples as a terminal group, ring structure and a bonding group in compound (1), and an effect of the groups on physical properties also apply to a compound of a subordinate formula of compound (1).

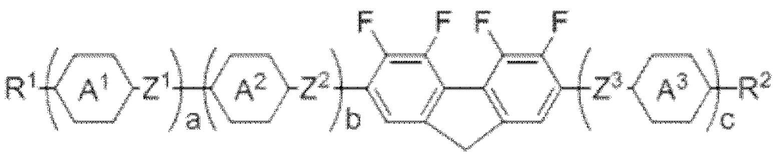
(1)

In formula (1), $R^1$ and $R^2$ are independently alkyl having 1 to 15 carbons or alkenyl having 2 to 15 carbons, and in the groups, at least one piece of hydrogen may be replaced by halogen. The groups have a straight chain or a branched chain, and contain no cyclic group such as cyclohexyl. In the groups, the straight chain is preferred to the branched chain.
A preferred configuration of -CH=CH- in the alkenyl depends on a position of a double bond. In alkenyl having the double bond in an odd-numbered position, such as $-CH=CHCH_3$, $-CH=CHC_2H_5$, $-CH=CHC_3H_7$, $-CH=CHC_4H_9$, $-C_2H_4CH=CHCH_3$ and $-C_2H_4CH=CHC_2H_5$, a trans configuration is preferred. In alkenyl having the double bond in an even-numbered position, such as $-CH_2CH=CHCH_3$, $-CH_2CH=CHC_2H_5$ and $-CH_2CH=CHC_3H_7$, a cis configuration is

preferred. An alkenyl compound having a preferred configuration has a high clearing point or a wide temperature range of the liquid crystal phase. A detailed description is found in Mol. Cryst. Liq. Cryst. , 1985, 131, 109 and Mol. Cryst. Liq. Cryst. , 1985, 131, 327.

Preferred examples of $R^1$ or $R^2$ include alkyl, alkenyl, and alkyl in which at least one piece of hydrogen is replaced by fluorine. Further preferred examples of $R^1$ or $R^2$ include alkyl and alkenyl.

Examples of alkyl include $-CH_3$, $-C_2H_5$, $-C_3H_7$, $-C_4H_9$, $-C_5H_{11}$, $-C_6H_{13}$, $-C_7H_{15}$, $-C_8H_{17}$, $-C_9H_{19}$, $-C_{10}H_{21}$, $-C_{11}H_{23}$, $-C_{12}H_{25}$, $-C_{13}H_{27}$, $-C_{14}H_{29}$ and $-C_{15}H_{31}$.

Examples of alkenyl include $-CH=CH_2$, $-CH=CHCH_3$, $-CH_2CH=CH_2$, $-CH=CHC_2H_5$, $-CH_2CH=CHCH_3$, $-(CH_2)_2-CH=CH_2$, $-CH=CHC_3H_7$, $-CH_2CH=CHC_2H_5$, $-(CH_2)_2-CH=CHCH_3$ and $-(CH_2)_3-CH=CH_2$.

Examples of alkyl in which at least one piece of hydrogen is replaced by halogen include $-CH_2F$, $-CHF_2$, $-CF_3$, $-(CH_2)_2-F$, $-CF_2CH_3$, $-CF_2CH_2F$, $-CF_2CHF_2$, $-CH_2CF_3$, $-CF_2CF_3$, $-(CH_2)_3-F$, $-CF_2CH_2CH_3$, $-CH_2CHFCH_3$, $-CH_2CF_2CH_3$, $-(CF_2)_3-F$, $-CF_2CHFCF_3$, $-CHFCF_2CF_3$, $-(CH_2)_4-F$, $-CF_2(CH_2)_2CH_3$, $-(CF_2)_4-F$, $-(CH_2)_5-F$, $-(CF_2)_5-F$, $-CH_2Cl$, $-CHCl_2$, $-CCl_3$, $-(CH_2)_2-Cl$, $-CCl_2CH_3$, $-CCl_2CH_2Cl$, $-CCl_2CHCl_2$, $-CH_2CCl_3$, $-CCl_2CCl_3$, $-(CH_2)_3-Cl$, $-CCl_2CH_2CH_3$, $-(CCl_2)_3-Cl$, $-CCl_2CHClCCl_3$, $-CHClCCl_2CCl_3$, $-(CH_2)_4-Cl$, $-(CCl_2)_4-Cl$, $-CCl_2(CH_2)_2CH_3$, $-(CH_2)_5-Cl$ and $-(CCl_2)_5-Cl$.

Examples of alkenyl in which at least one piece of hydrogen is replaced by halogen include $-CH=CHF$, $-CH=CF_2$, $-CF=CHF$, $-CH=CHCH_2F$, $-CH=CHCF_3$, $-(CH_2)_2-CH=CF_2$, $-CH_2CH=CHCF_3$, $-CH=CHCF_2CF_3$, $-CH=CHCl$, $-CH=CCl_2$, $-CCl=CHCl$, $-CH=CHCH_2Cl$, $-CH=CHCCl_3$, $-(CH_2)_2-CH=CCl_2$, $-CH_2CH=CHCCl_3$ and $-CH=CHCCl_2CCl_3$.

In formula (1), ring $A^1$, ring $A^2$ and ring $A^3$ are independently 1,4-cyclohexylene, 1,4-cyclohexenylene, 1,4-phenylene, 1,4-phenylene in which at least one piece of hydrogen is replaced by halogen, or tetrahydropyran-2,5-diyl.

Preferred examples of ring $A^1$, $A^2$ or $A^3$ include 1, 4-cyclohexylene, 1,4-cyclohexenylene, 1,4-phenylene, 1,4-phenylene in which at least one piece of hydrogen is replaced by fluorine, or tetrahydropyran-2,5-diyl. Further preferred examples include 1,4-cyclohexylene or 1,4-cyclohexenylene. A configuration of cis and trans exists in 1,4-cyclohexylene. From a viewpoint of a high maximum temperature, the trans configuration is preferred.

Preferred examples of 1, 4-phenylene in which at least one piece of hydrogen is replaced by halogen include rings (A-1) to (A-17). In order to have a large negative dielectric anisotropy, group (A-1), (A-5), (A-6), (A-7), (A-8), (A-9), (A-10) or (A-11) is further preferred.

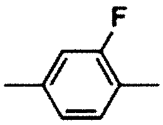
(A-1)

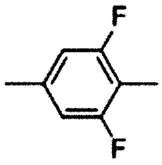
(A-2)

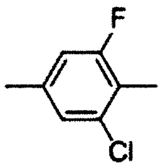
(A-3)

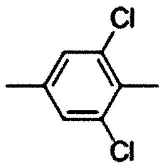
(A-4)

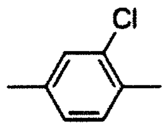
(A-5)

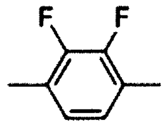
(A-6)

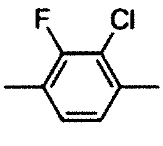
(A-7)

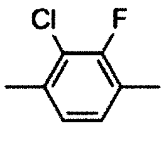
(A-8)

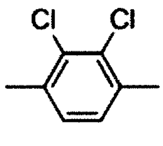
(A-9)

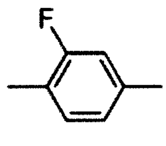
(A-10)

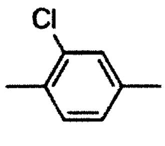
(A-11)

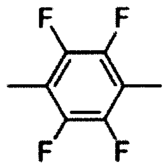
(A-12)

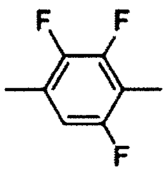
(A-13)

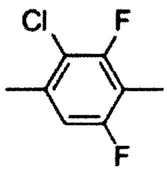
(A-14)

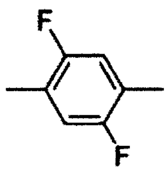
(A-15)

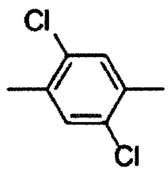
(A-16)

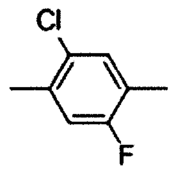
(A-17)

In formula (1), $Z^1$, $Z^2$ and $Z^3$ are independently a single bond, $-(CH_2)_2-$, $-CH=CH-$, $-C\equiv C-$, $-CF=CF-$, $-(CH_2)_4-$, $-CH=CH-(CH_2)_2-$ or $-(CH_2)_2-CH=CH-$. Preferred examples of $Z^1$, $Z^2$ and $Z^3$ include a single bond, $-(CH_2)_2-$, $-CH=CH-$, $-(CH_2)_4-$, $-CH=CH-(CH_2)_2-$ or $-(CH_2)_2-CH=CH-$.

In formula (1), a, b and c are independently 0 or 1, and a sum of a, b and c is 0, 1 or 2. A preferred combination of a, b and c includes combinations (a = b = c = 0), (b = 1, a = c = 0), (b = c = 1, a = 0) or (a = b = 1, c = 0). A further

preferred combination of a, b and c includes a combination (b = 1, a = c = 0) or (b = c = 1, a = 0).

1-2. Physical properties of compound (1)

In compound (1), physical properties such as a clearing point, optical anisotropy and dielectric anisotropy can be arbitrarily adjusted by suitably selecting a kind of $R^1$, $R^2$, ring $A^1$, ring $A^2$, ring $A^3$, $Z^1$, $Z^2$ and $Z^3$, and the combination of a, b and c. Compound (1) may also contain an isotope such as $^2H$ (deuterium) or $^{13}C$ in an amount larger than an amount of natural abundance because no significant difference is in the physical properties of the compound. A main effect of the kind of $R^1$ or the like on the physical properties of compound (1) will be described below.

When $R^1$ or $R^2$ has the straight chain, the temperature range of the liquid crystal phase is wide, and the viscosity is small. When $R^1$ or $R^2$ has the branched chain, the compatibility with other liquid crystal compounds is good. A compound in which $R^1$ or $R^2$ has optical activity is useful as a chiral dopant. A reverse twisted domain to be generated in the liquid crystal display device can be prevented by adding the compound to the composition. A compound in which $R^1$ or $R^2$ has no optical activity is useful as a component of the composition. A preferred configuration when $R^1$ or $R^2$ is alkenyl depends on a position of a double bond. An alkenyl compound having the preferred configuration has small viscosity, the high maximum temperature or the wide temperature range of the liquid crystal phase.

When at least one of ring $A^1$, ring $A^2$ and ring $A^3$ is 2-fluoro-1,4-phenylene, 2-chloro-1,4-phenylene, 2,3-difluoro-1,4-phenylene, 2,3-dichloro-1,4-phenylene, 2-chloro-3-fluoro-1,4-phenylene, or tetrahydropyran-2,5-diyl, such a compound has a particularly large negative dielectric anisotropy.

When the combination of a, b and cis (b = 1, a = c = 0) and ring $A^2$ is 1,4-cyclohexylene or 1,4-cyclohexenylene, the clearing point is high, the viscosity is small and the compatibility other compounds is excellent. When the combination is (b = 1, a = c = 0) and ring $A^2$ is 1,4-phenylene, or 1,4-phenylene in which at least one piece of hydrogen is replaced by halogen, the optical anisotropy is large and also an orientational order parameter is large. When the combination is (b = 1, a = c = 0), and ring $A^2$ is tetrahydropyran-2, 5-diyl, the compatibility with other compounds is excellent. When the combination is (b = c = 1, a = 0) or (a = b = 1, c = 0), and ring $A^1$, ring $A^2$ and ring $A^3$ are 1,4-cyclohexylene, 1,4-cyclohexenylene or a combination thereof, the clearing point is significantly high and the viscosity is comparatively small. When the combination is (b = c = 1, a = 0) or (a = b = 1, c = 0), and ring $A^1$, ring $A^2$ and ring $A^3$ are 1,4-phenylene, 1,4-phenylene in which at least one piece of hydrogen is replaced by halogen or a combination thereof, the clearing point is high and the optical anisotropy is significantly large.

When at least one of $Z^1$, $Z^2$ and $Z^3$ is a single bond, $-CH_2CH_2-$, or -CH=CH-, the viscosity is small. When at least one of $Z^1$, $Z^2$ and $Z^3$ is $-(CH_2)_2-$, -CH=CH-, $-(CH_2)_4-$, $-CH=CH-(CH_2)_2-$ or $-(CH_2)_2-CH=CH-$, an elastic constant (K) is large. When at least one of $Z^1$, $Z^2$ and $Z^3$ is -CH=CH-, -C=C- or -CF=CF-, the optical anisotropy is large. When all of $Z^1$, $Z^2$ and $Z^3$ are a single bond, $-CH_2CH_2-$ or $-(CH_2)_4-$, chemical stability is high.

When the combination of a, band cis (a = b = c = 0), the negative dielectric anisotropy is large. When the combination of a, b and c is (b = 1, a = c = 0), the negative dielectric anisotropy is large the compatibility with other liquid crystal compounds is good. When the combination of a, b and c is (b = c = 1, a = 0), the clearing point is high and the compatibility with other liquid crystal compounds is excellent. When the combination of a, b and c is combination (a = b = 1, c = 0), the clearing point is significantly high.

1-3. Preferred compound

Specific preferred examples of compound (1) include compounds (1-5) to (1-12) described in item 4.

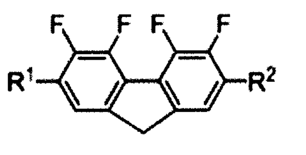
(1-5)

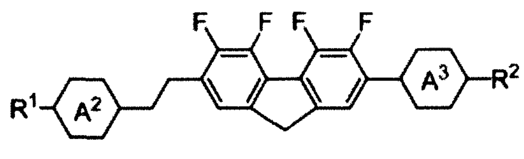
(1-9)

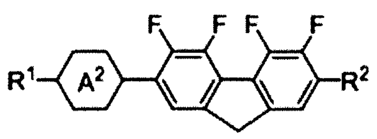
(1-6)

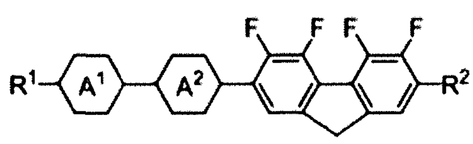
(1-10)

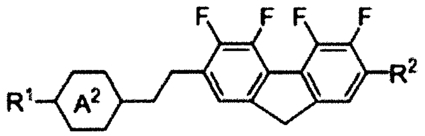
(1-7)

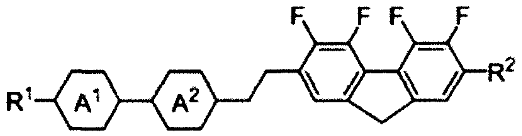
(1-11)

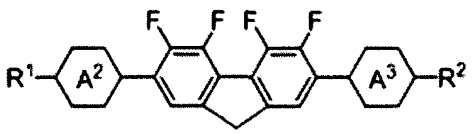
(1-8)

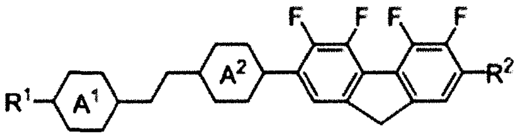
(1-12)

wherein, in formulas (1-5) to (1-12),

$R^1$ and $R^2$ are independently alkyl having 1 to 10 carbons or alkenyl having 2 to 10 carbons;
ring $A^1$, ring $A^2$ and ring $A^3$ are independently 1, 4-cyclohexylene, 1,4-cyclohexenylene, 1,4-phenylene, 1,4-phenylene in which at least one piece of hydrogen is replaced by fluorine, or tetrahydropyran-2,5-diyl.

Specific preferred examples of compound (1) include compounds (1-13) to (1-22) described in item 5.

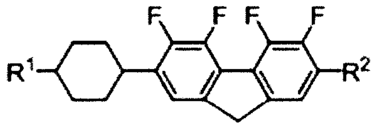
(1-13)

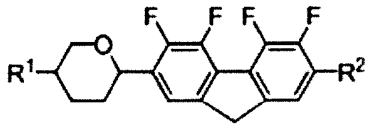
(1-18)

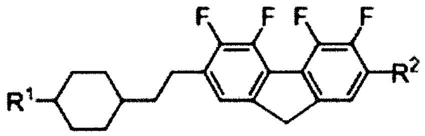
(1-14)

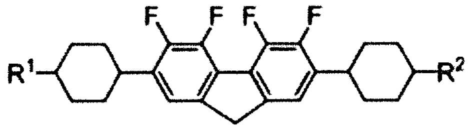
(1-19)

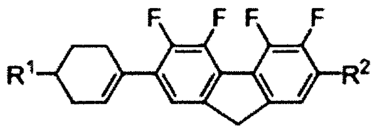
(1-15)

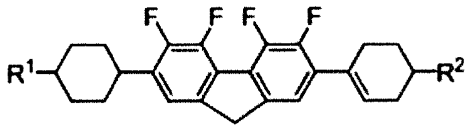
(1-20)

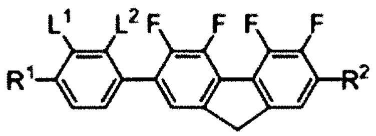
(1-16)

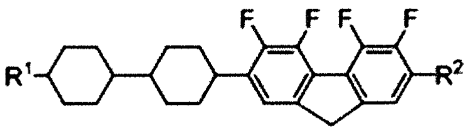
(1-21)

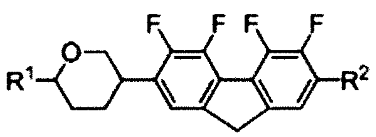
(1-17)

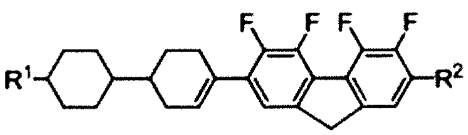
(1-22)

wherein, in formulas (1-13) to (1-22), $R^1$ and $R^2$ are independently alkyl having 1 to 10 carbons or alkenyl having 2 to 10 carbons; and $L^1$ and $L^2$ are independently hydrogen or fluorine.

Specific most preferred examples of compound (1) include compounds (1-23) to (1-25) described in item 6.

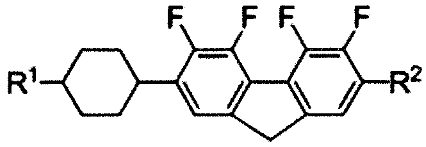

(1-23)

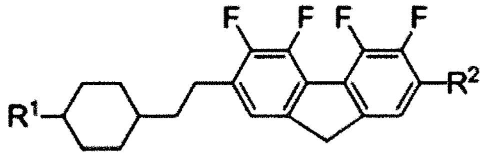

(1-24)

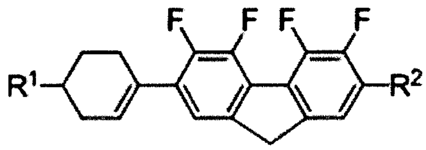

(1-25)

wherein, in formulas (1-23) to (1-25), $R^1$ and $R^2$ are independently alkyl having 1 to 7 carbons or alkenyl having 2 to 7 carbons.

1-4. Synthesis of compound (1)

A synthetic method of compound (1) will be described. Compound (1) can be synthesized by suitably combining methods in synthetic organic chemistry. A method of introducing an objective terminal group, a ring and a bonding group into a starting material is described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

1-4-1. Formation of bonding group

An example of a method of forming a bonding group in compound (1) is as described in a scheme below. In the scheme, $MSG^1$ (or $MSG^2$) is a monovalent organic group having at least one ring. The monovalent organic groups represented by a plurality of $MSG^1$ (or $MSG^2$) may be identical or different. Compounds (1A) to (1G) correspond to compound (1) or an intermediate of compound (1).

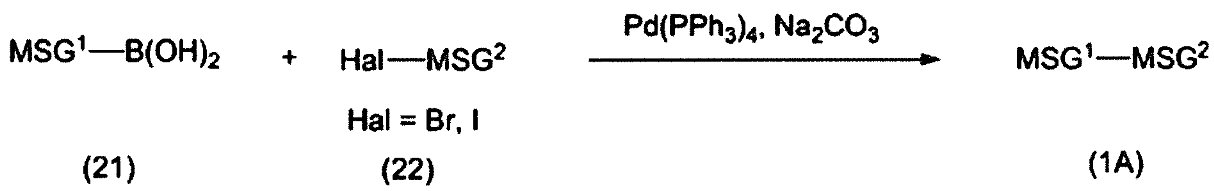


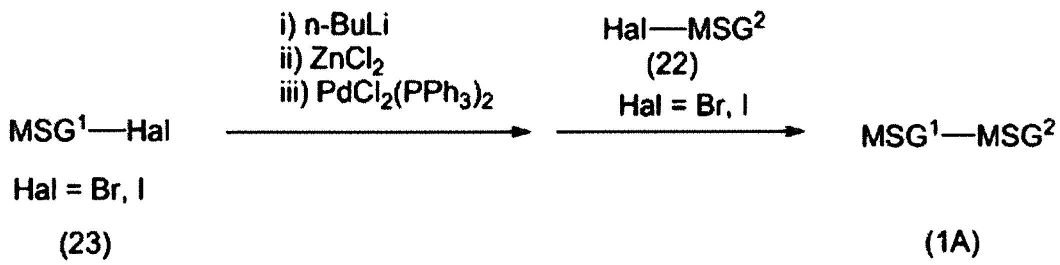

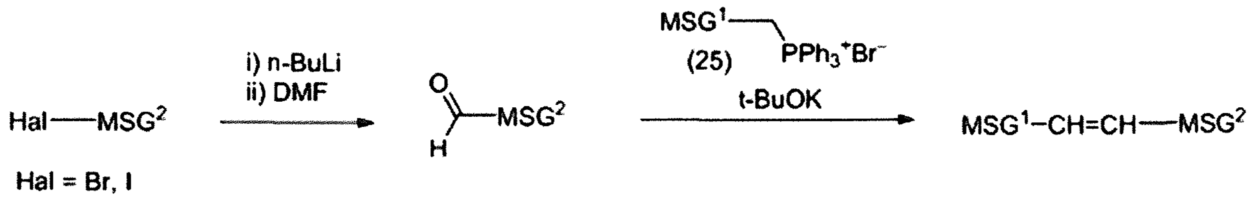


(22) (24) (1B)

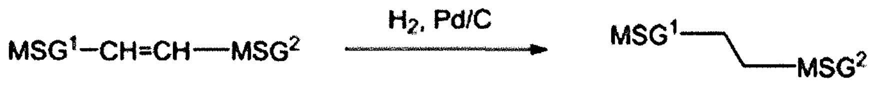


(1B) (1C)

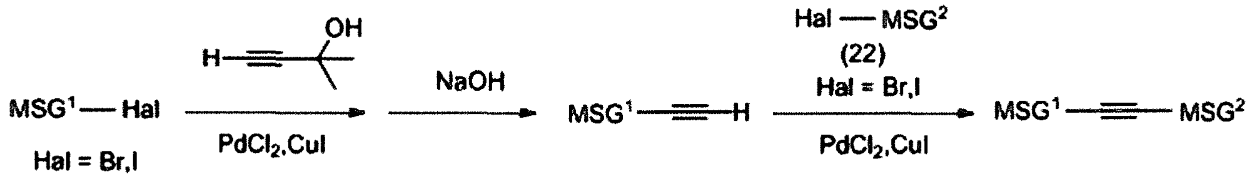


(23) (25) (1D)

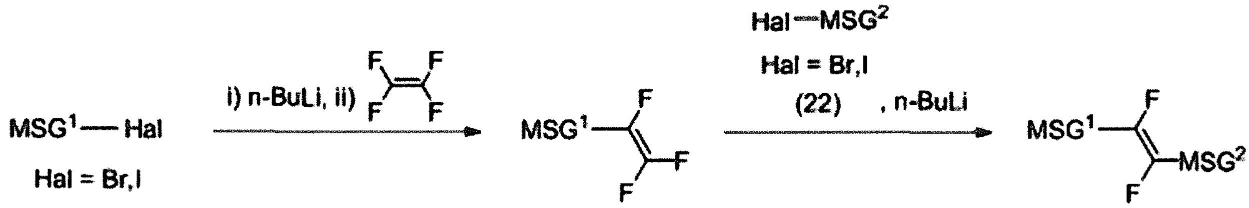


(23) (26) (1E)

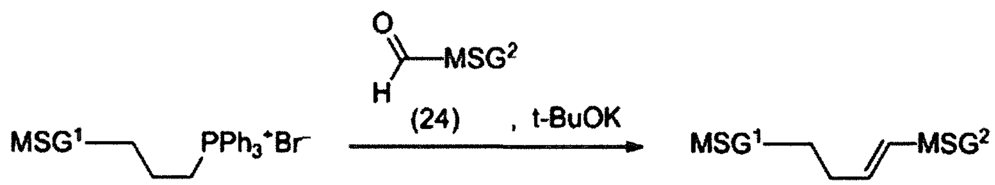


(27) (1F)

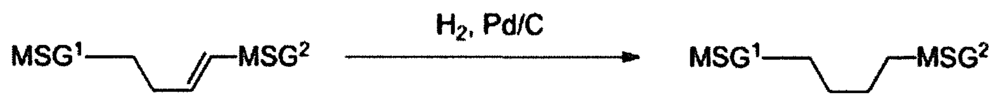


(1F) (1G)

(I) Formation of a single bond

Compound (1A) is prepared by allowing arylboronic acid (21) to react with compound (22) in the presence of carbonate and a tetrakis (triphenylphosphine) palladium catalyst. The compound (1A) can also be prepared by allowing compound (23) to react with n-butyllithium, and subsequently with zinc chloride, and allowing the resulting product to react with compound (22) in the presence of a dichlorobis(triphenylphosphine)palladium catalyst.

(II) -CH=CH- formation

Aldehyde (24) is obtained by allowing compound (22) to react with n-butyllithium, and subsequently with N,N-dimethylformamide (DMF) . Compound (1B) is prepared by allowing aldehyde (24) to react with phosphorus ylide generated by allowing phosphonium salt (25) to react with potassium t-butoxide. A cis isomer is formed depending on reaction conditions, and the cis isomer is isomerized into a trans isomer according to a known method, when necessary.

(III) Formation of $-CH_2CH_2-$

Compound (1C) is prepared by hydrogenating compound (1B) in the presence of a palladium on carbon catalyst.

(IV) Formation of -C=C-

Compound (25) is obtained by allowing compound (23) to react with 2-methyl-3-butyn-2-ol in the presence of a catalyst including dichloropalladium and copper iodide, and performing deprotection under basic conditions. Compound (1D) is prepared by allowing compound (25) to react with compound (22) in the presence of a catalyst of dichlorobis(triphenylphosphine)palladium and copper halide.

(V) Formation of -CF=CF-

Compound (26) is obtained by treating compound (23) with n-butyllithium and then allowing the treated compound to react with tetrafluoroethylene. Compound (1E) is prepared by treating compound (22) with n-butyllithium and then allowing the resulting compound to react with compound (26).

(VI) Formation of $-(CH_2)_2$-CH=CH- and -CH=CH- $(CH_2)_2-$

Compound (1F) is prepared by using phosphonium salt (27) according to section (II). A compound having -CH=CH- $(CH_2)_2-$ is also prepared according to the method.

(VII) Formation of $-(CH_2)_4-$

Compound (1G) is prepared by hydrogenating compound (1F) in the presence of a palladium on carbon catalyst.

1-4-2. Formation of ring $A^1$ and ring $A^2$

With regard to rings such as 1,4-cyclohexylene, 1,4-cyclohexenylene, 1,4-phenylene, 2-fluoro-1,4-phenylene, 2,3-difluoro-1,4-phenylene and tetrahydropyran-2,5-diyl, a starting material of the ring is commercially available or a synthetic method thereof is well known.

1-4-3. Synthesis example

An example of a method of preparing compound (1) is as described below. In the compounds, $R^1$, $R^2$, ring $A^1$, ring $A^2$, ring $A^3$, $Z^1$, $Z^2$, $Z^3$, a, b and c are defined in a manner identical with the definitions in item 1.

An example of a method of preparing compound (1) is as described below. Compound (53) is obtained by preparing a Grignard reagent by acting magnesium on compound (51) synthesized by a known method, and then allowing the Grignard reagent to react with compound (52) . Next, compound (54) is obtained by acting triethyl silane and a boron trifluoride-diethylether complex on compound (53). Compound (1) can be derived from compound (54) by acting s-butyllithium, copper (II) chloride and nitrobenzene on compound (54). Compound (1) can also be obtained by acting s-butyllithium and iron(III) chloride on compound (54).

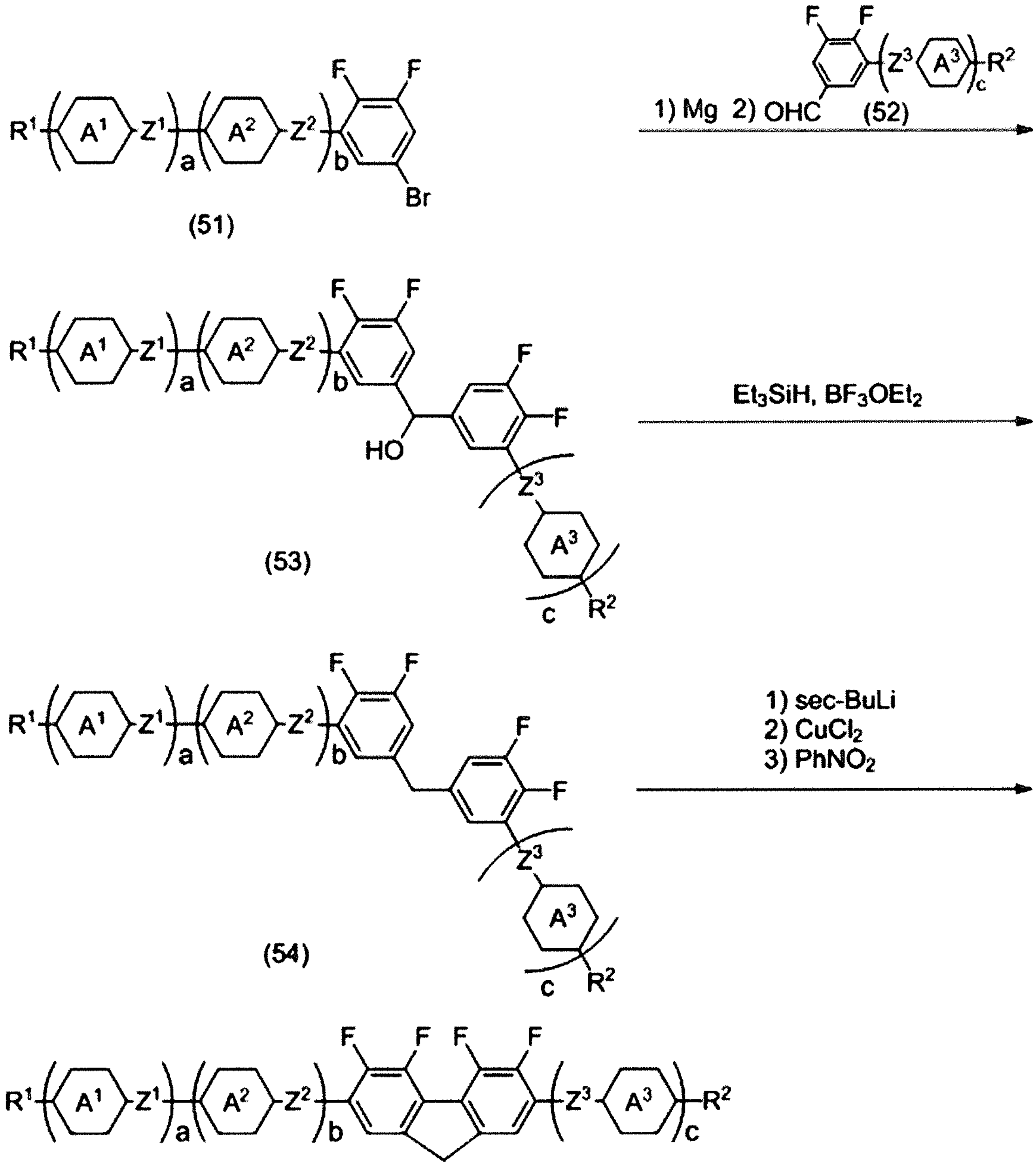


(51)

(53)

(54)

(1)

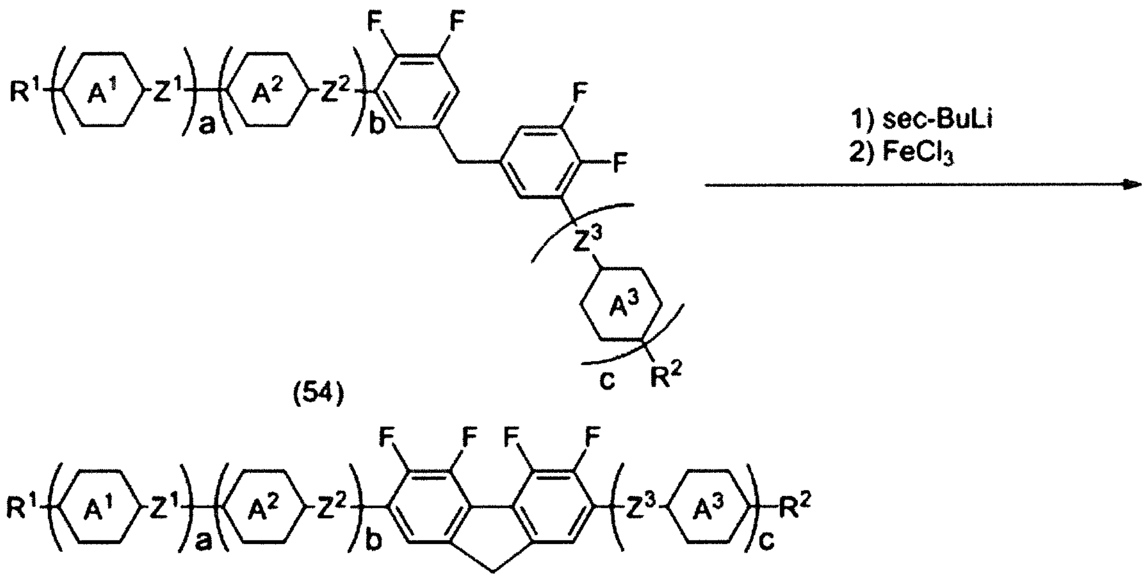


(54)

(1)

2. Composition (1)

Liquid crystal composition (1) of the invention will be described. Composition (1) contains at least one compound (1) as component A. Composition (1) may contain two or more compounds (1) . A component of the liquid crystal compound may be compound (1) only. Composition (1) preferably contains at least one compound (1) in the range of 1 to 99% by weight in order to develop excellent physical properties. In a composition having positive dielectric anisotropy, a preferred content of compound (1) is in the range of 5 to 60% by weight. In a composition having negative dielectric anisotropy, the preferred content of compound (1) is 30% by weight or less. Composition (1) may contain compound (1) and various liquid crystal compounds that are not described herein.

A preferred liquid crystal composition contains a compound selected from components B, C, D and E shown below. When composition (1) is prepared, the component can also be selected in consideration of the dielectric anisotropy of compound (1), for example. A composition in which the component is suitably selected has a high maximum temperature of a nematic phase, a low minimum temperature of the nematic phase, small viscosity, suitable optical anisotropy, large dielectric anisotropy and a suitable elastic constant.

Component B includes compounds (2) to (4). Component C includes compound (5). Component D includes compounds (6) to (12). Component E includes compounds (13) to (15). The components will be described in the order.

Component B is a compound having a halogen-containing or fluorine-containing group at a right terminal. Specific preferred examples of component B include compounds (2-1) to (2-16), compounds (3-1) to (3-113) or compounds (4-1) to (4-57).

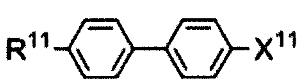
(2-1)

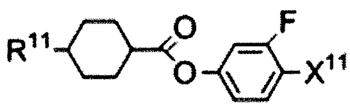
(2-9)

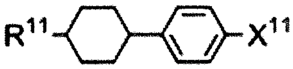
(2-2)

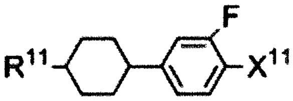
(2-3)

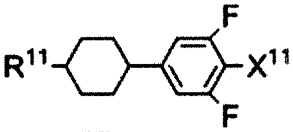
(2-4)

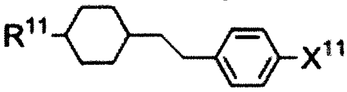
(2-5)

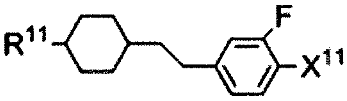
(2-6)

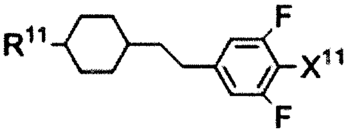
(2-7)

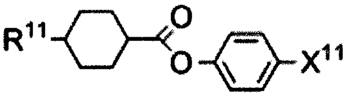
(2-8)

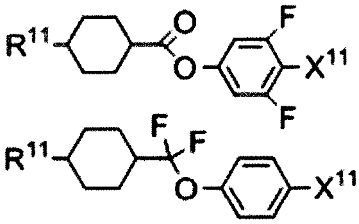
(2-10)

(2-11)

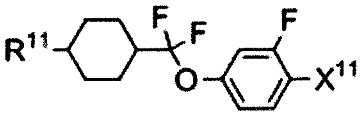
(2-12)

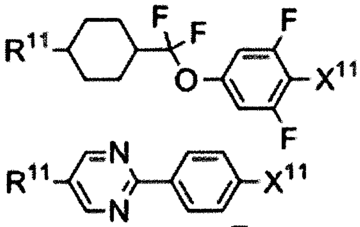
(2-13)

(2-14)

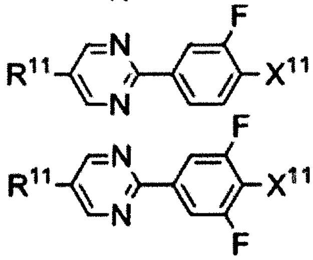
(2-15)

(2-16)

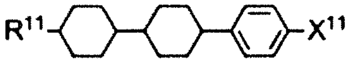
(3-1)

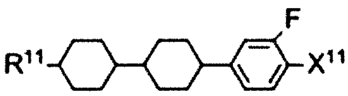
(3-2)

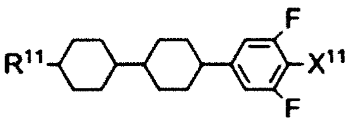
(3-3)

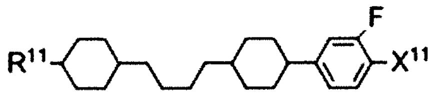
(3-20)

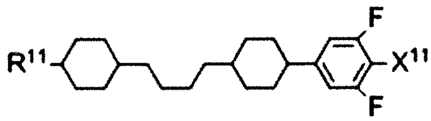
(3-21)

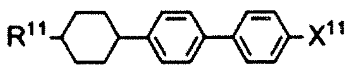
(3-22)

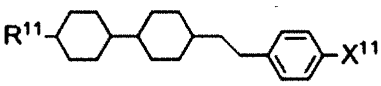
(3-4)

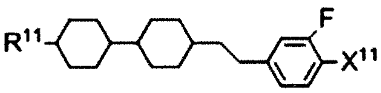
(3-5)

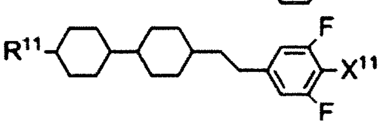
(3-6)

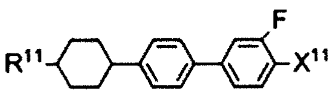
(3-23)

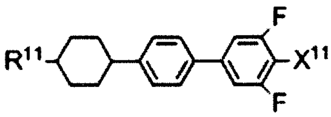
(3-24)

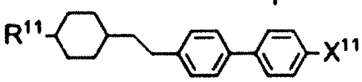
(3-25)

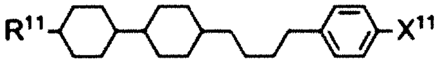 (3-7)

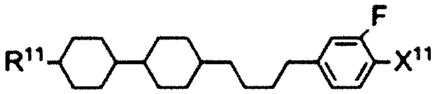 (3-8)

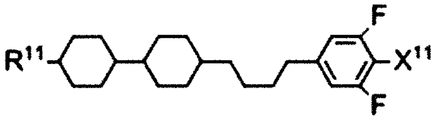 (3-9)

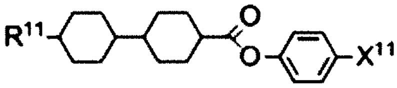 (3-10)

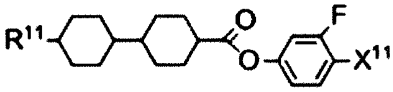 (3-11)

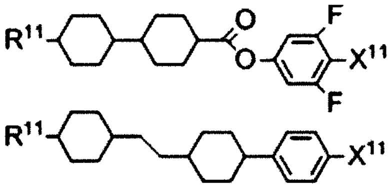 (3-12)

(3-13)

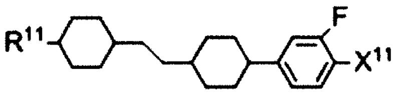 (3-14)

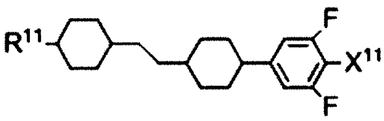 (3-15)

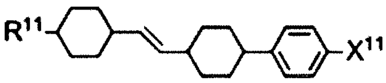 (3-16)

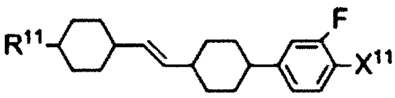 (3-17)

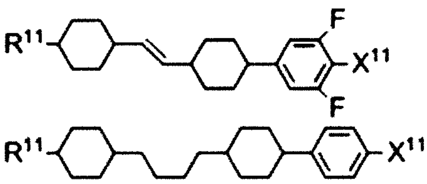 (3-18)

(3-19)

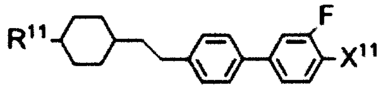 (3-26)

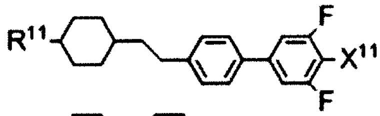 (3-27)

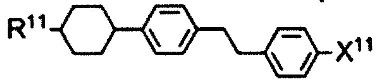 (3-28)

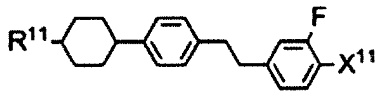 (3-29)

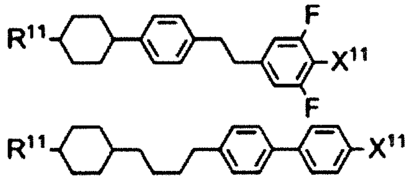 (3-30)

(3-31)

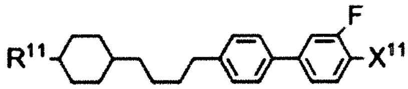 (3-32)

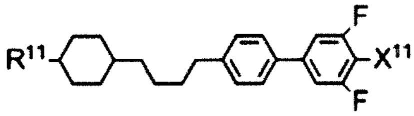 (3-33)

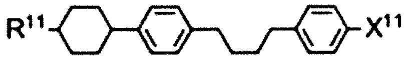 (3-34)

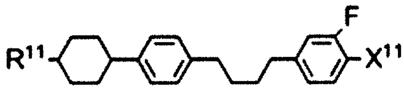 (3-35)

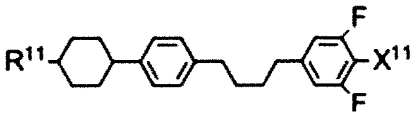 (3-36)

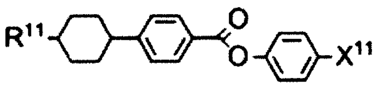 (3-37)

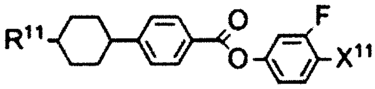 (3-38)

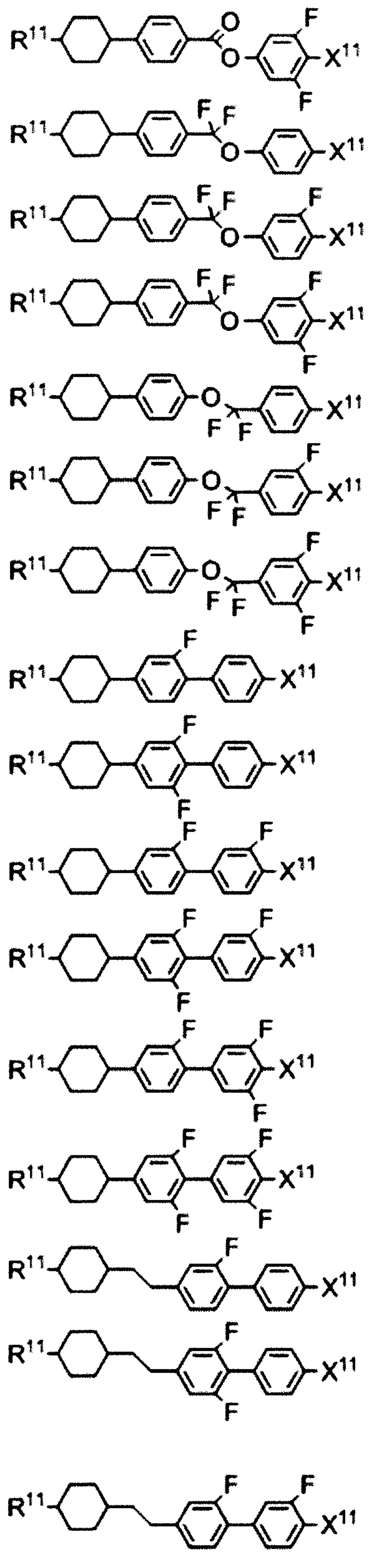

(3-39)

(3-40)

(3-41)

(3-42)

(3-43)

(3-44)

(3-45)

(3-46)

(3-47)

(3-48)

(3-49)

(3-50)

(3-51)

(3-52)

(3-53)

(3-54)

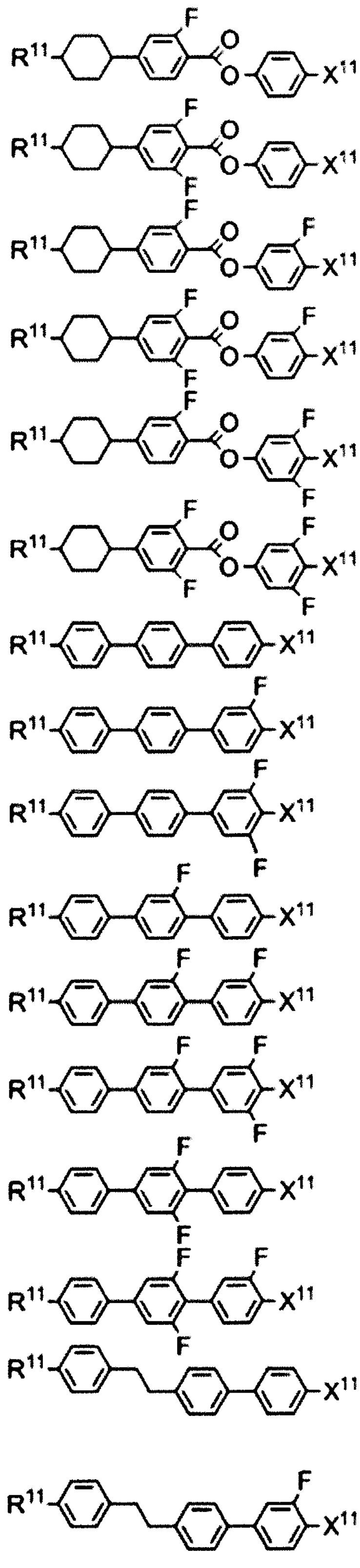

(3-58)

(3-59)

(3-60)

(3-61)

(3-62)

(3-63)

(3-64)

(3-65)

(3-66)

(3-67)

(3-68)

(3-69)

(3-70)

(3-71)

(3-72)

(3-73)

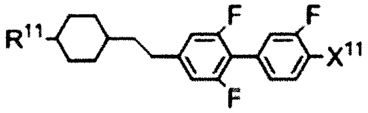
(3-55)

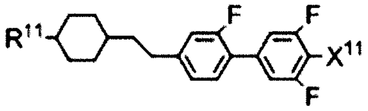
(3-56)

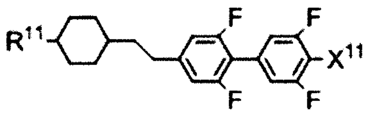
(3-57)

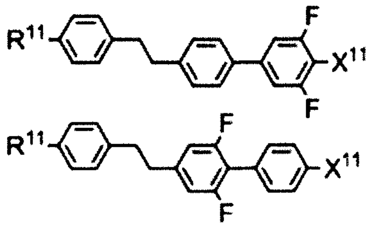
(3-74)

(3-75)

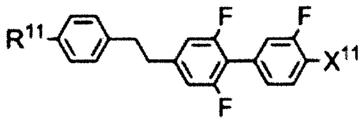
(3-76)

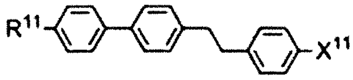
(3-77)

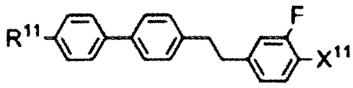
(3-78)

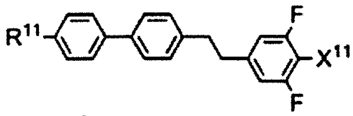
(3-79)

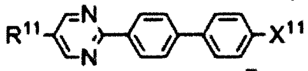
(3-80)

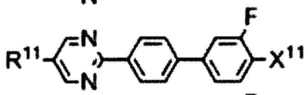
(3-81)

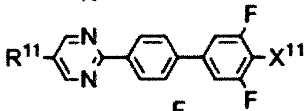
(3-82)

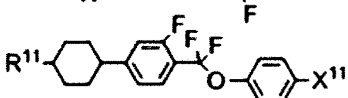
(3-83)

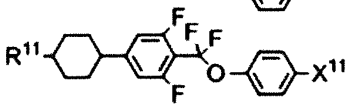
(3-84)

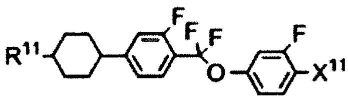
(3-85)

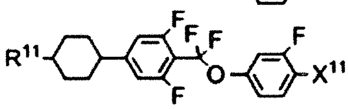
(3-86)

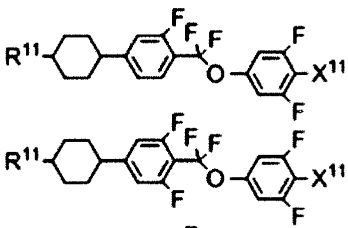
(3-87)

(3-88)

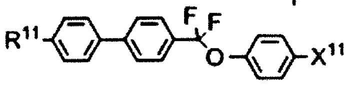
(3-89)

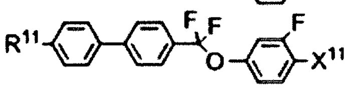
(3-90)

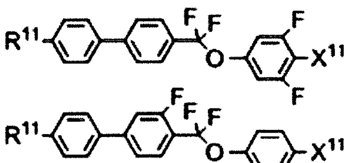
(3-91)

(3-92)

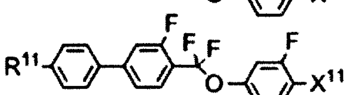
(3-93)

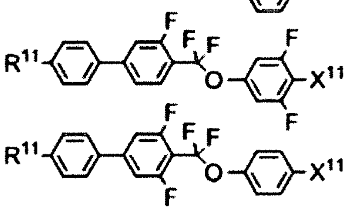
(3-94)

(3-95)

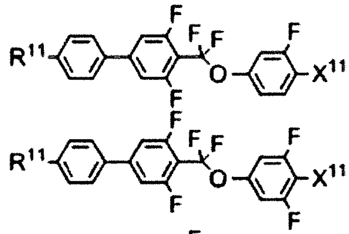
(3-96)

(3-97)

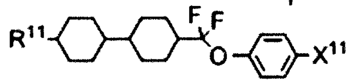
(3-98)

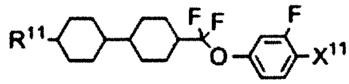
(3-99)

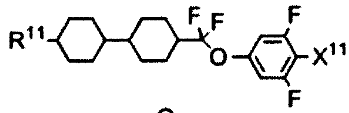
(3-100)

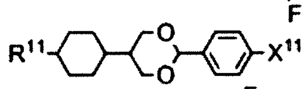
(3-101)

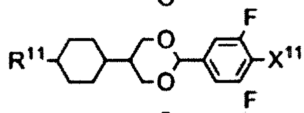
(3-102)

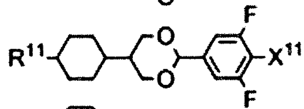
(3-103)

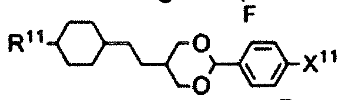
(3-104)

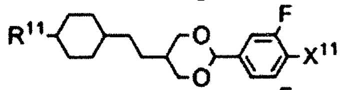
(3-105)

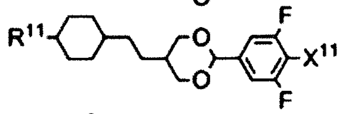
(3-106)

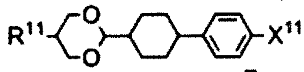
(3-107)

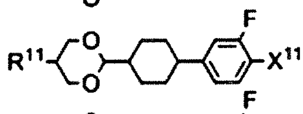
(3-108)

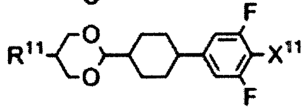
(3-109)

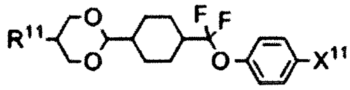
(3-110)

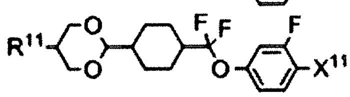
(3-111)

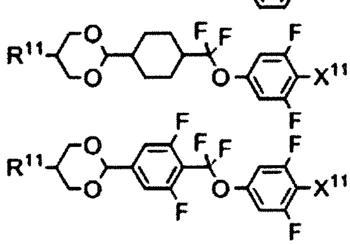
(3-112)

(3-113)

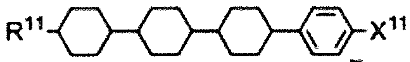
(4-1)

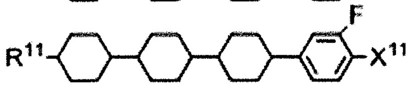
(4-2)

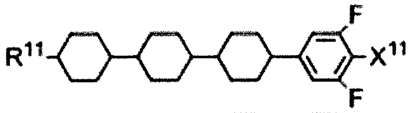
(4-3)

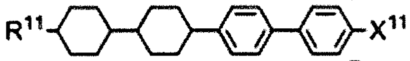
(4-4)

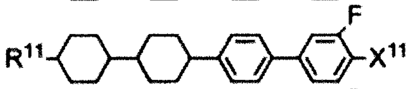
(4-5)

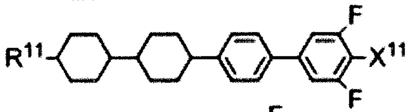
(4-6)

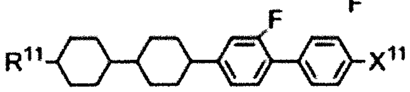
(4-7)

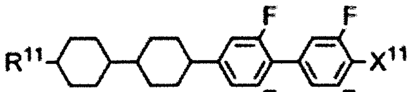
(4-8)

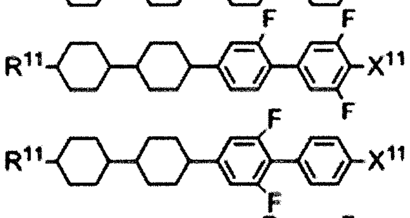
(4-9)

(4-10)

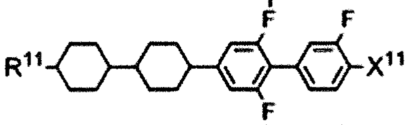
(4-11)

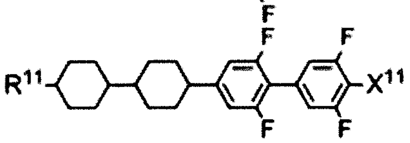
(4-12)

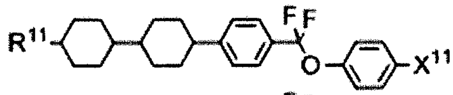
(4-21)

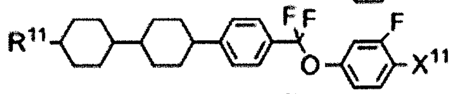
(4-22)

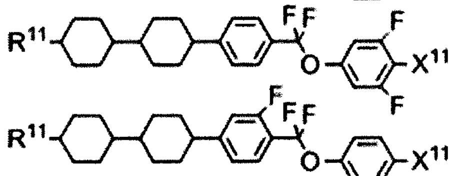
(4-23)

(4-24)

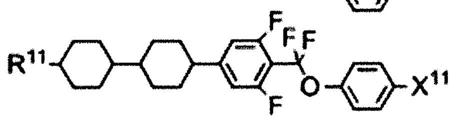
(4-25)

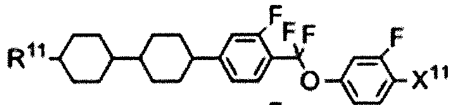
(4-26)

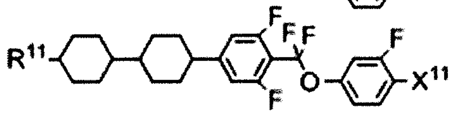
(4-27)

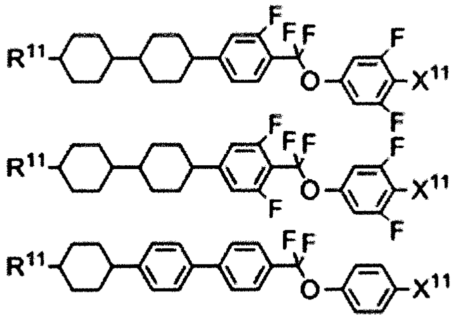
(4-28)

(4-29)

(4-30)

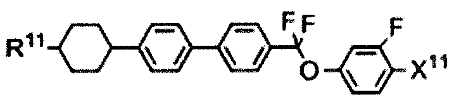
(4-31)

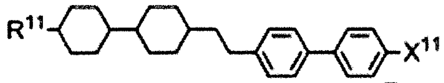
(4-13)

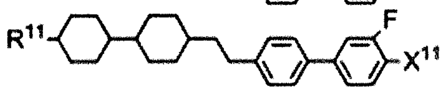
(4-14)

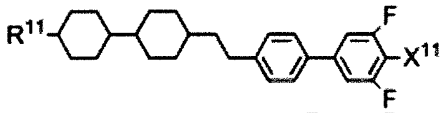
(4-15)

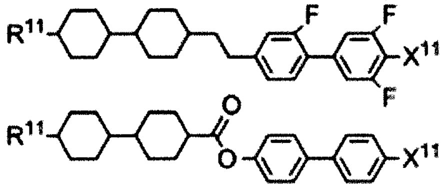
(4-16)

(4-17)

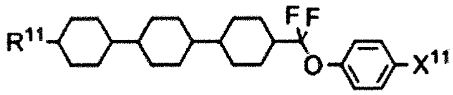
(4-18)

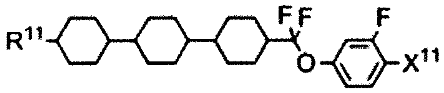
(4-19)

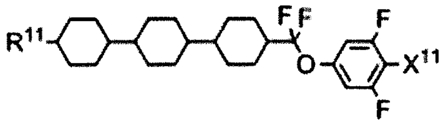
(4-20)

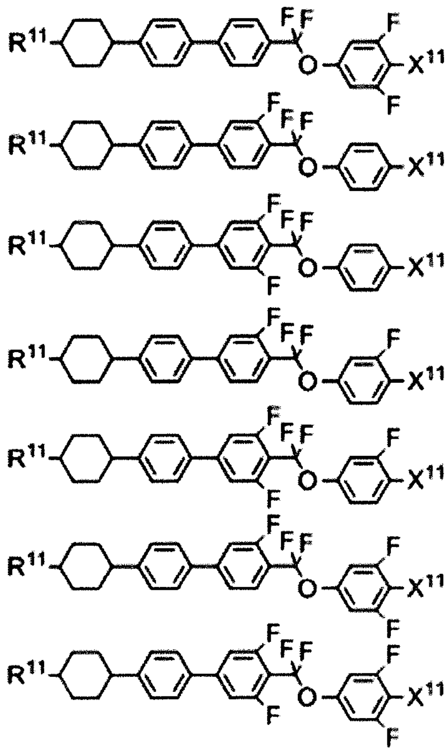
(4-32)

(4-33)

(4-34)

(4-35)

(4-36)

(4-37)

(4-38)

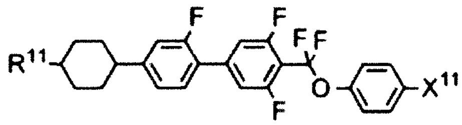
(4-39)

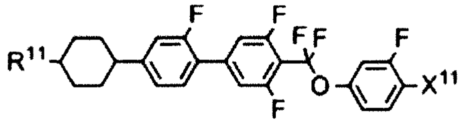
(4-40)

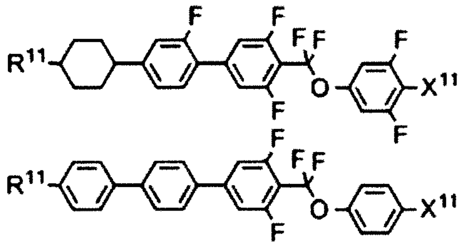
(4-41)

(4-42)

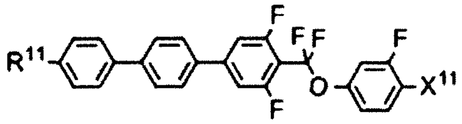
(4-43)

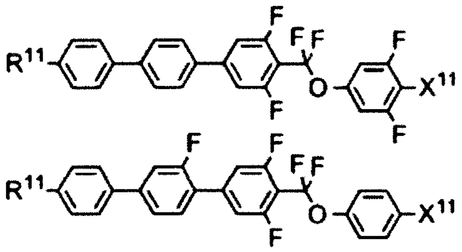
(4-44)

(4-45)

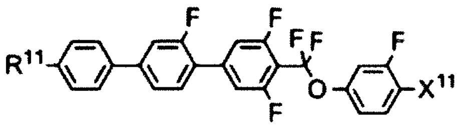
(4-46)

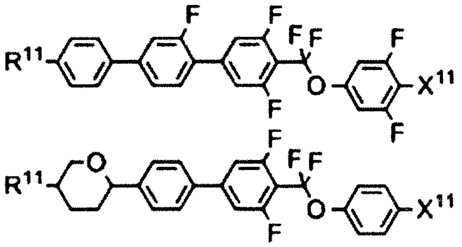
(4-47)

(4-48)

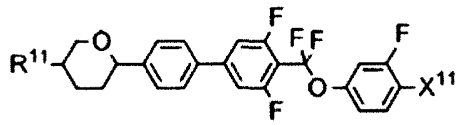
(4-49)

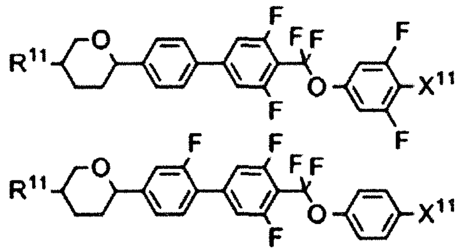
(4-50)

(4-51)

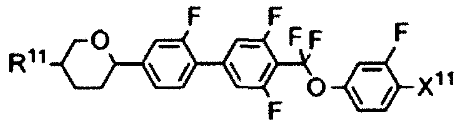
(4-52)

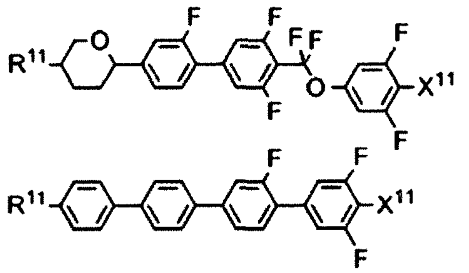
(4-53)

(4-54)

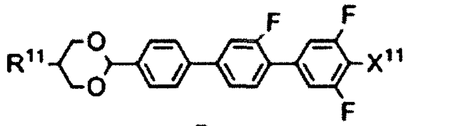
(4-55)

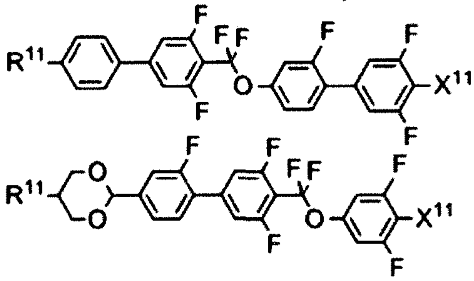
(4-56)

(4-57)

In the compounds (component B), $R^{11}$ and $X^{11}$ are defined in a manner identical with the definitions in formulas (2) to (4) in item 11.

Component B has the positive dielectric anisotropy and superb stability to heat, light or the like, and therefore is used when a composition for a TFT mode or a PSA mode is prepared. A content of component (B) is suitably in the range of 1 to 99% by weight, preferably in the range of 10 to 97% by weight, and further preferably in the range of 40 to 95% by weight, based on the weight of the composition. The viscosity of the composition can be adjusted by further adding compounds (12) to (14) (component E) thereto.

Component C is compound (5) in which a right terminal group is -C=N or -C≡C-C≡N. Specific preferred examples of component C include compounds (5-1) to (5-64).

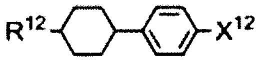 (5-1)

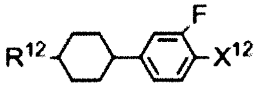 (5-2)

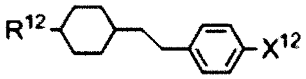 (5-3)

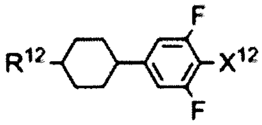 (5-4)

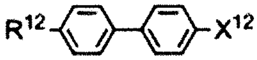 (5-5)

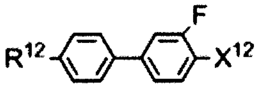 (5-6)

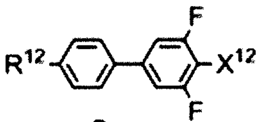 (5-7)

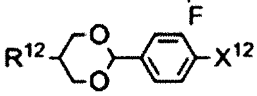 (5-8)

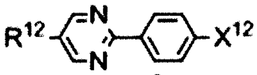 (5-9)

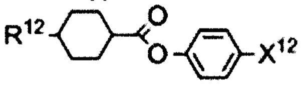 (5-10)

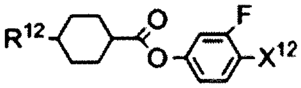 (5-11)

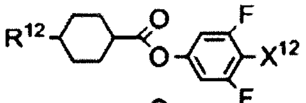 (5-12)

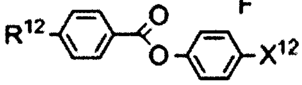 (5-13)

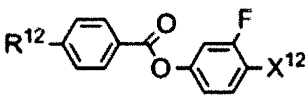 (5-14)

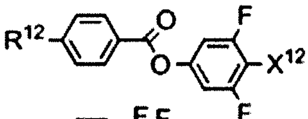 (5-15)

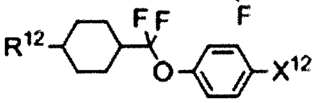 (5-16)

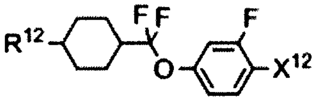 (5-17)

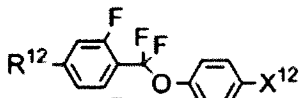 (5-22)

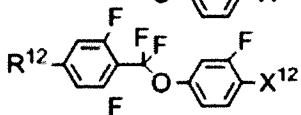 (5-23)

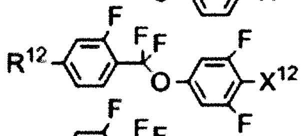 (5-24)

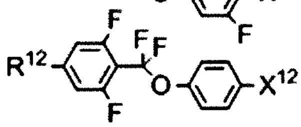 (5-25)

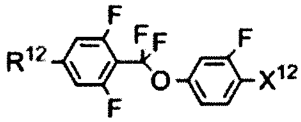 (5-26)

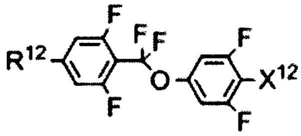 (5-27)

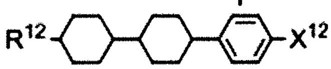 (5-28)

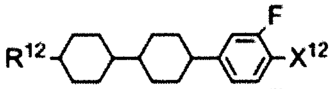 (5-29)

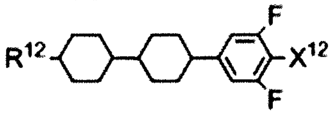 (5-30)

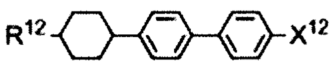 (5-31)

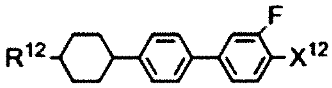 (5-32)

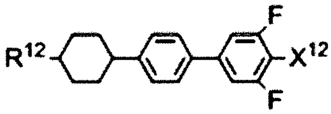 (5-33)

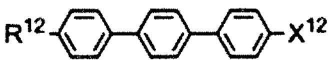 (5-34)

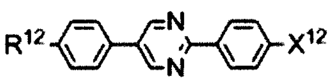 (5-35)

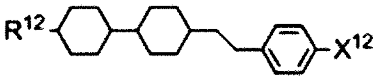 (5-36)

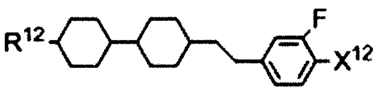 (5-37)

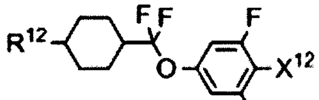 (5-18)

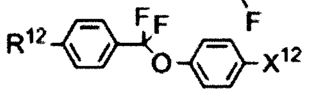 (5-19)

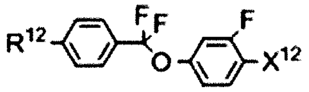 (5-20)

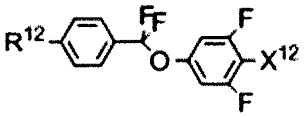 (5-21)

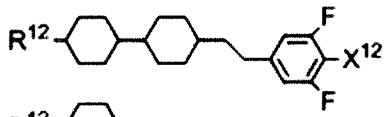 (5-38)

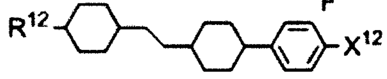 (5-39)

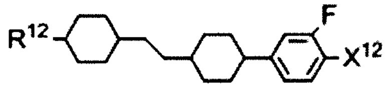 (5-40)

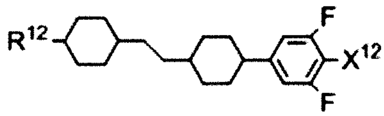 (5-41)

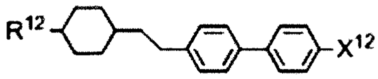 (5-42)

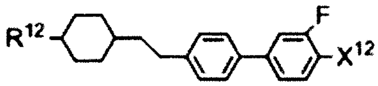 (5-43)

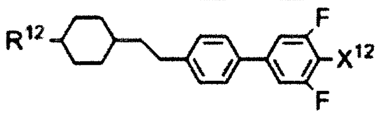 (5-44)

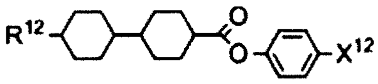 (5-45)

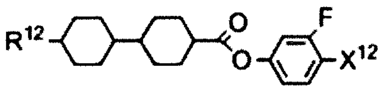 (5-46)

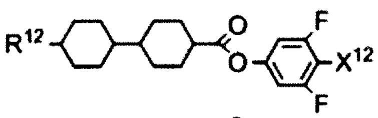 (5-47)

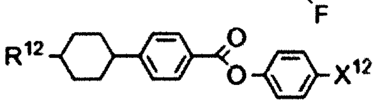 (5-48)

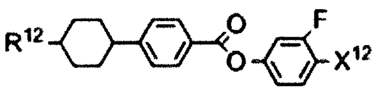 (5-49)

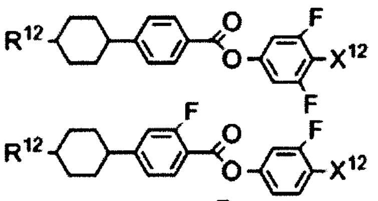 (5-50)

(5-51)

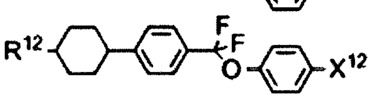 (5-52)

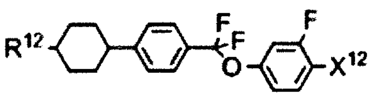 (5-53)

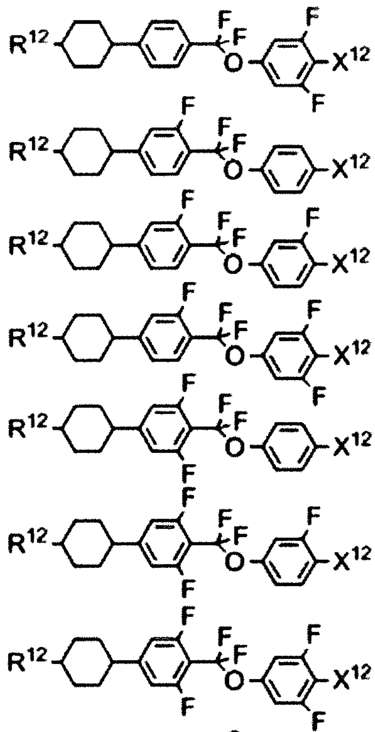 (5-54)

(5-55)

(5-56)

(5-57)

(5-58)

(5-59)

(5-60)

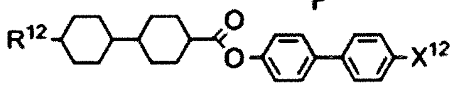 (5-61)

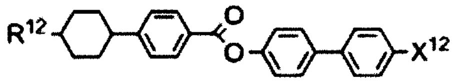 (5-62)

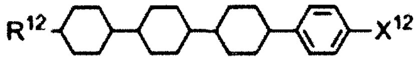 (5-63)

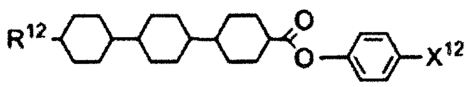 (5-64)

In the compounds (component C), $R^{12}$ and $X^{12}$ are defined in a manner identical with the definitions in formula (5) in item 12.

Component C has the positive dielectric anisotropy and the value thereof is large, and therefore is mainly used when a composition for an STN mode, a TN mode or the PSA mode. The dielectric anisotropy of the composition can be increased by adding component C thereto. Component C is effective in extending the temperature range of the liquid crystal phase, adjusting the viscosity or the optical anisotropy. Component C is also useful in adjusting a voltage-transmittance curve of the device.

When the composition for the STN mode or the TN mode, a content of component C is suitably in the range of 1 to 99% by weight, preferably in the range of 10 to 97% by weight, and further preferably in the range of 40 to 95% by weight, based on the weight of the composition. The temperature range of the liquid crystal phase, the viscosity, the optical anisotropy, the dielectric anisotropy or the like of the composition can be adjusted by adding component E thereto.

Component D includes compounds (6) to (12). The compounds have a benzene ring in which atoms in a lateral position are replaced by two halogens, such as 2,3-difluoro-1,4-phenylene. Specific preferred examples of component D include compounds (6-1) to (6-8), compounds (7-1) to (7-17), compound (8-1), compounds (9-1) to (9-3), compounds (10-1) to (10-11), compounds (11-1) to (11-3) or compounds (12-1) to (12-3).

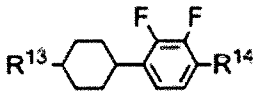
(6-1)

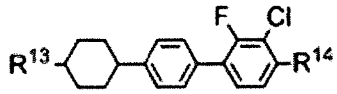
(7-13)

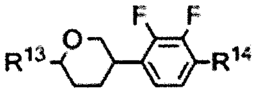
(6-2)

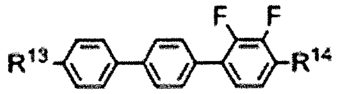
(7-14)

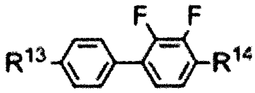
(6-3)

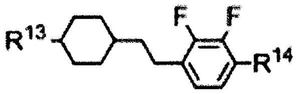
(6-4)

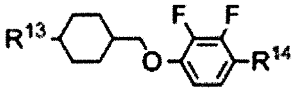
(6-5)

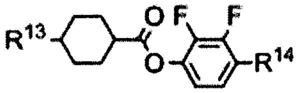
(6-6)

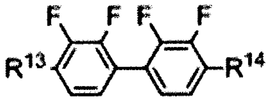
(6-7)

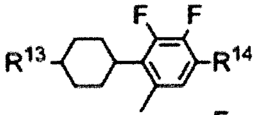
(6-8)

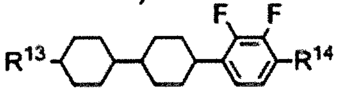
(7-1)

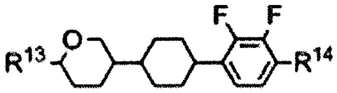
(7-2)

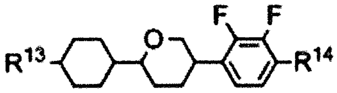
(7-3)

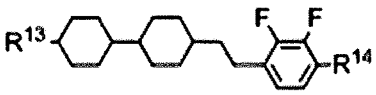
(7-4)

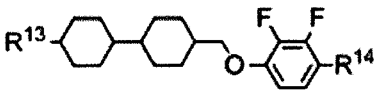
(7-5)

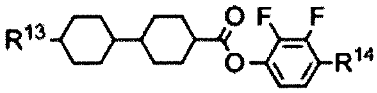
(7-6)

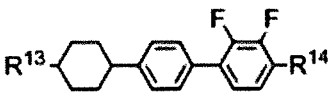
(7-7)

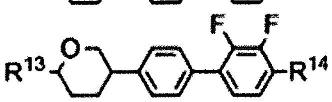
(7-8)

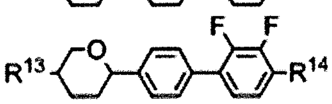
(7-9)

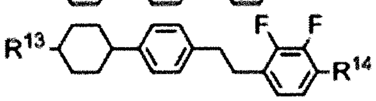
(7-10)

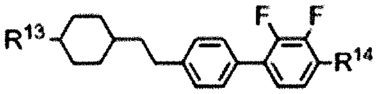
(7-11)

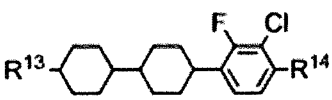
(7-12)

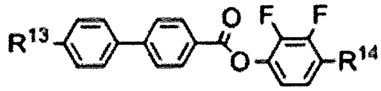
(7-15)

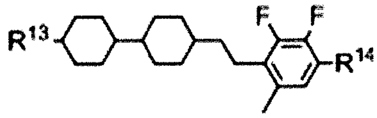
(7-16)

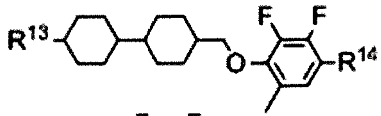
(7-17)

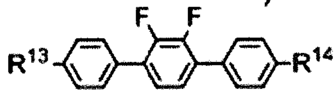
(8-1)

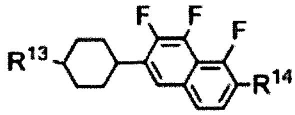
(9-1)

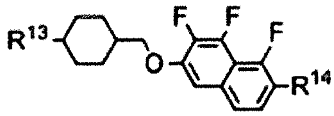
(9-2)

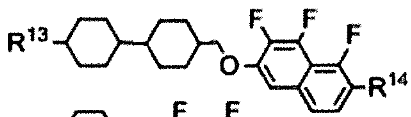
(9-3)

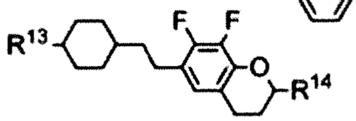
(10-1)

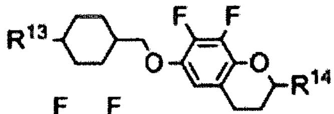
(10-2)

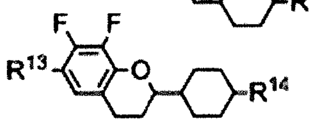
(10-3)

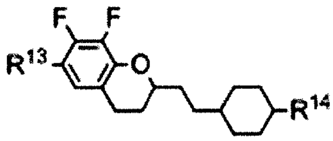
(10-4)

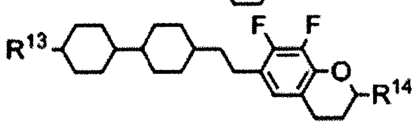
(10-5)

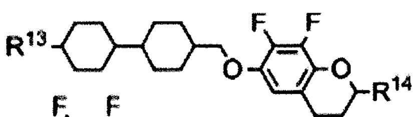
(10-6)

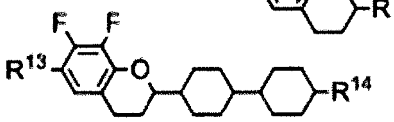
(10-7)

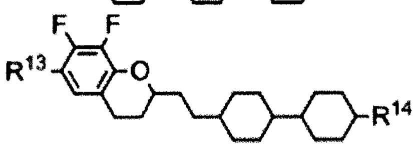
(10-8)

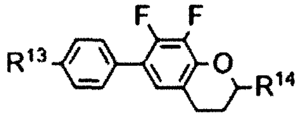
(10-9)

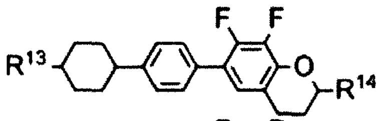
(10-10)

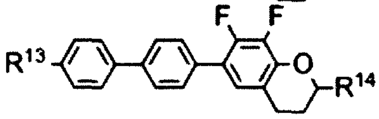
(10-11)

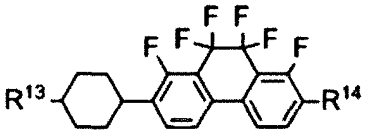
(11-1)

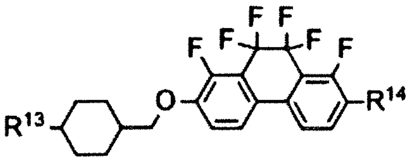
(11-2)

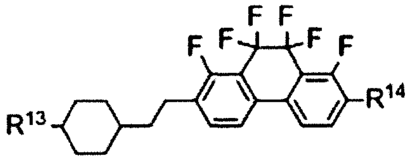
(11-3)

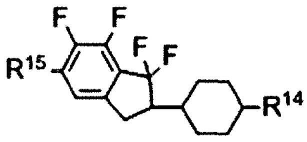
(12-1)

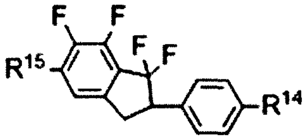
(12-2)

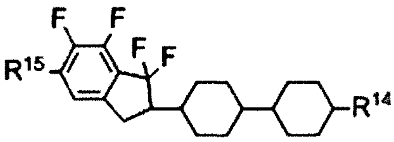
(12-3)

In the compounds (component D), $R^{13}$, $R^{14}$ and $R^{15}$ are defined in a manner identical with the definitions in formulas (6) to (12) in item 9.

Component D is a compound having negative dielectric anisotropy. Component D is mainly used when a composition for a VA mode or the PSA mode is prepared. Among types of compound D, compound (6) is a bicyclic compound, and therefore is effective mainly in adjusting the viscosity, the optical anisotropy or the dielectric anisotropy. Compounds (7) and (8) are a tricyclic compound, and therefore are effective in increasing the maximum temperature, the optical anisotropy or the dielectric anisotropy. Compounds (9) to (12) are effective in increasing the dielectric anisotropy.

When the composition for the VA or the PSA mode is prepared, a content of component D is preferably 40% by weight or more, and further preferably in the range of 50 to 95% by weight based on the weight of the composition,. When component D is added to the composition having positive dielectric anisotropy, a content of component D is preferably 30% by weight or less based on the weight of the composition. The elastic constant of the composition is adjusted by adding component D, and the voltage-transmittance curve of the device can be adjusted.

Component E is a compound in which two terminal groups are alkyl or the like. Specific preferred examples of component E include compounds (13-1) to (13-11), compounds (14-1) to (14-19) or compounds (15-1) to (15-7).

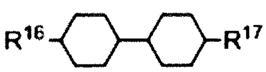
(13-1)

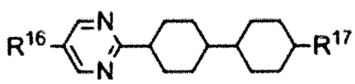
(14-9)

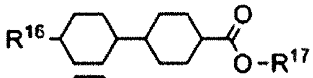
(13-2)

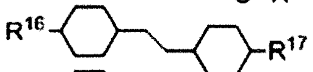
(13-3)

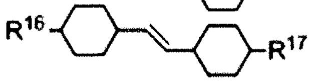
(13-4)

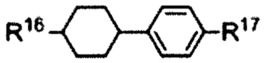
(13-5)

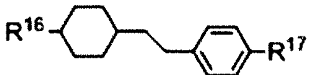
(13-6)

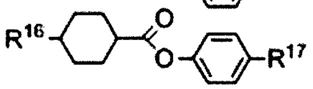
(13-7)

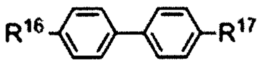
(13-8)

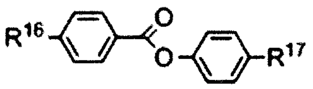
(13-9)

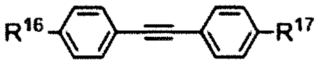
(13-10)

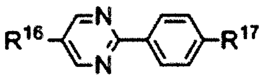
(13-11)

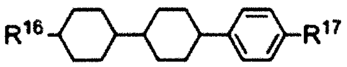
(14-1)

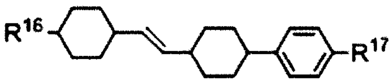
(14-2)

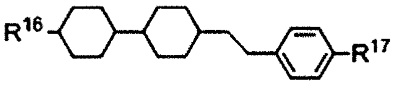
(14-3)

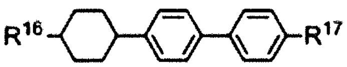
(14-4)

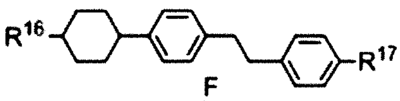
(14-5)

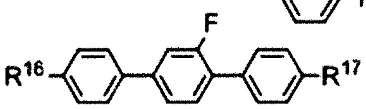
(14-6)

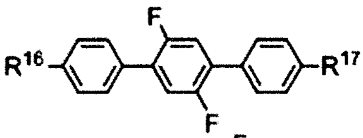
(14-7)

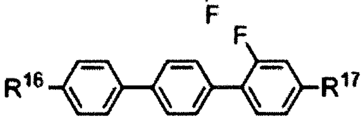
(14-8)

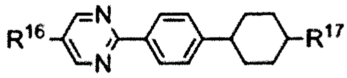
(14-10)

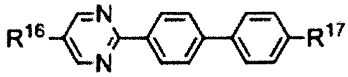
(14-11)

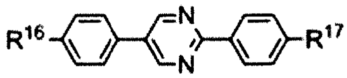
(14-12)

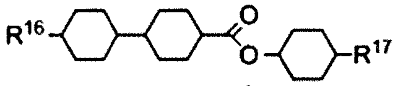
(14-13)

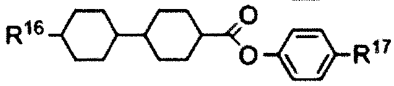
(14-14)

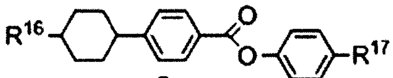
(14-15)

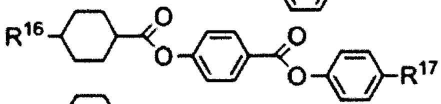
(14-16)

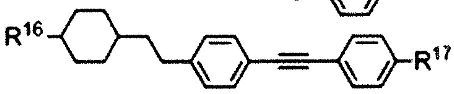
(14-17)

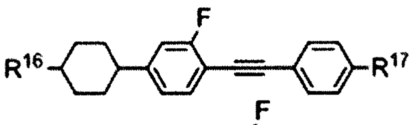
(14-18)

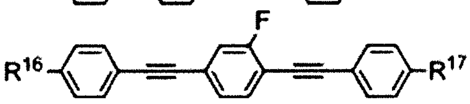
(14-19)

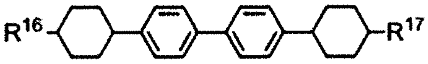
(15-1)

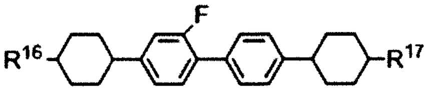
(15-2)

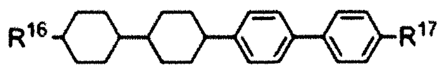
(15-3)

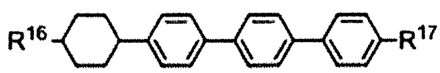
(15-4)

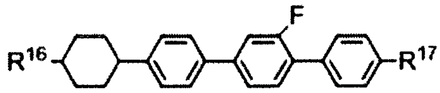
(15-5)

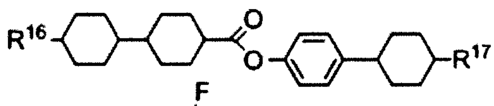
(15-6)

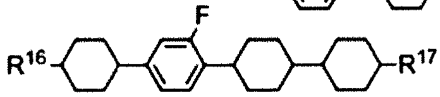
(15-7)

In the compounds (component E) , $R^{16}$ and $R^{17}$ are defined in a manner identical with the definitions in formulas (13) to (15) in item 10.

Component E has a small absolute value of the dielectric anisotropy, and therefore is a compound close to neutrality. Compound (13) is effective mainly in adjusting the viscosity or the optical anisotropy. Compounds (14) and (15) are effective in extending the temperature range of the nematic phase by increasing the maximum temperature, or adjusting the optical anisotropy.

If a content of component E is increased, the viscosity of the composition decreases, but the dielectric anisotropy thereof also decreases. Therefore, the content is preferably as large as possible as long as the composition meets a desired value of threshold voltage of the device. Accordingly, when the composition for the VA mode or the PSA mode

is prepared, a content of component E is preferably 30% or more by weight, and further preferably 40% or more by weight, based on the weight of the composition.

Preparation of composition (1) is performed by a method of dissolving required components at a high temperature, or the like. According to an application, an additive may be added to the composition. Examples of the additive include an optically active compound, a polymerizable compound, a polymerization initiator, an antioxidant, an ultraviolet light absorber, a light stabilizer, a heat stabilizer and an antifoaming agent. Such additives are well known to those skilled in the art, and described in literature.

Composition (1) may further contain at least one optically active compound. As the optically active compound, a publicly known chiral dopant can be added. The chiral dopant has an effect of preventing a reverse twist by inducing helical structure in liquid crystal molecules to give a required twist angle. Specific preferred examples of the chiral dopant include compounds (Op-1) to (Op-18). In compound (Op-18), ring J is 1,4-cyclohexylene or 1,4-phenylene, and $R^{24}$ is alkyl having 1 to 10 carbons.

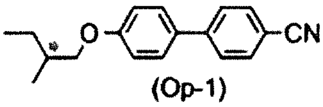
(Op-1)

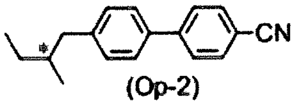
(Op-2)

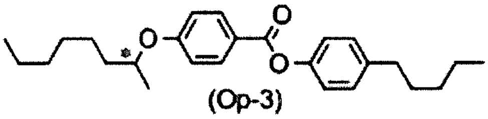
(Op-3)

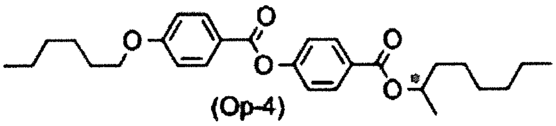
(Op-4)

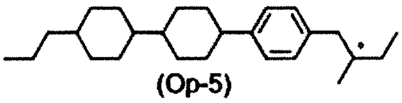
(Op-5)

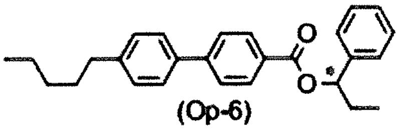
(Op-6)

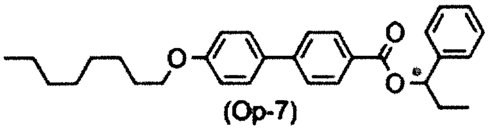
(Op-7)

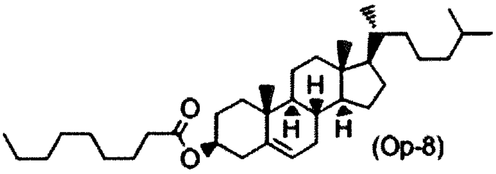
(Op-8)

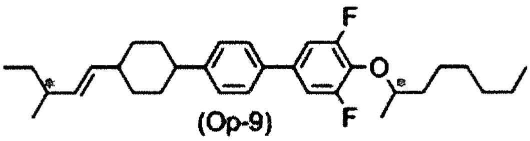
(Op-9)

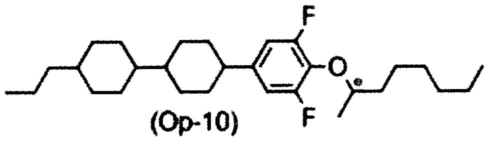
(Op-10)

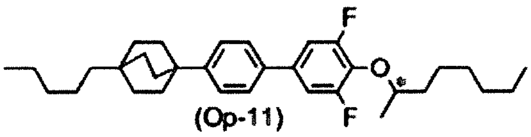
(Op-11)

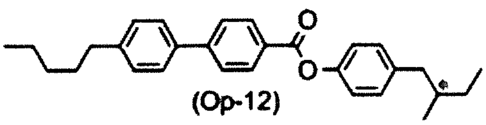
(Op-12)

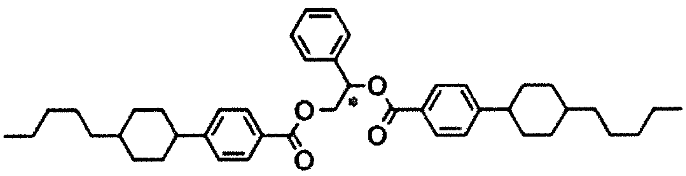
(Op-13)

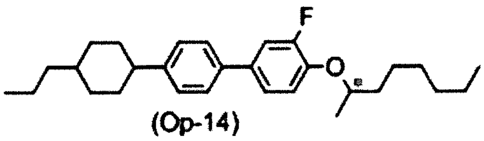
(Op-14)

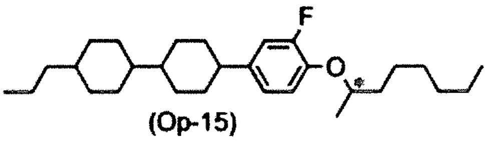
(Op-15)

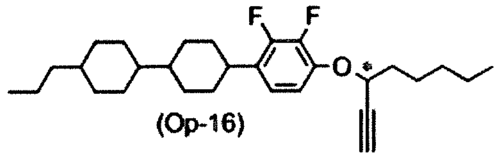
(Op-16)

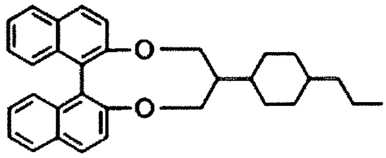
(Op-17)

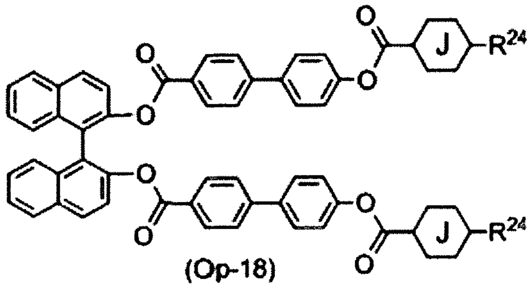
(Op-18)

A helical pitch of composition (1) is adjusted by adding such an optically active compound. The helical pitch is preferably adjusted in the range of 40 to 200 micrometers in the composition for the TFT mode and the TN mode. The helical pitch is preferably adjusted in the range of 6 to 20 micrometers in the composition for the STN mode. The helical pitch is preferably adjusted in the range of 1. 5 to 4 micrometers in the case of a composition for a BTN mode. Two or more optically active compounds may be added for the purpose of adjusting temperature dependence of the helical pitch.

The composition (1) can also be used for the PSA mode by adding the polymerizable compound thereto. Examples of the polymerizable compound include acrylate, methacrylate, a vinyl compound, a vinyloxy compound, propenyl ether, an epoxy compound (oxirane, oxetane), and vinyl ketone, and specific preferred examples include compounds (M-1) and (M-12) described below. The polymerizable compound is polymerized by irradiation with ultraviolet light or the like. The polymerizable compound may be polymerized in the presence of a suitable initiator such as a photopolymerization initiator. Suitable conditions for polymerization and a suitable type and suitable amount of the initiator are known to those skilled in the art and are described in literature.

In compounds (M-1) to (M-12), $R^{20}$ is hydrogen or methyl; s is 0 or 1; t and u are independently an integer from 1 to 10. Parenthesized symbol F means hydrogen or fluorine.

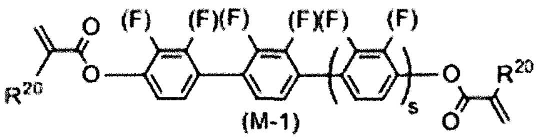
(M-1)

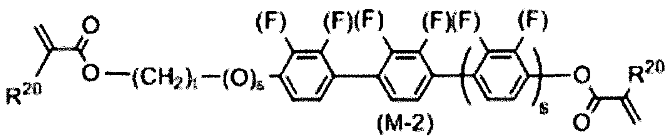
(M-2)

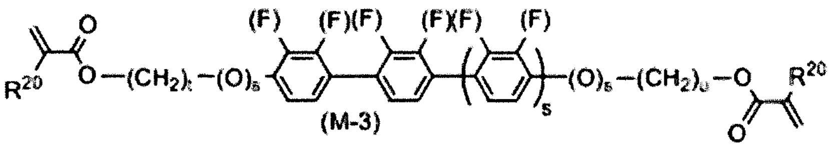
(M-3)

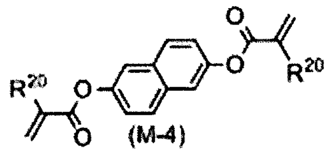
(M-4)

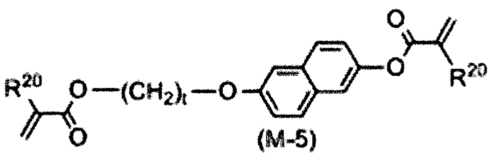
(M-5)

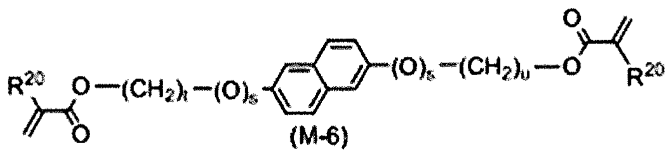
(M-6)

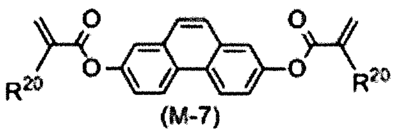
(M-7)

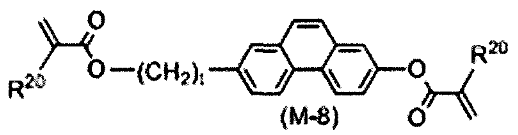
(M-8)

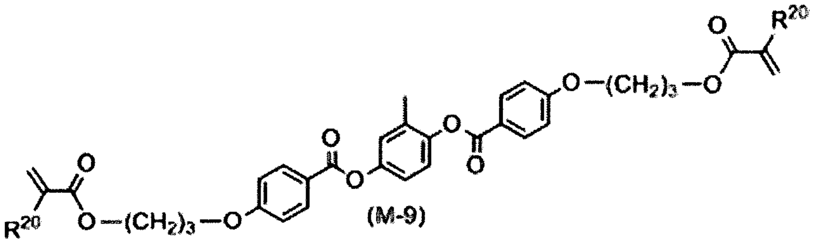

(M-9)

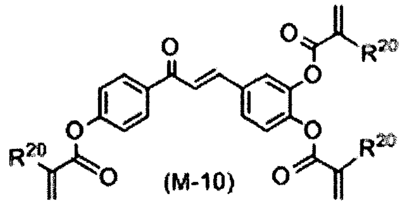

(M-10)

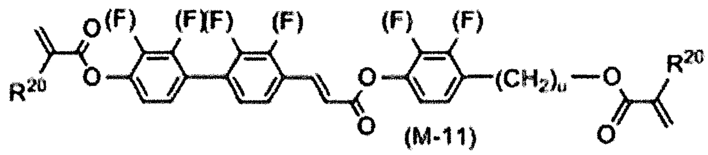

(M-11)

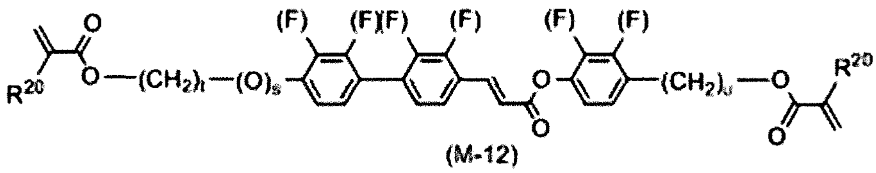

(M-12)

The antioxidant is effective for maintaining a large voltage holding ratio. Specific preferred examples of the antioxidant include compound (AO-1) or (AO-2); and IRGANOX 415, IRGANOX 565, IRGANOX 1010, IRGANOX 1035, IRGANOX 3114 or IRGANOX 1098 (trade names: BASF SE). The ultraviolet light absorber is effective for preventing a decrease of the maximum temperature. Preferred examples of the ultraviolet light absorber include a benzophenone derivative, a benzoate derivative, and a triazole derivative. Specific examples thereof include compounds (AO-3) or (AO-4) described below; TINUVIN 329, TINUVIN P, TINUVIN 326, TINUVIN 234, TINUVIN 213, TINUVIN 400, TINUVIN 328 and TINUVIN 99-2 (trade names: BASF SE); or 1,4-diazabicyclo[2.2.2]octane (DABCO).

A light stabilizer such as amine having steric hindrance is preferred for maintaining the large voltage holding ratio. Specific preferred examples of the light stabilizer include compound (AO-5) or (AO-6) described below; and TINUVIN 144, TINUVIN 765 or TINUVIN 770DF (trade names: BASF SE). The heat stabilizer is also effective for maintaining the large voltage holding ratio, and specific preferred examples include IRGAFOS 168 (trade name: BASF SE). The antifoaming agent is effective for preventing foam formation. Preferred examples of the antifoaming agent include dimethyl silicone oil and methylphenyl silicone oil.

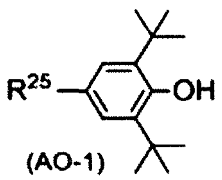

(AO-1)

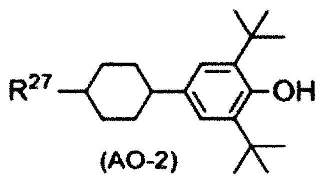

(AO-2)

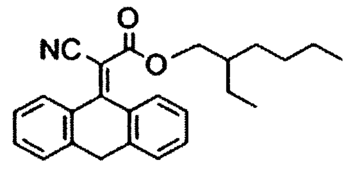

(AO-3)

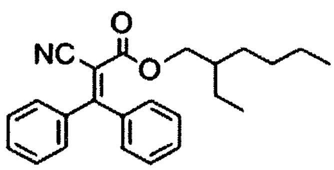

(AO-4)

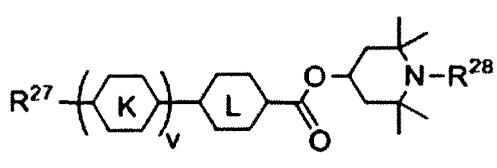

(AO-5)

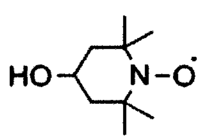

(AO-6)

In compound (AO-1), $R^{25}$ is alkyl having 1 to 20 carbons, alkoxy having 1 to 20 carbons, $-COOR^{26}$ or $-CH_2CH_2COOR^{26}$; and $R^{26}$ is alkyl having 1 to 20 carbons. In compounds (AO-2) and (AO-5), $R^{27}$ is alkyl having 1 to 20 carbons. In compound (AO-5), ring K and ring L are 1, 4-cyclohexylene or 1, 4-phenylene, v is 0, 1 or 2, and $R^{28}$ is hydrogen, methyl or O.

Composition (1) can also be used for a guest host (GH) mode if a dichroic dye such as a merocyanine, stylyl, azo, azomethine, azoxy, quinophthalone, anthraquinone or tetrazine dye is added thereto.

In composition (1), the maximum temperature can be adjusted to 70°C or higher and the minimum temperature to be -10°C or lower by suitably adjusting a kind and a proportion of the component compounds, and therefore the temperature range of the nematic phase is wide. Accordingly, the liquid crystal display device including the composition can be used in the wide temperature range.

In composition (1), the optical anisotropy can be adjusted in the range of 0.10 to 0.13, or the range of 0.05 to 0.18 by suitably adjusting the kind and the proportion of the component compounds. In a similar manner, the dielectric anisotropy thereof can be adjusted in the range of -5.0 to -2.0. Preferred dielectric anisotropy is in the range of -4.5 to -2.5. Composition (1) having the dielectric anisotropy in this range can be preferably used in the liquid crystal display device to be operated by the IPS mode, the VA mode, the PSA mode or the like.

4. Liquid crystal display device

Composition (1) can be used in an AM device. The composition can be further used in a PM device. The composition can be used in the AM device and the PM device both having the modes such as PC, TN, STN, ECB, OCB, IPS, VA and FPA modes. The composition is particularly preferably used in the AM device having the TN, the OCB mode, the IPS mode or the FFS mode. In the AM device having the IPS mode or the FFS mode, alignment of the liquid crystal molecules in the state in which no voltage is applied may be homogeneous or homeotropic relative to a glass substrate. The device may be of a reflective type, a transmissive type or a transflective type. The composition is preferably used in the transmissive type device. The composition can also be preferably used in an amorphous silicon-TFT device or a polycrystal silicon-TFT device. The composition can also be preferably used in a nematic curvilinear aligned phase (NCAP) device prepared by microencapsulating the composition, or in a polymer dispersed (PD) device in which a three-dimensional network polymer is formed in the composition.

Composition (1) has the negative dielectric anisotropy, and therefore can be preferably used in a liquid crystal display device that has an operating mode such as the VA mode, the IPS mode and the PSA mode, and is driven by the AM mode. The liquid crystal composition can be particularly preferably used in the liquid crystal display device that is driven by the AM mode.

A direction of an electric field is perpendicular to a liquid crystal layer in the liquid crystal display device that is operated by the TN mode, the VA mode or the like. Meanwhile, the direction of the electric field is parallel to the liquid crystal layer in the liquid crystal display device that is operated by the IPS mode or the like. A structure of the liquid crystal display device that is operated by the VA mode is reported in SID '97 Digest of Technical Papers, 28, 845 (1997) by K. Ohmuro, S. Kataoka, T. Sasaki and Y. Koike. A structure of the liquid crystal display device that is operated by the IPS mode is reported in WO 91/10936 A (family: US 5576867 B).

**Examples**

The invention will be described in greater detail by way of Examples. However, the invention is not limited by the Examples.

1-1. Example of compound (1)

Compound (1) was synthesized by procedures described below. The thus prepared compound was identified by a method such as NMR analysis. Physical properties of the compound were measured by methods described below.

NMR analysis

As a measuring apparatus, DRX-500 made by Bruker BioSpin Corporation was used. In $^{1}$H-NMR measurement, a sample was dissolved in a deuterated solvent such as $CDCl_3$, and measurement was carried out under conditions of room temperature, 500 MHz and 16 times of accumulation. Tetramethylsilane was used as an internal standard. In $^{19}$F-NMR measurement, $CFCl_3$ was used as an internal standard, and measurement was carried out under conditions of 24 times of accumulation. In the explanation of a nuclear magnetic resonance spectrum, s, d, t, q, quin, sex, m and br stand for a singlet, a doublet, a triplet, a quartet, a quintet, a sextet, a multiplet and br being broad, respectively.

Sample for measurement

Upon measuring phase structure and a transition temperature, a liquid crystal compound itself was used as a sample. Upon measuring physical properties such as a maximum temperature of a nematic phase, viscosity, optical anisotropy and dielectric anisotropy, a composition prepared by mixing a compound with a base liquid crystal was used as the sample.

When the sample prepared by mixing the compound with the base liquid crystal was used, measurement was carried out according to the method described below. The sample was prepared by mixing 10% by weight of the compound and 90% by weight of the base liquid crystal. Then, an extrapolated value was calculated from measured values of the sample, according to the extrapolation method represented by the equation below, and the extrapolated value was described:

{Extrapolated value} = {100 × (measured value of a sample) - (% by weight of a base liquid crystal) × (measured value of the base liquid crystal)} / (% by weight of a compound).

When crystals (or a smectic phase) precipitated at 25°C even at the proportion, a proportion of the compound to the base liquid crystal was changed in the order of (5% by weight : 95% by weight), (3% by weight : 97% by weight) and (1% by weight : 99% by weight). Physical properties of the sample were measured at the proportion at which no crystal (or no smectic phase) precipitate at 25°C. In addition, unless otherwise noted, the proportion of the compound to the base liquid crystal is 10% by weight : 90% by weight.

As the base liquid crystal, base liquid crystal (i) described below was used. A proportion of each component was expressed in terms of % by weight.

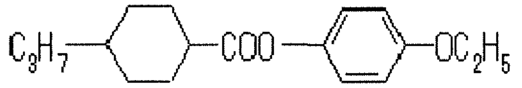 17. 2wt%

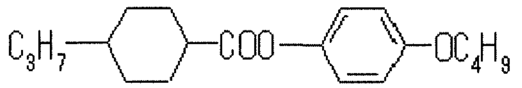 27.6wt%

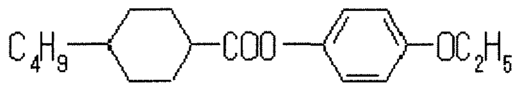 20.7wt%

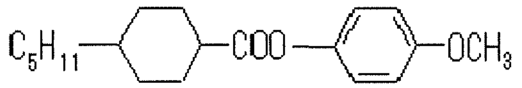 20.7wt%

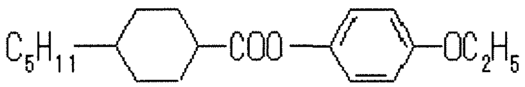 13.8wt%

Measuring method

Measurement of characteristics was carried out by the methods described below. Most of the measuring methods are described in the Standard of Japan Electronics and Information Technology Industries Association (hereinafter, abbreviated as JEITA) (JEITA ED-2521B) discussed and established in JEITA or modified thereon. No thin film transistor (TFT) was attached to a TN device used for measurement.

(1) Phase structure

A sample was placed on a hot plate of a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.

(2) Transition temperature (°C)

A sample was heated and then cooled at a rate of 3°C per minute, and a starting point of an endothermic peak or an exothermic peak caused by a phase change of the sample was determined by extrapolation, and thus a transition temperature was determined by using Scanning Calorimeter DSC-7 System or Diamond DSC System made by PerkinElmer, Inc. A melting point and a polymerization start temperature of a compound were also measured using the apparatus. Temperature at which the compound undergoes transition from a solid to a liquid crystal phase such as a

smectic phase and a nematic phase may be occasionally abbreviated as "minimum temperature of the liquid crystal phase." Temperature at which the compound undergoes transition from the liquid crystal phase to liquid may be occasionally abbreviated as "clearing point."

A crystal was expressed as C. When kinds of the crystals were distinguishable, each of the crystals was expressed as $C_1$ or $C_2$. The smectic phase or the nematic phase was expressed as S or N. When a smectic A phase, a smectic B phase, a smectic C phase or a smectic F phase was distinguishable among the smectic phases, the phases were expressed as $S_A$, $S_B$, $S_C$ or $S_F$, respectively. A liquid (isotropic) was expressed as I. The transition temperature was expressed as "C 50.0 N 100.0 I, " for example. The expression indicates that the transition temperature from the crystal to the nematic phase was 50.0°C, and the transition temperature from the nematic phase to the liquid was 100.0°C.

(3) Compatibility at a low temperature

Samples in which the base liquid crystal and the compound were mixed for the compound to be 20% by weight, 15% by weight, 10% by weight, 5% by weight, 3% by weight and 1% by weight were prepared, and placed in glass vials . After the glass vials were kept in freezers at -10°C or -20°C for a predetermined period of time, whether or not crystals (or the smectic phase) precipitated was observed.

(4) Maximum temperature of nematic phase ($T_{NI}$ or NI; °C)

A sample was placed on a hot plate of a melting point apparatus equipped with a polarizing microscope, and heated at a rate of 1°C per minute. Temperature when a part of the sample began to change from a nematic phase to an isotropic liquid was measured. A maximum temperature of the nematic phase may be occasionally abbreviated as "maximum temperature." When the sample was a mixture of a compound and a base liquid crystal, the maximum temperature was expressed using a symbol $T_{NI}$. When the sample was a mixture of the compound and component (B) or the like, the maximum temperature was expressed using a symbol NI.

(5) Minimum temperature of nematic phase ($T_c$; °C)

Samples each having a nematic phase were put in glass vials and kept in freezers at temperatures of 0°C, -10°C, -20°C, -30°C and -40°C for 10 days, and then liquid crystal phases were observed. For example, when the sample maintained the nematic phase at -20°C and changed to crystals or a smectic phase at -30°C, $T_c$ was expressed as $T_c$ < -20°C. A minimum temperature of the nematic phase may be occasionally abbreviated as "minimum temperature."

(6) Viscosity (bulk viscosity; η; measured at 20°C; mPa·s)

Measurement was carried out by using a cone-plate (E type) rotational viscometer.

(7) Viscosity (rotational viscosity; γ1; measured at 25°C; mPa·s)

Measurement was carried out according to a method described in M. Imai et al., Molecular Crystals and Liquid Crystals, Vol. 259, p. 37 (1995). A sample was put in a VA device in which a distance (cell gap) between two glass substrates was 20 micrometers. Voltage was applied stepwise to the device in the range of 39 V to 50 V at an increment of 1 V. After 0.2 second without voltage application, voltage was repeatedly applied under conditions of only one rectangular wave (rectangular pulse; 0.2 second) and no voltage application (2 seconds) . A peak current and a peak time of a transient current generated by the applied voltage were measured. A value of rotational viscosity was obtained from the measured values and calculation equation (8) on page 40 of the paper presented by M. Imai et al. As dielectric anisotropy required for the calculation, a value measured in the section of dielectric anisotropy described below was used.

(8) Optical anisotropy (refractive index anisotropy; measured at 25°C; Δn)

Measurement was carried out by an Abbe refractometer having a polarizing plate mounted on an ocular, using light at a wavelength of 589 nanometers. A surface of a main prism was rubbed in one direction, and then a sample was added dropwise onto the main prism. A refractive index (n||) was measured when a direction of polarized light was parallel to a direction of rubbing. A refractive index (n⊥) was measured when a direction of polarized light was perpendicular to a direction of rubbing. A value of optical anisotropy was calculated from an equation: $\Delta n = n_{||} - n_{\perp}$.

(9) Dielectric anisotropy ($\Delta\varepsilon$; measured at 25°C)

A value of dielectric anisotropy was calculated from an equation: $\Delta\varepsilon = \varepsilon|| - \varepsilon\perp$. Dielectric constants ($\varepsilon||$ and $\varepsilon\perp$) were measured as described below.

(1) Measurement of dielectric constant ($\varepsilon||$) : An ethanol solution (20 mL) of octadecyl triethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive. Sine waves (0.5 V, 1 kHz) were applied to the device, and after 2 seconds, a dielectric constant ($\varepsilon||$) in a major axis direction of liquid crystal molecules was measured.

(2) Measurement of dielectric constant ($\varepsilon\perp$): A polyimide solution was applied to a well-cleaned glass substrate. After calcining the glass substrate, rubbing treatment was applied to the alignment film obtained. A sample was put in a TN device in which a distance (cell gap) between two glass substrates was 9 micrometers and a twist angle was 80 degrees. Sine waves (0.5 V, 1 kHz) were applied to the device, and after 2 seconds, a dielectric constant ($\varepsilon\perp$) in a minor axis direction of liquid crystal molecules was measured.

(10) Elastic constant ($K_{11}$ and $K_{33}$; measured at 25°C; pN)

Elastic Constant Measurement System Model EC-1 made by TOYO Corporation was used for measurement. A sample was put in a vertical alignment device in which a distance (cell gap) between two glass substrates was 20 micrometers. An electric charge of 20 V to 0 V was applied to the device, and electrostatic capacity and an applied voltage were measured. Values of electrostatic capacity (C) and applied voltage (V) were fitted to equation (2.98) and equation (2.101) on page 75 of the "Liquid Crystal Device Handbook (Ekisho Debaisu Handobukku, in Japanese)" (The Nikkan Kogyo Shimbun, Ltd.), and a value of elastic constant was obtained from equation (2.100).

(11) Threshold voltage (Vth; measured at 25°C; V)

An LCD-5100 luminance meter made by Otsuka Electronics Co., Ltd. was used for measurement. A light source was a halogen lamp. A sample was put in a normally black mode VA device in which a distance (cell gap) between two glass substrates was 4 micrometers and a rubbing direction was anti-parallel, and the device was sealed with an ultraviolet-curable adhesive. A voltage (60 Hz, rectangular waves) to be applied to the device was stepwise increased from 0 V to 20 V at an increment of 0.02 V. On the occasion, the device was irradiated with light from a direction perpendicular to the device, and an amount of light transmitted through the device was measured. A voltage-transmittance curve was prepared, in which a maximum amount of light corresponds to 100% transmittance and a minimum amount of light corresponds to 0% transmittance. A threshold voltage was expressed in terms of a voltage at 10% transmittance.

(12) Voltage holding ratio (VHR-1; measured at 25°C; %)

A TN device used for measurement had a polyimide alignment film, and a distance (cell gap) between two glass substrates was 5 micrometers . A sample was put in the device, and the device was sealed with an ultraviolet-curable adhesive. The device was charged by applying a pulse voltage (60 microseconds at 5 V). A decaying voltage was measured for 16.7 milliseconds with a high-speed voltmeter, and area A between a voltage curve and a horizontal axis in a unit cycle was determined. Area B was an area without decay. A voltage holding ratio was expressed in terms of a percentage of area A to area B.

(13) Voltage holding ratio (VHR-2; measured at 80°C; %)

A voltage holding ratio (VHR-2) was measured by a method used in the measurement of VHR-1 except that the voltage holding ratio was measured at 80°C.

Raw material

SolmixA-11 (trade name) is a mixture of ethanol (85.5%), methanol (13.4%) and isopropanol (1.1%), and was purchased from Japan Alcohol Trading Co., Ltd.

**Example 1**

Synthesis of compound (No. 1-2-5)

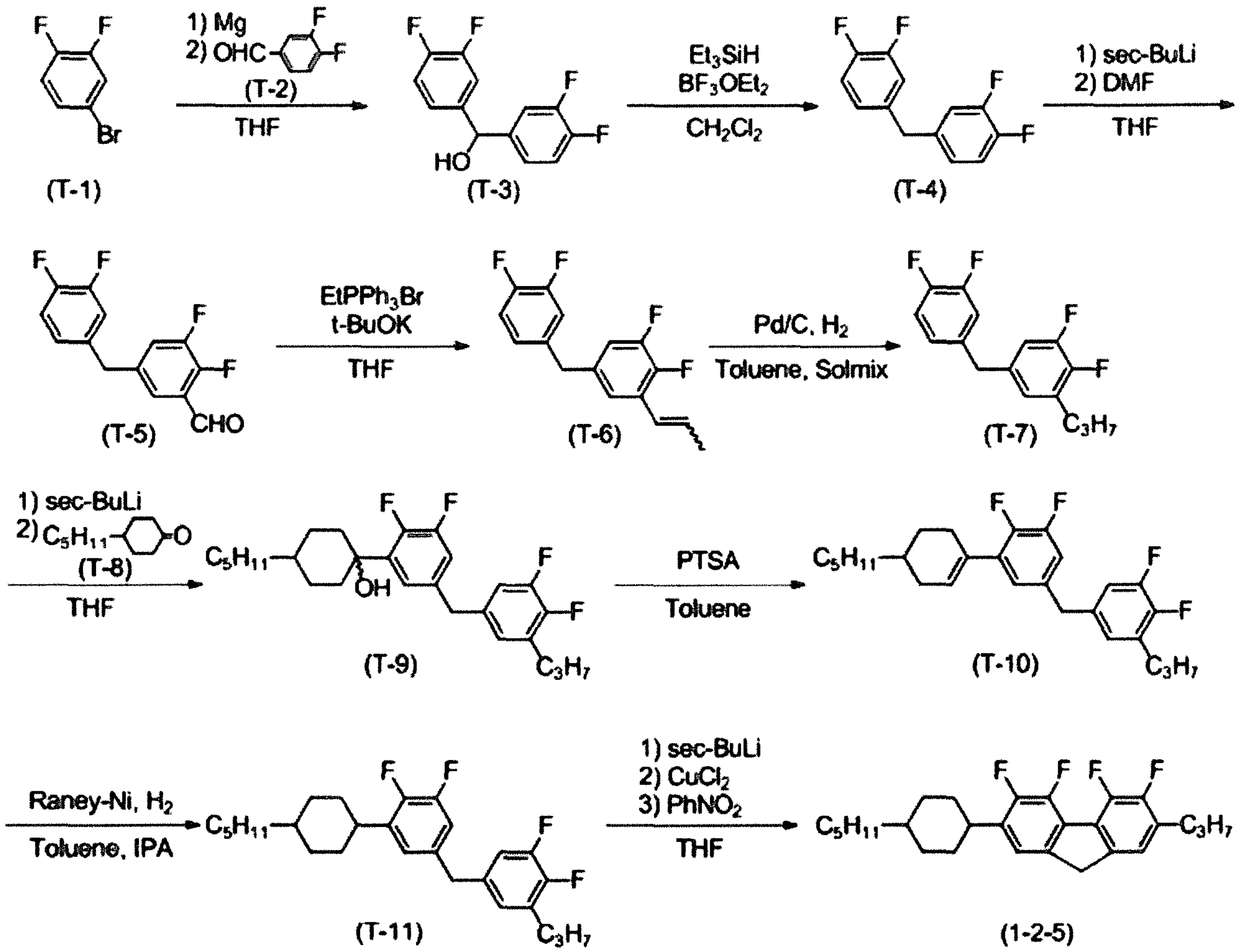


First step

Under a nitrogen atmosphere, magnesium (20.8 g) and THF (30.0 mL) were put in a reaction vessel, and the resulting mixture was heated at 45°C. A THF (150 mL) solution of compound (T-1) (150 g) was slowly added thereto in the temperature range of 45°C to 55°C, and the resulting mixture was stirred for 2 hours. Next, the resulting mixture was cooled to 0°C and a THF (200 mL) solution of compound (T-2) (127 g) was slowly added thereto, and the resulting mixture was stirred for 2 hours while returning to room temperature. The reaction mixture was poured into a saturated aqueous solution of ammonium chloride, and the aqueous layer was subjected to extraction with ethyl acetate. The combined organic layer was washed with water, and then dried over anhydrous magnesium sulfate. The solution was concentrated under reduced pressure, and the residue was purified by silica gel chromatography to obtain compound (T-3) (188 g; 95%).

Second step

Under a nitrogen atmosphere, compound (T-3) (188 g) and dichloromethane (750 mL) were put in a reaction vessel, and the resulting mixture was cooled to -50°C. Triethyl silane (140 mL) and a boron trifluoride-diethylether complex (205 mL) were slowly added thereto, and the resulting mixture was stirred for 12 hours while returning to room temperature. The reaction mixture was poured into ice water, and the aqueous layer was subjected to extraction with dichloromethane. The combined organic layer was washed with water, and then dried over anhydrous magnesium sulfate. The solution was concentrated under reduced pressure, and the residue was purified by silica gel chromatography

to obtain compound (T-4) (145 g; 82%)

Third step

Under a nitrogen atmosphere, compound (T-4) (120 g) and THF (1200 mL) were put in a reaction vessel, and the resulting mixture was cooled to -70°C. Then, sec-butyllithium (1.01 M; cyclohexane, n-hexane solution; 500 mL) were slowly added thereto, and the resulting mixture was stirred for 1 hour. Next, DMF (77.4 mL) was slowly added thereto, and the resulting mixture was stirred for 12 hours while returning to room temperature. The reaction mixture was poured into a saturated aqueous solution of ammonium chloride, and the aqueous layer was subjected to extraction with toluene. The combined organic layer was washed with brine, and then dried over anhydrous magnesium sulfate. The solution was concentrated under reduced pressure, and the residue was purified by silica gel chromatography (heptane : toluene = 1 : 1 in a volume ratio) to obtain compound (T-5) (57.2 g; 43%).

Fourth step

Under a nitrogen atmosphere, ethyltriphenylphosphonium bromide (26 g) and THF (850 mL) were put in a reaction vessel, and the resulting mixture was cooled to -30°C. Potassium t-butoxide (82.8 g) was slowly added thereto, and the resulting mixture was stirred for 30 minutes. Next, a THF (800 mL) solution of compound (T-5) (165 g) was slowly added thereto, and the resulting mixture was stirred for 2 hours while returning to room temperature. The reaction mixture was poured into water, and the aqueous layer was subjected to extraction with toluene. The combined organic layer was washed with brine, and then dried over anhydrous magnesium sulfate. The solution was concentrated under reduced pressure, and the residue was purified by silica gel chromatography (heptane) to obtain compound (T-6) (172 g; 100%).

Fifth step

In a reaction vessel, compound (T-6) (172 g), a palladium on carbon catalyst (NX type of 5% Pd/C (50% wet); 8.62 g; made by N.E. Chemcat Corporation) and Solmix (registered tradename) A-11 (500 mL) were put, and the resulting mixture was stirred for 12 hours. After removing the catalyst by filtrating the resulting material, the solution was concentrated under reduced pressure, and the residue was purified by silica gel chromatography (heptane) to obtain compound (T-7) (170 g; 98%).

Sixth step

Under a nitrogen atmosphere, compound (T-7) (135 g) and THF (1000 mL) were put in a reaction vessel, and the resulting mixture was cooled to -70°C. Then, sec-butyllithium (1.01 M; cyclohexane, n-hexane solution; 500 mL) was slowly added thereto, and the resulting mixture was stirred for 1 hour. Next, a THF (350 mL) solution of compound (T-8) (96.6 g) was slowly added thereto, and the resulting mixture was stirred for 3 hours while returning to room temperature. The reaction mixture was poured into a saturated aqueous solution of ammonium chloride, and the aqueous layer was subjected to extraction with ethyl acetate. The combined organic layer was washed with brine, and then dried over anhydrous magnesium sulfate. The solution was concentrated under reduced pressure to obtain compound (T-9) (215 g; 100%).

Seventh step

Under a nitrogen atmosphere, compound (T-9) (215 g), PTSA (para-toluenesulfonic acid monohydrate) (6.47 g) and toluene (1000 mL) were put in a reaction vessel, and the resulting mixture was refluxed under heating for 1 hour while removing distilled-off water. The reaction mixture was washed with water, and dried over anhydrous magnesium sulfate. The solution was concentrated under reduced pressure, and the residue was purified by silica gel chromatography (heptane) to obtain compound (T-10) (244 g; 87%).

Eighth step

In a reaction vessel, compound (T-10) (244 g), Raney nickel (24.4 g), toluene (200 mL), and IPA (200 mL) were put, and the resulting mixture was stirred under a hydrogen atmosphere for 24 hours. After removing the catalyst by filtrating the resulting material, the solution was concentrated under reduced pressure, and the residue was purified by silica gel chromatography (heptane) to obtain compound (T-1) (236 g; 96%).

Ninth step

Under a nitrogen atmosphere, compound (T-11) (50.0 g) and THF (2500 mL) were put in a reaction vessel, and the resulting mixture was cooled to -70°C. Then, sec-butyllithium (1.07 M; cyclohexane, n-hexane solution; 237 mL) was slowly added thereto, and the resulting mixture was stirred for 2 hours. Next, copper(II) chloride (35.6 g) was added thereto, and the resulting mixture was stirred for 1 hour. Then, nitrobenzene (28.6 mL) was slowly added thereto, and the resulting mixture was stirred for 12 hours while returning to room temperature. The reaction mixture was poured into a saturated aqueous solution of ammonium chloride, and the aqueous layer was subjected to extraction with ethyl acetate. The combined organic layer was washed with brine, and then dried over anhydrous magnesium sulfate. The solution was concentrated under reduced pressure, and the residue was purified by silica gel chromatography (heptane), and further purified by recrystallization from a mixed solvent of heptane and IPA (1 : 1 in a volume ratio) to obtain compound (1-2-5) (7.83 g; 16%).

Chemical shift δ (ppm; $CDCl_3$): 7.10 (d, J = 5.4 Hz, 1H), 7.05 (d, J = 5.6 Hz, 1H), 3.85 (s, 2H), 2.89 (tt, J = 12.2 Hz, J = 3.0 Hz, 1H), 2.69 (t, J = 7.6 Hz, 2H), 1.95 - 1.85 (m, 4H), 1.73 - 1.63 (m, 2H), 1.55 - 1.44 (m, 2H), 1.38 - 1.20 (m, 9H), 1.17 - 1.05 (m, 2H), 0.98 (t, J = 7.3 Hz, 3H), 0.91 (t, J = 7.3 Hz, 3H).

Physical properties of compound (No. 1-2-5) are as described below.

Transition temperature: C 110 ($S_A$ 100) I.

Maximum temperature ($T_{NI}$) = 84.6°C; optical anisotropy (Δn) = 0.145; dielectric anisotropy (Δε) = -10.4; viscosity (η) = 117 mPa·s.

**Example 2**

Synthesis of compound (No. 1-2-6)

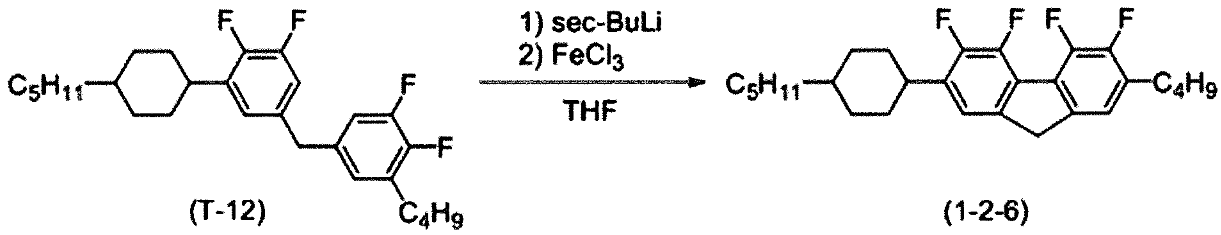


(T-12) (1-2-6)

First step

Under a nitrogen atmosphere, a compound (T-12) (196 g) synthesized in a manner similar to the technique in the seventh step in Example 1 and THF (5000 mL) were put in a reaction vessel, and the resulting mixture was cooled to -70°C. Then, sec-butyllithium (1.01 M; cyclohexane, n-hexane solution; 1000 mL) was slowly added thereto, and the resulting mixture was stirred for 3 hours. Next, iron(III) chloride (92.1 g) was added thereto, and the resulting mixture was stirred for 12 hours while returning to room temperature. The reaction mixture was poured into 1 N hydrochloric acid, and the aqueous layer was subjected to extraction with toluene. The combined organic layer was washed with brine, and then dried over anhydrous magnesium sulfate. The solution was concentrated under reduced pressure, and the residue was purified by silica gel chromatography (heptane), and further purified by recrystallization from heptane to obtain compound (1-2-6) (25.2 g; 13%).

Chemical shift δ (ppm; $CDCl_3$) : 7.09 (d, J = 5.4Hz, 1H), 7.05 (d, J = 5.6 Hz, 1H), 3.83 (s, 2H), 2.89 (tt, J = 12.2 Hz, J = 3.0 Hz, 1H), 2.70 (t, J = 7.7 Hz, 2H), 1.95 - 1.85 (m, 4H), 1.68 - 1.58 (m, 2H), 1.55 - 1.44 (m, 2H), 1.44 - 1.20 (m, 11H), 1.16 - 1.05 (m, 2H), 0.98 (t, J = 7.3Hz, 3H), 0.91 (t, J = 7.3Hz, 3H).

Physical properties of compound (No. 1-2-6) were described as follows.

Transition temperature: C 108 ($S_A$ 102) I.

Maximum temperature ($T_{NI}$) = 80.6°C; optical anisotropy (Δn) = 0.141; dielectric anisotropy (Δε) = -9.79; viscosity (η) = 120 mPa·s.

**Example 3**

Synthesis of compound (No. 1-2-15)

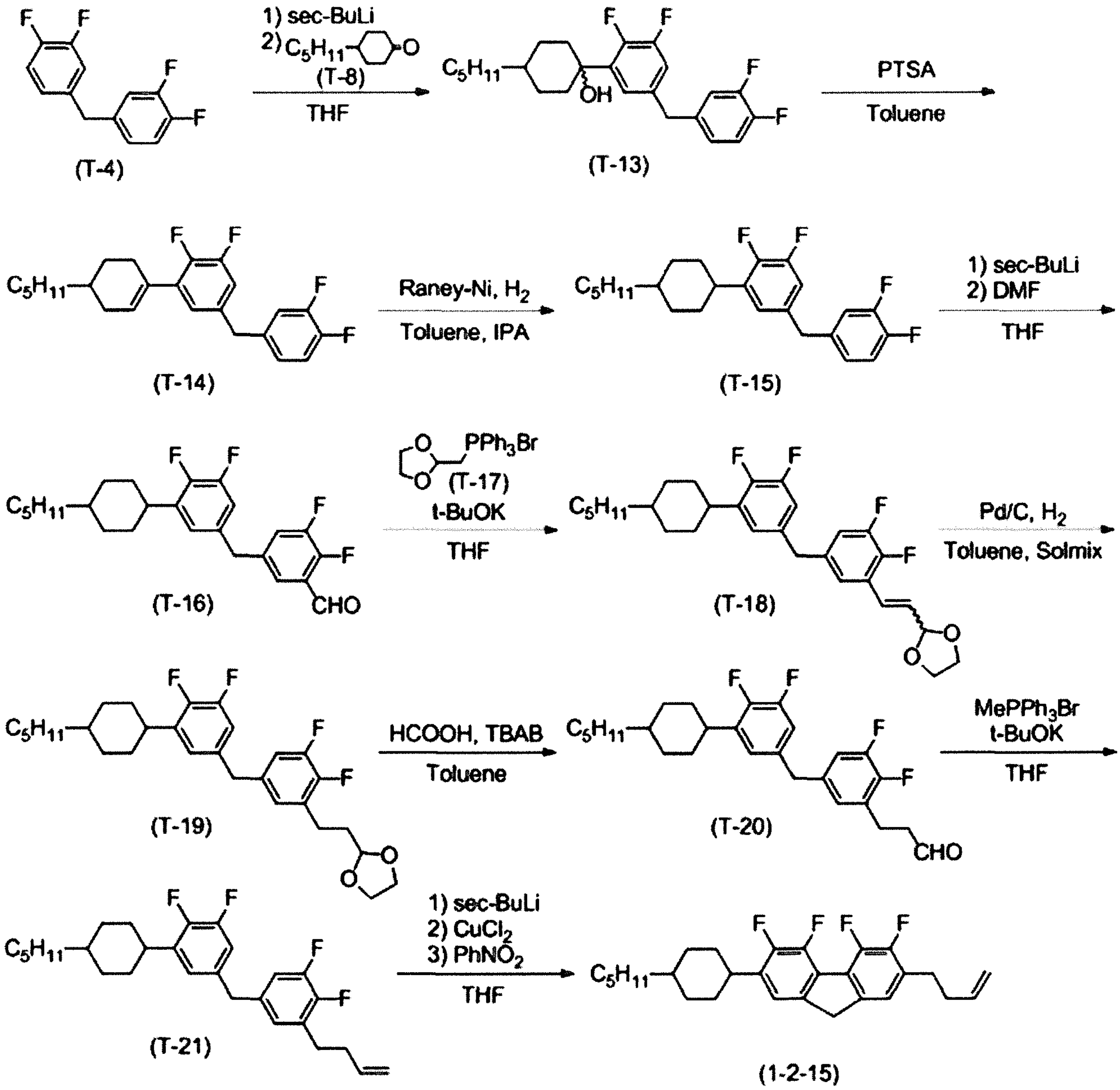


(T-4) (T-13) (T-14) (T-15) (T-16) (T-18) (T-19) (T-20) (T-21) (1-2-15)

First step

Under a nitrogen atmosphere, compound (T-4) (50.0 g) and THF (900 mL) were put in a reaction vessel, and the resulting mixture was cooled to -70°C. Then, sec-butyllithium (0.97 M; cyclohexane, n-hexane solution; 215 mL) was slowly added thereto, and the resulting mixture was stirred for 1 hour. Next, a THF (100 mL) solution of compound (T-8) (42.0 g) was slowly added thereto, and the resulting mixture was stirred for 12 hours while returning to room temperature. The reaction mixture was poured into a saturated aqueous solution of ammonium chloride, and the aqueous layer was subjected to extraction with ethyl acetate . The combined organic layer was washed with water, and dried over anhydrous magnesium sulfate. The solution was concentrated under reduced pressure, and the residue was purified by silica gel chromatography (toluene : ethyl acetate = 10 : 1 in a volume ratio) to obtain compound (T-13) (48.0 g; 56%).

Second step

Compound (T-14) (42.4 g; 93%) was obtained by using compound (T-13) (48.0 g) as a raw material in a manner similar to the technique in the seventh step in Example 1.

Third step

Compound (T-15) (42.0 g; 99%) was obtained by using compound (T-14) (42.4 g) as a raw material in a manner similar to the technique in the eighth step in Example 1.

Fourth step

Compound (T-16) (40.5 g; 90%) was obtained by using compound (T-15) (42.0 g) as a raw material in a manner similar to the technique in the third step in Example 1.

Fifth step

Under a nitrogen atmosphere, compound (T-17) (19.1 g) and THF (150 mL) were put in a reaction vessel, and the resulting mixture was cooled to -30°C. Then, potassium t-butoxide (4.80 g) was slowly added thereto, and the resulting mixture was stirred for 30 minutes. Next, a THF (150 mL) solution of compound (T-16) (15.0 g) was slowly added thereto, and the resulting mixture was stirred for 12 hours while returning to room temperature. The reaction mixture was poured into water, and the aqueous layer was subjected to extraction with toluene. The combined organic layer was washed with brine, and then dried over anhydrous magnesium sulfate. The solution was concentrated under reduced pressure, and the residue was purified by silica gel chromatography (toluene) to obtain compound (T-18) (17.5 g; 100%).

Sixth step

Compound (T-19) (17.1 g; 97%) was obtained by using compound (T-18) (17.5 g) as a raw material in a manner similar to the technique in the fifth step in Example 1.

Seventh step

Under a nitrogen atmosphere, compound (T-19) (17.1 g), formic acid (85.5 mL), TBAB (tetrabutylammonium bromide) (3.36 g) and toluene (170 mL) were put in a reaction vessel, and the resulting mixture was stirred for 12 hours. The reaction mixture was poured into water and the aqueous layer was subjected to extraction with toluene. The combined organic layer was washed with a saturated aqueous solution of sodium hydrogencarbonate and brine in the order, and then dried over anhydrous magnesium sulfate. The solution was concentrated under reduced pressure, and the residue was purified by silica gel chromatography (toluene) to obtain compound (T-20) (14.7 g; 94%).

Eighth step

Under a nitrogen atmosphere, methyltriphenylphosphonium bromide (14.6 g) and THF (110 mL) were put in a reaction vessel, and the resulting mixture was cooled to -30°C. Potassium t-butoxide (4.41 g) was slowly added thereto, and the resulting mixture was stirred for 30 minutes. Next, a THF (110 mL) solution of compound (T-20) (14.7 g) was slowly added thereto, and the resulting mixture was stirred for 12 hours while returning to room temperature. The reaction mixture was poured into water and the aqueous layer was subjected to extraction with toluene. The combined organic layer was washed with brine, and dried over anhydrous magnesium sulfate. The solution was concentrated under reduced pressure, and the residue was purified by silica gel chromatography (heptane) to obtain compound (T-21) (11.0 g; 75%).

Ninth step

Compound (No. 1-2-15) (1.11 g; 13%) was obtained by using compound (T-21) (8.89 g) as a raw material in a manner similar to the technique in the ninth step in Example 1.

Chemical shift δ (ppm; $CDCl_3$): 7.10 (d, J = 5.4 Hz, 1H), 7.06 (d, J = 5.5 Hz, 1H), 5.92 -5.80 (m, 1H), 5.09 - 4.98 (m, 2H), 3.85 (s, 2H), 2.89 (tt, J = 12.3 Hz , J = 3.0 Hz, 1H), 2.81 (t, J = 7.7 Hz, 2H), 2.45 - 2.36 (m, 2H), 1.95 -1.85 (m, 4H), 1.55 -1.44 (m, 2H), 1.38 - 1.20 (m, 9H), 1.17 - 1.06 (m, 2H), 0.90 (t, J = 7.2 Hz , 3H) .

Physical properties of compound (No. 1-2-15) were described as follows.

Transition temperature: C 111 ($S_A$ 102 N 103) I.

Maximum temperature ($T_{NI}$) = 90.6°C; optical anisotropy ($\Delta n$) = 0.150; dielectric anisotropy ($\Delta\varepsilon$) = -8.76; viscosity ($\eta$) = 105 mPa·s.

**Example 4**

Synthesis of compound (No. 1-2-41)

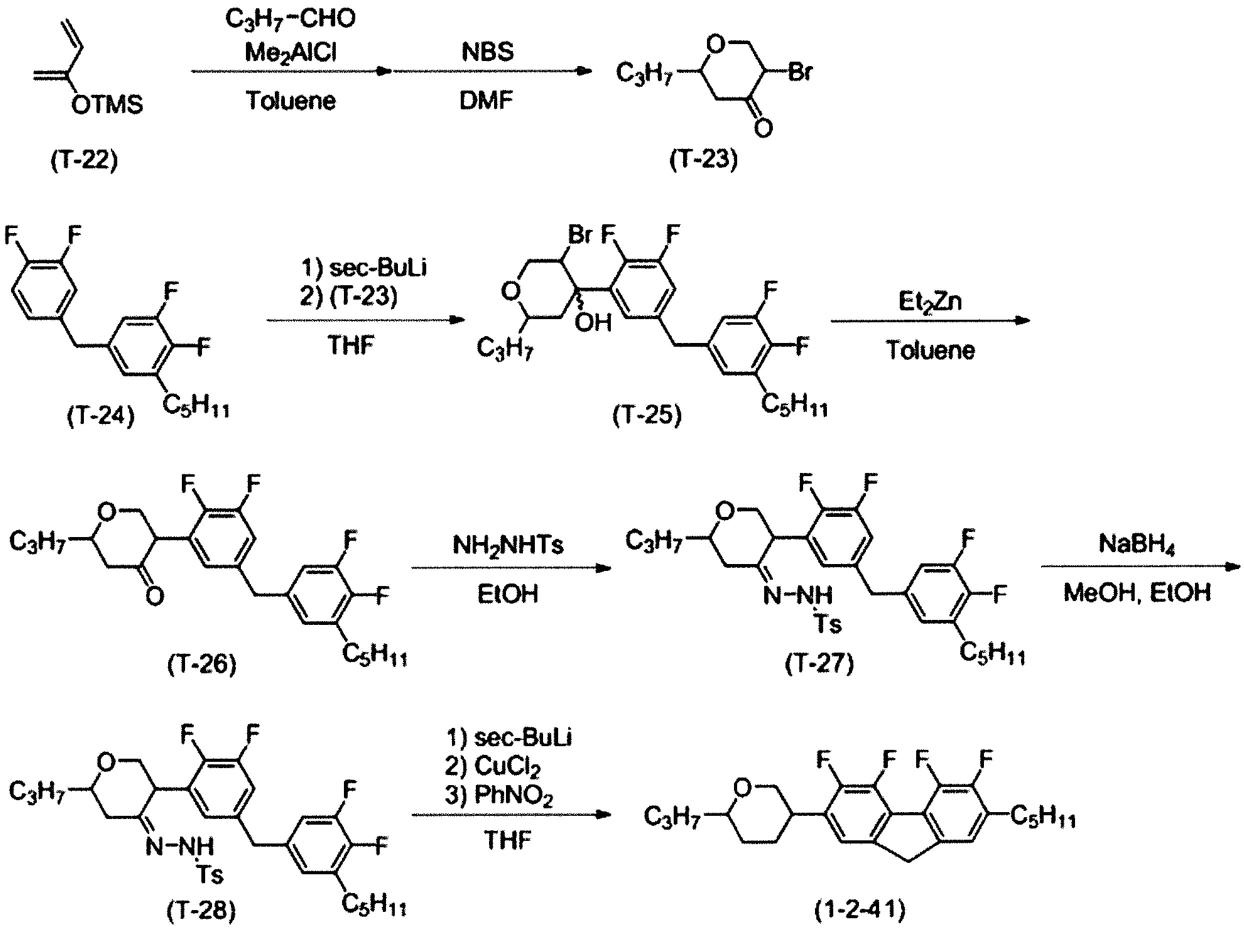


First step

Under a nitrogen atmosphere, butylaldehyde (9.45 g) and toluene (200 mL) were put in a reaction vessel, and the resulting mixture was cooled to -70°C. Dimethylaluminum chloride (1.00 M; n-hexane solution; 19.7 mL) was added thereto, and the resulting mixture was stirred for 30 minutes. Next, compound (T-22) (25.0 mL) was slowly added thereto, and the resulting mixture was stirred for 3 hours. Next, the resulting mixture was heated at -40°C, and NBS (N-bromosuccinimide) (46.6 g) and DMF (150 mL) was added thereto, and the resulting mixture was stirred for 1 hour while returning to room temperature. The reaction mixture was poured into a saturated aqueous solution of ammonium chloride, and the aqueous layer was subjected to extraction with ethyl acetate. The combined organic layer was washed with brine, and dried over anhydrous magnesium sulfate. The solution was concentrated under reduced pressure, and the residue was purified by silica gel chromatography (heptane : toluene = 1 : 9 in a volume ratio) to obtain compound (T-23) (9.40 g; 33%).

Second step

Under a nitrogen atmosphere, compound (T-19) (17.1 g; 97%) synthesized in a manner similar to the technique in the fifth step in Example 1 and THF (100 mL) were put in a reaction vessel, and the resulting mixture was cooled to -70°C. Then, sec-butyllithium (1.01 M; cyclohexane, n-hexane solution; 21.8 mL) was slowly added thereto, and the

resulting mixture was stirred for 1 hour. Next, a THF (50.0 mL) solution of compound (T-23) (4.90 g) was slowly added thereto, and the resulting mixture was stirred for 3 hours while returning to room temperature. The reaction mixture was poured into a saturated aqueous solution of ammonium chloride, and the aqueous layer was subjected to extraction with ethyl acetate. The combined organic layer was washed with brine, and dried over anhydrous magnesium sulfate. The solution was concentrated under reduced pressure, and the residue was purified by silica gel chromatography (toluene) to obtain compound (T-25) (7.43 g; 69%).

Third step

Under a nitrogen atmosphere, compound (T-25) (7.43 g), diethylzinc (1.00 M; n-hexane solution; 9.79 mL) and toluene (110 mL) were put in a reaction vessel, and the resulting mixture was stirred at 75°C for 3 hours. The reaction mixture was poured into a saturated aqueous solution of ammonium chloride, and the aqueous layer was subjected to extraction with ethyl acetate. The combined organic layer was washed with brine, and dried over anhydrous magnesium sulfate. The solution was concentrated under reduced pressure, and the residue was purified by silica gel chromatography (heptane : ethyl acetate = 8 : 1 in a volume ratio) to obtain compound (T-26) (4.45 g; 71%).

Fourth step

Under a nitrogen atmosphere, compound (T-26) (4.45 g), p-tosyl hydrazide (2.21 g) and ethanol (110 mL) were put in a reaction vessel, and the resulting mixture was stirred at 60°C for 3 hours. The reaction mixture was concentrated under reduced pressure, and the residue was purified by silica gel chromatography (toluene : ethyl acetate = 20 : 1 in a volume ratio) to obtain compound (T-27) (4.33 g; 71%).

Fifth step

Under a nitrogen atmosphere, compound (T-27) (4.12 g), sodium borohydride (2.52 g), methanol (40.0 mL) and ethanol (80.0 mL) were put in a reaction vessel, and the resulting mixture was stirred at 75°C for 6 hours. The reaction mixture was poured into water, and the aqueous layer was subjected to extraction with ethyl acetate. The combined organic layer was washed with brine, and dried over anhydrous magnesium sulfate. The solution was concentrated under reduced pressure, and the residue was purified by silica gel chromatography (heptane : ethyl acetate = 10 : 1 in a volume ratio) to obtain compound (T-28) (1.25g; 43%).

Sixth step

Compound (No. 1-2-41) (0.36 g; 29%) was obtained by using compound (T-28) (1.25 g) as a raw material in a manner similar to the technique in the ninth step in Example 1.

Chemical shift $\delta$ (ppm; $CDCl_3$) : 7.09 - 7.04 (m, 2H), 4.08 - 4.02 (m, 1H), 3.86 (s, 2H), 3.47 (t, J = 11.1 Hz, 1H), 3.40 - 3.33 (m, 1H), 3.26 - 3.18 (m, 1H), 2.70 (t, J = 7.5 Hz, 2H), 2.07 - 2.00 (m, 1H), 1.88 - 1.76 (m, 2H), 1.68 - 1.55 (m, 3H), 1.55 - 1.30 (m, 8H), 0.98 - 0.87 (m, 6H).

Physical properties of compound (No.1-2-41) were described as follows. In addition, a mixture of the compound (5% by weight) and the base liquid crystal (95% by weight) was used as the sample in measuring a maximum temperature ($T_{NI}$), optical anisotropy ($\Delta n$) and dielectric anisotropy ($\Delta\varepsilon$).

Transition temperature: C 117 I.

Maximum temperature ($T_{NI}$) = 54.6°C; optical anisotropy ($\Delta n$) = 0.129; dielectric anisotropy ($\Delta\varepsilon$) = -11.4; viscosity ($\eta$) = 142 mPa·s.

**Example 5**

Synthesis of compound (No. 1-2-22)

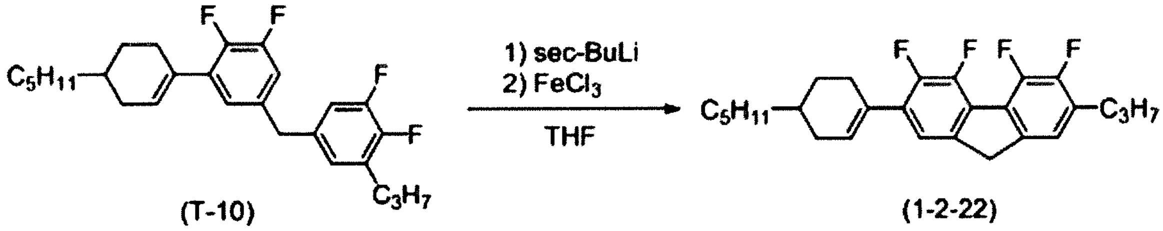


(T-10) (1-2-22)

First step

Compound (No.1-2-22) (4.43 g; 40%) was obtained by using compound (T-10) (10.0 g) as a raw material in a manner similar to the technique in the first step in Example 2.

Chemical shift $\delta$ (ppm; $CDCl_3$): 7.11 (d, J = 5.8 Hz, 1H), 7.05 (d, J = 5.6 Hz, 1H), 6.04 - 5.98 (m, 1H), 3.85 (s, 2H), 2.69 (t, J = 8.3 Hz, 2H), 2.54 - 2.29 (m, 3H), 1.94 - 1.80 (m, 2H), 1.73 - 1.58 (m, 3H), 1.43 - 1.25 (m, 9H), 0.98 (t, J = 7.4 Hz, 3H), 0.91 (t, J = 7.1 Hz, 3H).

Physical properties of compound (No. 1-2-22) were described as follows.

Transition temperature: C 111 $S_A$ 127 I.

Maximum temperature ($T_{NI}$) = 99.6°C; optical anisotropy ($\Delta n$) = 0.187; dielectric anisotropy ($\Delta\varepsilon$) = -10.0; viscosity ($\eta$) = 98.1 mPa·s.

**Example 6**

Synthesis of compound (No. 1-2-52)

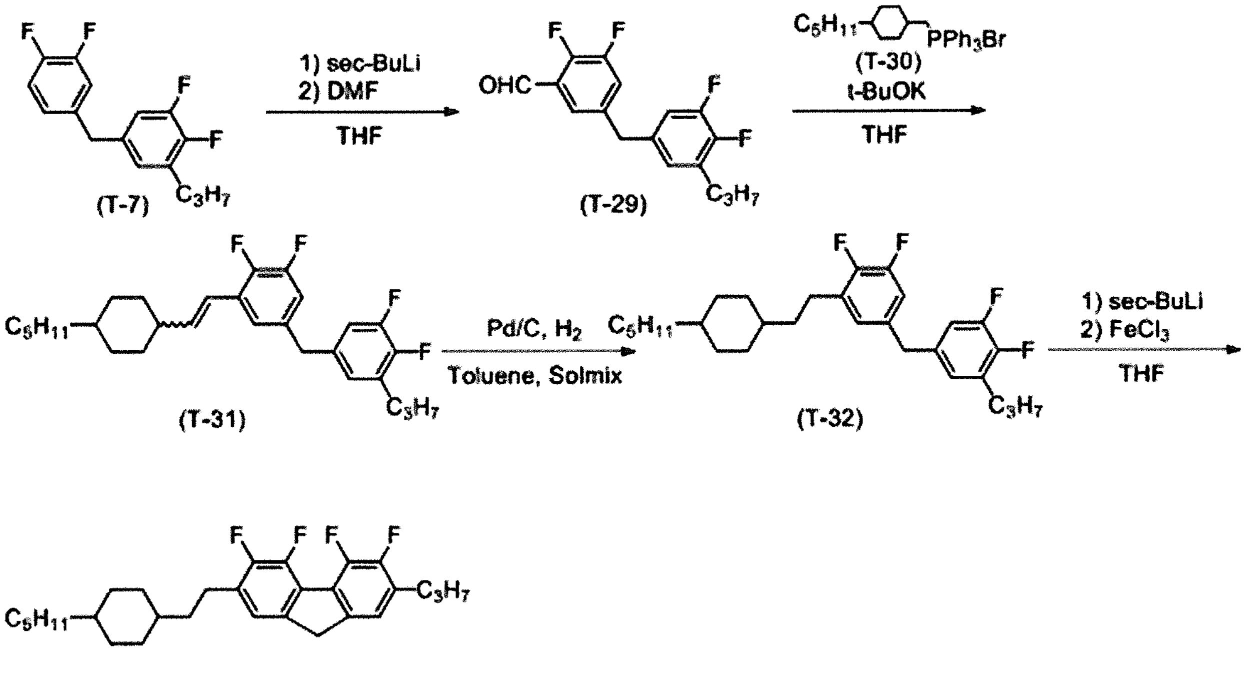


(T-7) (T-29) (T-31) (T-32) (1-2-52)

First step

Compound (T-29) (19.4 g; 85%) was obtained by using compound (T-7) (20.7 g) as a raw material in a manner similar to the technique in the third step in Example 1.

Second step

Under a nitrogen atmosphere, compound (T-30) (20.5 g) and THF (100 mL) were put in a reaction vessel, and the resulting mixture was cooled to -30°C. Potassium t-butoxide (4.34 g) was slowly added thereto, and the resulting mixture was stirred for 30 minutes. Next, a THF (100 mL) solution of compound (T-29) (10.0 g) was slowly added thereto, and the resulting mixture was stirred for 12 hours while returning to room temperature. The reaction mixture was poured into water and the aqueous layer was subjected to extraction with toluene. The combined organic layer was washed with brine, and dried over anhydrous magnesium sulfate. The solution was concentrated under reduced pressure, and the residue was purified by silica gel chromatography (heptane) to obtain compound (T-31) (9.23 g; 62%).

Third step

Compound (T-32) (8.61 g; 93%) was obtained by using compound (T-31) (9.23 g) as a raw material in a manner similar to the technique in the fifth step in Example 1.

Fourth step

Compound (No.1-2-52) (3.16 g; 37%) was obtained by using compound (T-32) (8.61 g) as a raw material in a manner similar to the technique in the first step in Example 2.

Chemical shift δ (ppm; $CDCl_3$): 7.05 (d, J = 5.6 Hz, 2H), 3.84 (s, 2H), 2.76 - 2.65 (m, 4H), 1.85 - 1.62 (m, 6H), 1.56 - 1.47 (m, 2H), 1.35 - 1.10 (m, 10H), 1.02 - 0.82 (m, 10H).

Physical properties of compound (No.1-2-52) were described as follows. In addition, a mixture of the compound (3% by weight) and the base liquid crystal (97% by weight) was used as the sample in measuring a maximum temperature ($T_{NI}$), optical anisotropy (Δn) and dielectric anisotropy (Δε).

Transition temperature: C 121 ($S_A$ 105 N 108) I.

Maximum temperature ($T_{NI}$) = 97.9°C; optical anisotropy (Δn) = 0.187; dielectric anisotropy (Δε) = -11.1; viscosity (η) = 98.8 mPa·s.

**Example 7**

Synthesis of compound (No. 1-2-31)

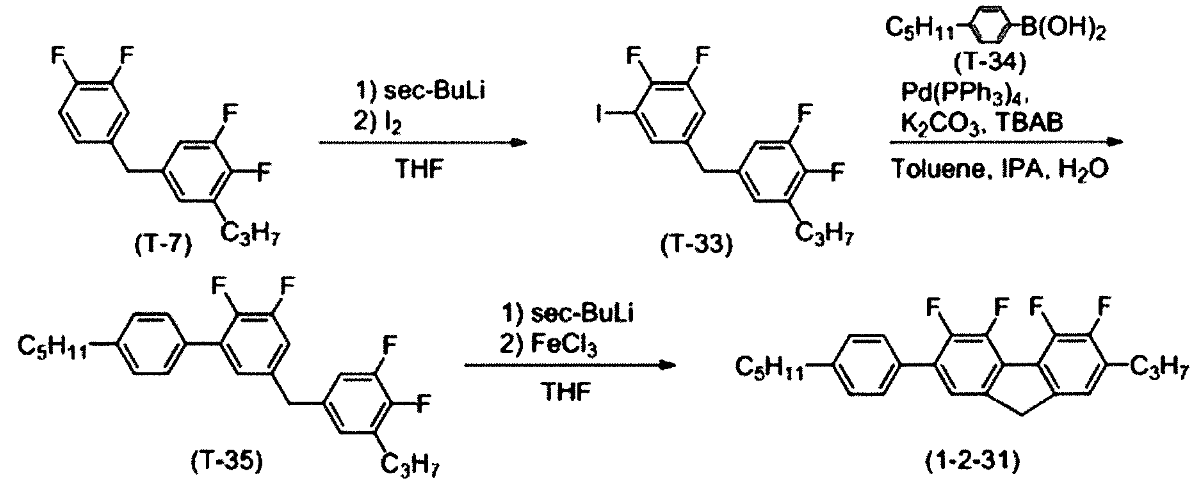


First step

Under a nitrogen atmosphere, compound (T-7) (17.7 g) and THF (255 mL) were put in a reaction vessel, and the resulting mixture was cooled to -70°C. Then, sec-butyllithium (1.03 M; cyclohexane, n-hexane solution; 63.9 mL) was slowly added thereto, and the resulting mixture was stirred for 1 hour. Next, a THF (100 mL) solution of iodine (19.1 g) was slowly added thereto, and the resulting mixture was stirred for 3 hours while returning to room temperature. The reaction mixture was poured into water, and the aqueous layer was subjected to extraction with toluene. The combined

organic layer was washed with an aqueous solution of sodium thiosulfate and water in the order, and dried over anhydrous magnesium sulfate. The solution was concentrated under reduced pressure, and the residue was purified by silica gel chromatography (heptane) to obtain compound (T-33) (25.3 g; 99%).

Second step

Under a nitrogen atmosphere, compound (T-33) (6.00 g), compound (T-34) (3.53 g), tetrakis(triphenylphosphine)palladium (0.170 g), potassium carbonate (4.06 g), TBAB (0.948 g), toluene (35.0 mL), IPA (35.0 mL) and water (35.0 mL) were put in a reaction vessel, and the resulting mixture was refluxed under heating for 3 hours. The reaction mixture was poured into water, and the aqueous layer was subjected to extraction with toluene. The combined organic layer was washed with water, and dried over anhydrous magnesium sulfate. The solution was concentrated under reduced pressure, and the residue was purified by silica gel chromatography (heptane) to obtain compound (T-35) (4.70 g; 75%).

Third step

Compound (No.1-2-31) (1.84 g; 39%) was obtained by using compound (T-35) (4.70 g) as a raw material in a manner similar to the technique in the first step in Example 2.

Chemical shift δ (ppm; $CDCl_3$):7.50 (d, J = 8.0 Hz, 2H), 7.33 - 7.26 (m, 3H), 7.08 (d, J = 5.4 Hz, 1H), 3.92 (s, 2H), 2.74 - 2.61 (m, 4H), 1.75 - 1.61 (m, 4H), 1.43 - 1.30 (m, 4H), 0.99 (t, J = 7.2 Hz, 3H), 0.91 (t, J = 6.7 Hz, 3H).

Physical properties of compound (No. 1-2-31) were described as follows.

Transition temperature: C 97.1 $S_A$ 132 I.

Maximum temperature ($T_{NI}$) = 89.6°C; optical anisotropy ($\Delta n$) = 0.227; dielectric anisotropy ($\Delta\varepsilon$) = -9.89; viscosity ($\eta$) = 103 mPa·s.

**Example 8**

Synthesis of compound (No. 1-1-3)

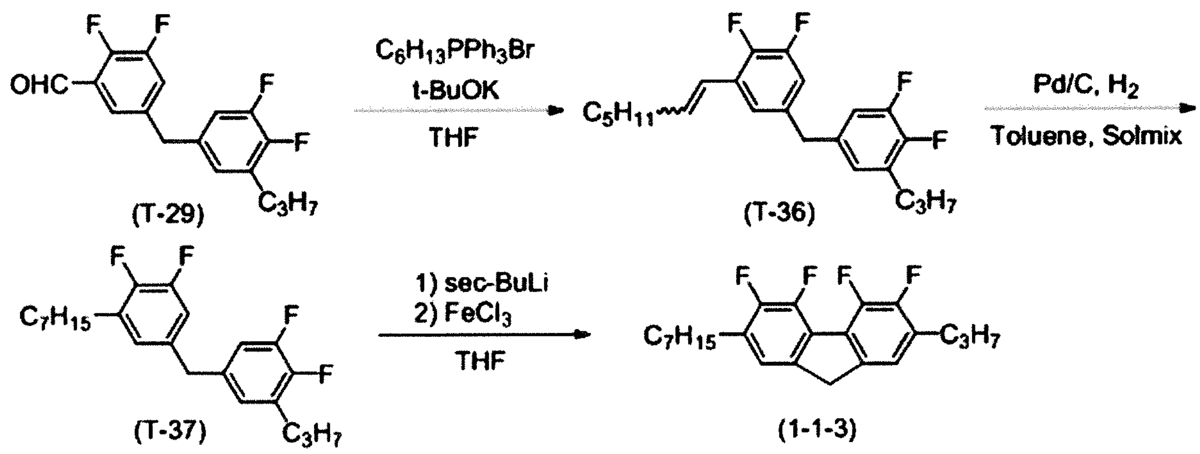


(T-29) (T-36) (T-37) (1-1-3)

First step

Under a nitrogen atmosphere, compound (T-36) (3.71 g; 88%) was obtained by using compound (T-29) (3.28 g) and n-hexyltriphenylphosphonium bromide (4.55 g) as a raw material in a manner similar to the technique in the second step in Example 6.

Second step

Compound (T-37) (3.20 g; 84%) was obtained by using compound (T-36) (3.71 g) as a raw material in a manner similar to the technique in the third step in Example 6.

Third step

Compound (No.1-1-3) (0.93 g; 29%) was obtained by using compound (T-37) (3.20 g) as a raw material in a manner similar to the technique in the first step in Example 2.

Chemical shift δ (ppm; $CDCl_3$): 7.05 (d, J = 5.6 Hz, 2H), 3.84 (s, 2H), 2.75 - 2.65 (m, 4H), 1.73 - 1.59 (m, 4H), 1.40 - 1.23 (m, 8H), 0.98 (t, J = 7.3 Hz, 3H), 0.88 (t, J = 7.0 Hz, 3H).

Physical properties of compound (No. 1-1-3) were described as follows.

Transition temperature: C 90.9 I.

Maximum temperature ($T_{NI}$) = -15.7°C; optical anisotropy (Δn) = 0.147; dielectric anisotropy (Δε) = -10.0; viscosity (η) = 73.2 mPa·s.

Compounds (1-1-1) to (1-1-15), compounds (1-2-1) to (1-2-80), compounds (1-3-1) to (1-3-40), or compounds (1-4-1) to (1-4-76) shown below can be synthesized according to the already-described methods for synthesizing compound (1), and the synthetic procedures described in Examples 1 to 6.

| No. | |
|---|---|
| 1-1-1 | 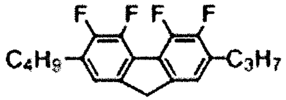 |
| 1-1-2 | 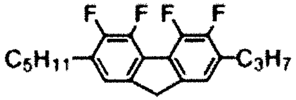 |
| 1-1-3 | 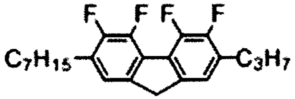<br>**C 90.9 I** |
| $T_{NI}$ = | -15.7 °C. $\Delta n$ = 0.147, $\Delta c$ = -10.0, $\eta$ = 73.2 mPas |
| 1-1-4 | 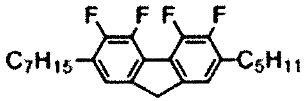 |
| 1-1-5 | 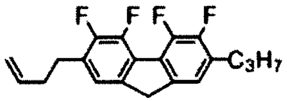 |
| 1-1-6 | 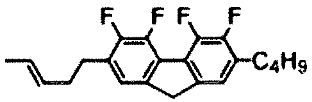 |
| 1-1-7 | 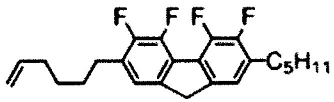 |
| 1-1-8 | 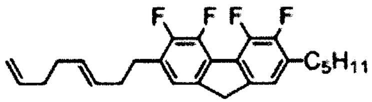 |
| 1-1-9 | 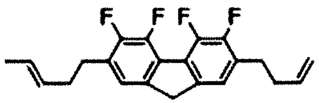 |

| No. | |
|---|---|
| 1-1-11 | 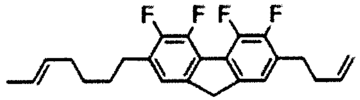 |
| 1-1-12 | 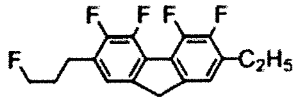 |
| 1-1-13 | 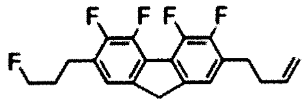 |
| 1-1-14 | 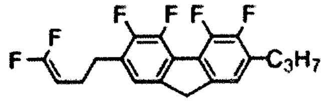 |
| 1-1-15 | 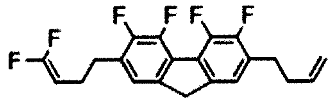 |

(continued)

| No. | | No. | |
|---|---|---|---|
| 1-1-10 | 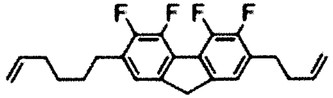 | | |
| 1-2-1 | 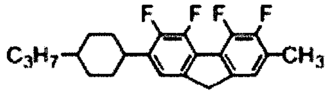 | 1-2-11 | 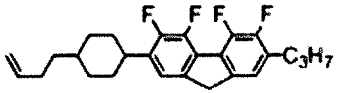 |
| 1-2-2 | 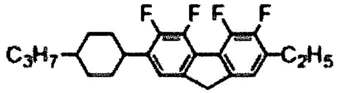 | 1-2-12 | 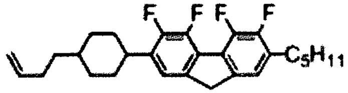 |
| 1-2-3 | 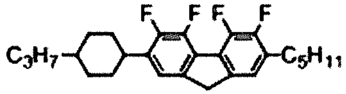 | 1-2-13 | 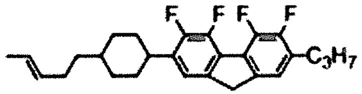 |
| 1-2-4 | 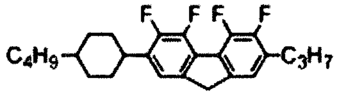 | 1-2-14 | 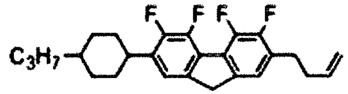 |
| 1-2-5 | 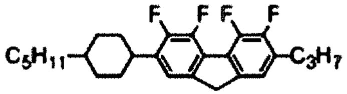 **C 110 ($S_A$ 100) I** $T_{NI}$ = 84.6 °C. Δn = 0.145. Δε = -10.4. η = 117 mPas | 1-2-15 | 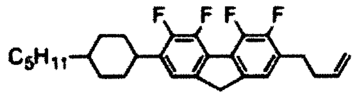 **C 111 ($S_A$ 102 N 103) I** $T_{NI}$ = 90.6 °C. Δn = 0.150. Δε = -8.76. η = 105 mPas |
| 1-2-6 | 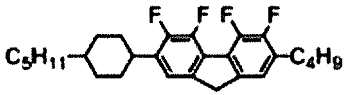 **C 108 ($S_A$ 102) I** $T_{NI}$ = 80.6 °C. Δn = 0.141. Δε = -9.79. η = 120 mPas | 1-2-16 | 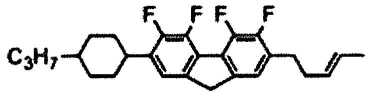 |
| 1-2-7 | 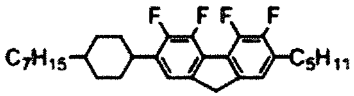 | 1-2-17 | 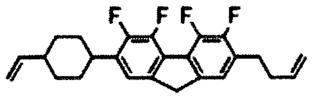 |
| 1-2-8 | 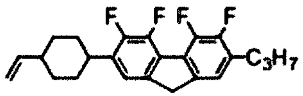 | 1-2-18 | 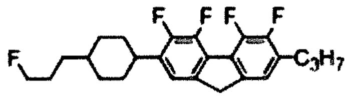 |

(continued)

| No. | | No. | |
|---|---|---|---|
| 1-2-9 | 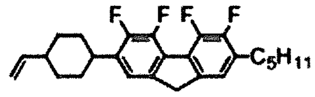 | 1-2-19 | 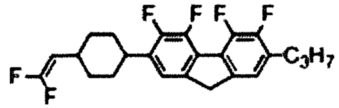 |
| 1-2-10 | 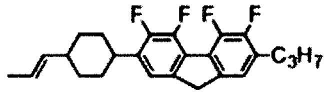 | 1-2-20 | 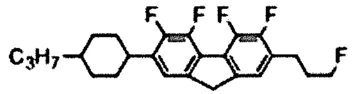 |
| 1-2-21 | 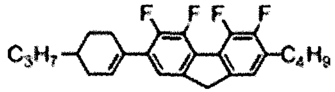 | 1-2-31 | 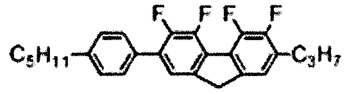<br>**C 97.1 $S_A$ 132 I**<br>$T_{NI}$ = 89.6 °C. Δn = 0.227. Δε = -9.89. η = 103 mPas |
| 1-2-22 | 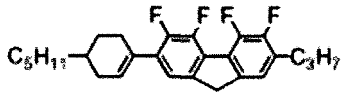<br>**C 111 $S_A$ 127 I**<br>$T_{NI}$ = 99.6 °C. Δn = 0.187. Δε = -10.0. η = 98.1 mPas | 1-2-32 | 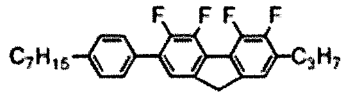 |
| 1-2-23 | 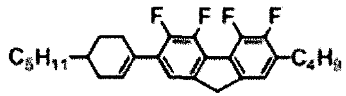 | 1-2-33 | 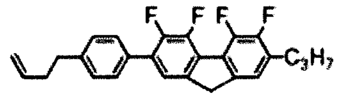 |
| 1-2-24 | 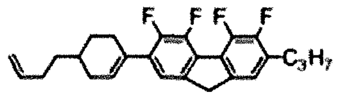 | 1-2-34 | 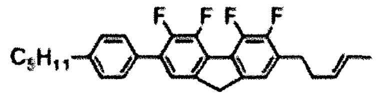 |
| 1-2-25 | 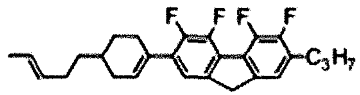 | 1-2-35 | 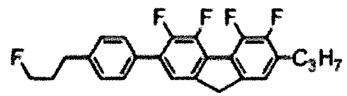 |
| 1-2-26 | 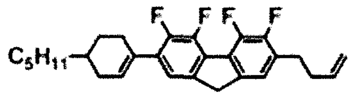 | 1-2-36 | 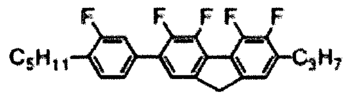 |
| 1-2-27 | 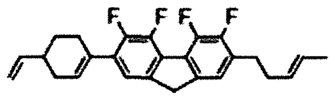 | 1-2-37 | 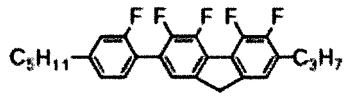 |

(continued)

| No. | | No. | |
|---|---|---|---|
| 1-2-28 | 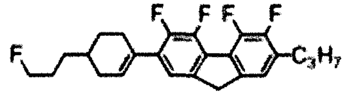 | 1-2-38 | 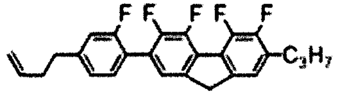 |
| 1-2-29 | 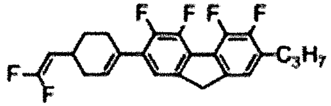 | 1-2-39 | 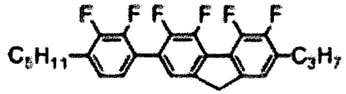 |
| 1-2-30 | 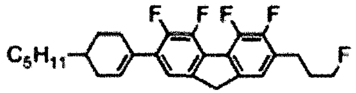 | 1-2-40 | 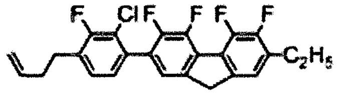 |
| 1-2-41 | 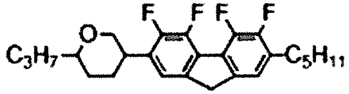<br>**C 117 I**<br>$T_{NI}$ = 54.6 °C. $\Delta n$ = 0.129, $\Delta\varepsilon$ = -11.4, $\eta$ = 142 mPas | 1-2-51 | 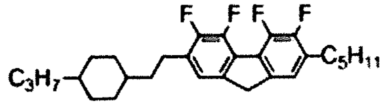 |
| 1-2-42 | 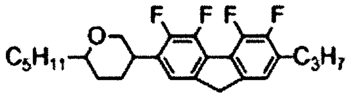 | 1-2-52 | 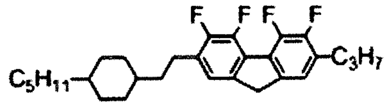<br>**C 121 ($S_A$ 105 N 108) I**<br>$T_{NI}$ = 97.9 °C. $\Delta n$ = 0.187. $\Delta\varepsilon$ = -11.1, $\eta$ = 98.8 mPas |
| 1-2-43 | 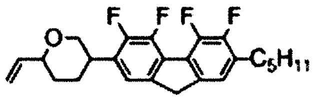 | 1-2-53 | 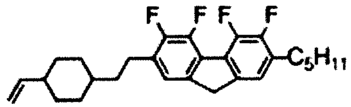 |
| 1-2-44 | 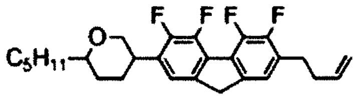 | 1-2-54 | 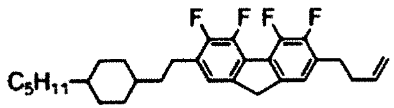 |
| 1-2-45 | 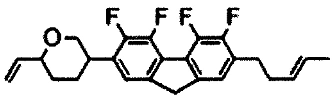 | 1-2-55 | 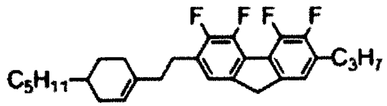 |

(continued)

| No. | | No. | |
|---|---|---|---|
| 1-2-46 | 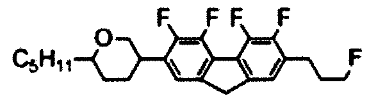 | 1-2-56 | 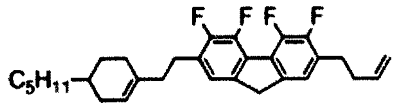 |
| 1-2-47 | 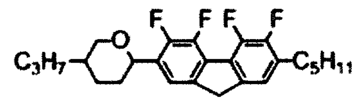 | 1-2-57 | 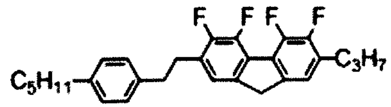 |
| 1-2-48 | 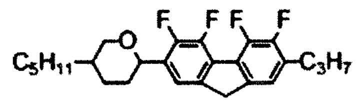 | 1-2-58 | 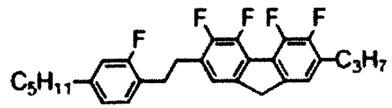 |
| 1-2-49 | 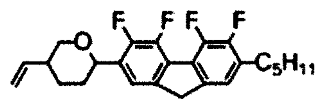 | 1-2-59 | 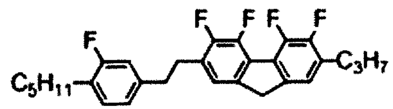 |
| 1-2-50 | 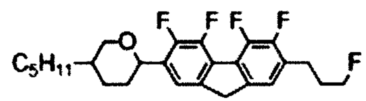 | 1-2-60 | 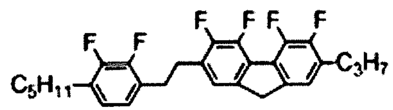 |
| 1-2-61 | 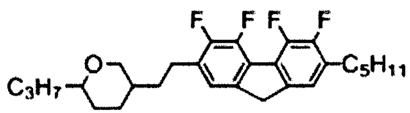 | 1-2-71 | 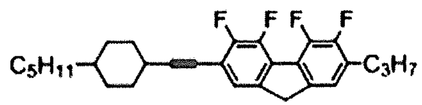 |
| 1-2-62 | 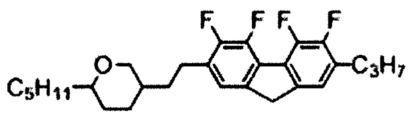 | 1-2-72 | 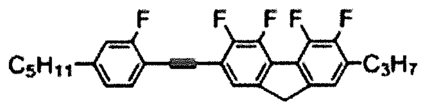 |
| 1-2-63 | 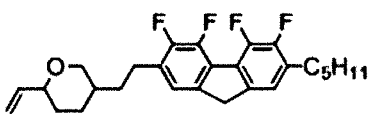 | 1-2-73 | 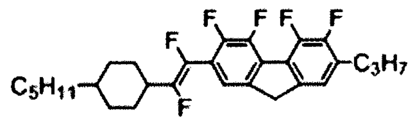 |
| 1-2-64 | 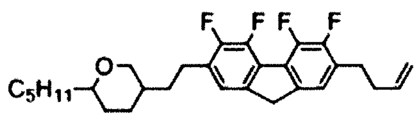 | 1-2-74 | 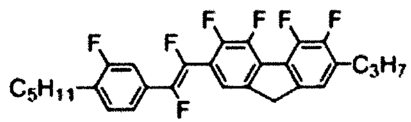 |

(continued)

| No. | | No. | |
|---|---|---|---|
| 1-2-65 | 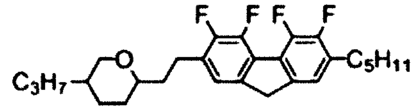 | 1-2-75 | 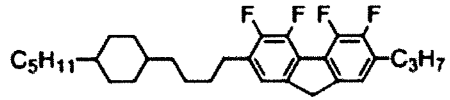 |
| 1-2-66 | 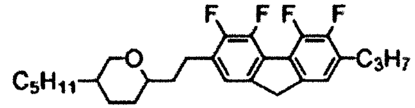 | 1-2-76 | 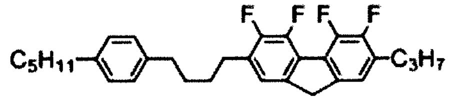 |
| 1-2-67 | 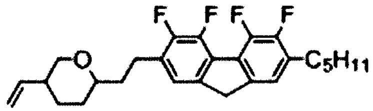 | 1-2-77 | 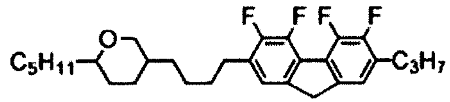 |
| 1-2-68 | 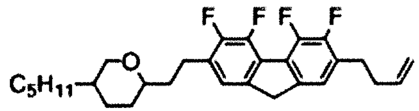 | 1-2-78 | 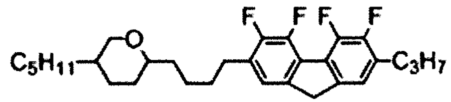 |
| 1-2-69 | 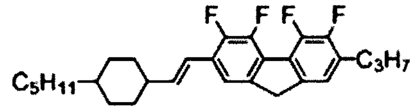 | 1-2-79 | 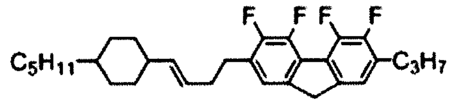 |
| 1-2-70 | 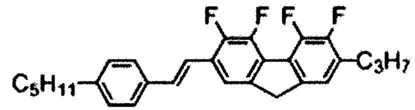 | 1-2-80 | 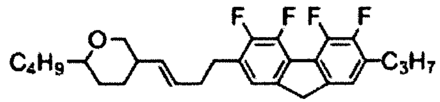 |
| 1-3-1 | 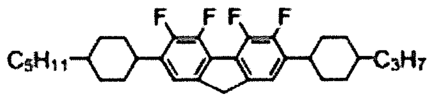 | 1-3-11 | 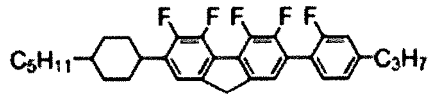 |
| 1-3-2 | 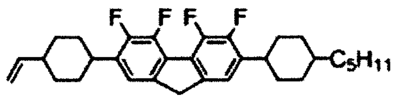 | 1-3-12 | 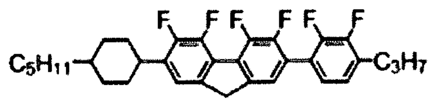 |
| 1-3-3 | 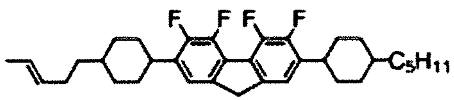 | 1-3-13 | 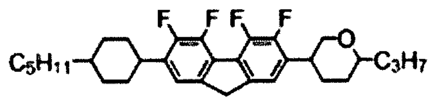 |

(continued)

| No. | | No. | |
|---|---|---|---|
| 1-3-4 | 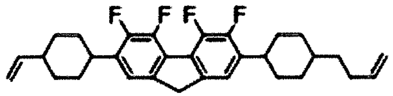 | 1-3-14 | 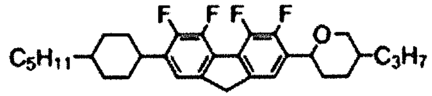 |
| 1-3-5 | 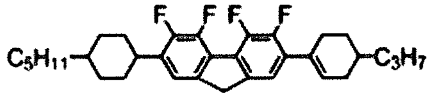 | 1-3-15 | 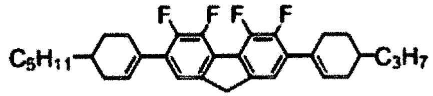 |
| 1-3-6 | 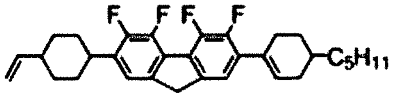 | 1-3-16 | 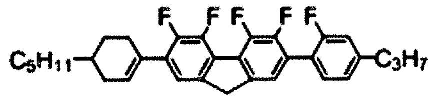 |
| 1-3-7 | 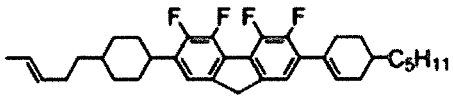 | 1-3-17 | 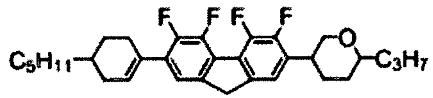 |
| 1-3-8 | 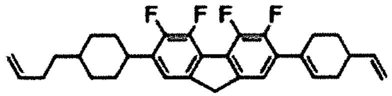 | 1-3-18 | 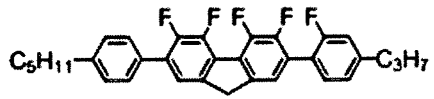 |
| 1-3-9 | 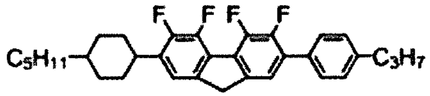 | 1-3-19 | 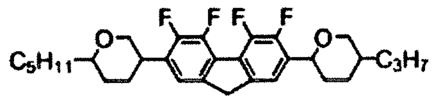 |
| 1-3-10 | 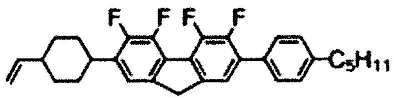 | 1-3-20 | 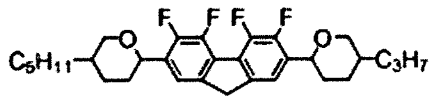 |
| 1-3-21 | 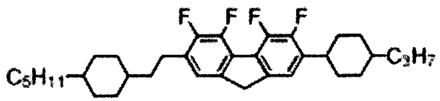 | 1-3-31 | 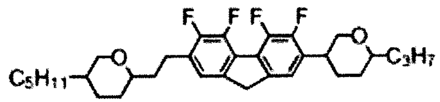 |
| 1-3-22 | 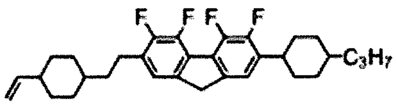 | 1-3-32 | 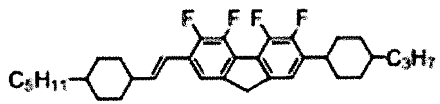 |
| 1-3-23 | 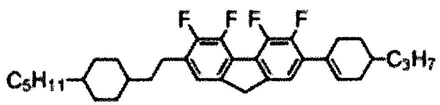 | 1-3-33 | 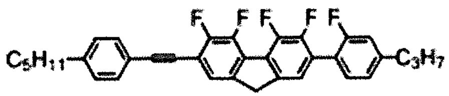 |

(continued)

| No. | | No. | |
|---|---|---|---|
| 1-3-24 | 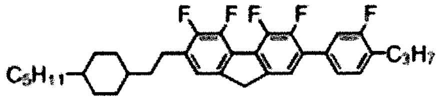 | 1-3-34 | 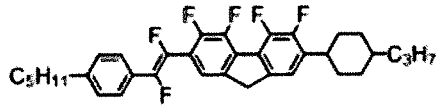 |
| 1-3-25 | 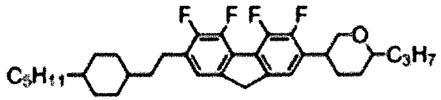 | 1-3-35 | 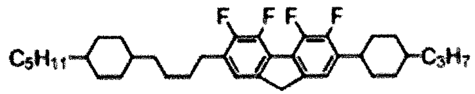 |
| 1-3-26 | 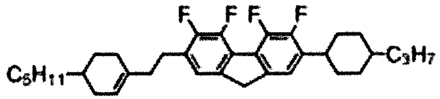 | 1-3-36 | 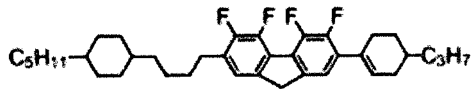 |
| 1-3-27 | 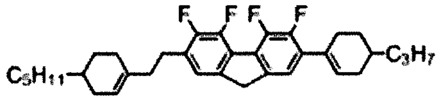 | 1-3-37 | 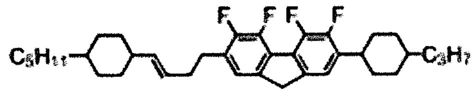 |
| 1-3-28 | 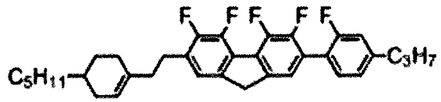 | 1-3-38 | 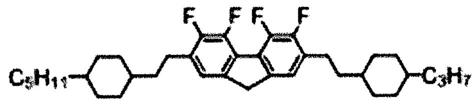 |
| 1-3-29 | 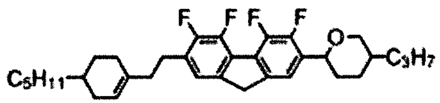 | 1-3-39 | 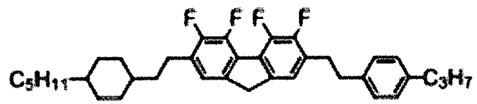 |
| 1-3-30 | 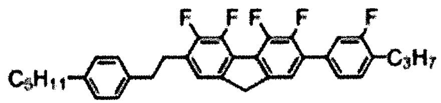 | 1-3-40 | 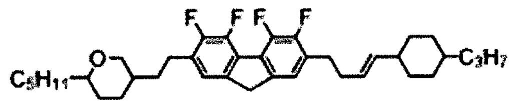 |
| 1-4-1 | 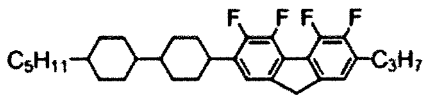 | 1-4-11 | 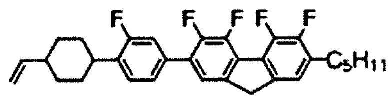 |
| 1-4-2 | 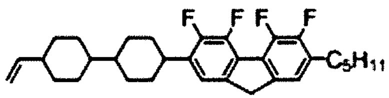 | 1-4-12 | 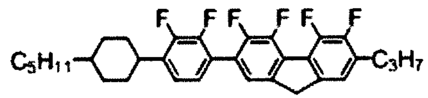 |
| 1-4-3 | 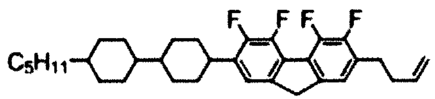 | 1-4-13 | 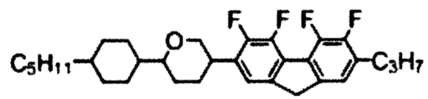 |

(continued)

| No. | | No. | |
|---|---|---|---|
| 1-4-4 | 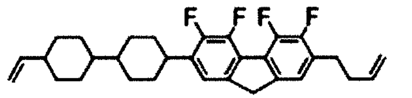 | 1-4-14 | 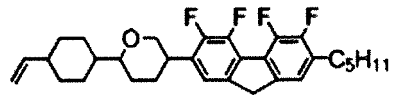 |
| 1-4-5 | 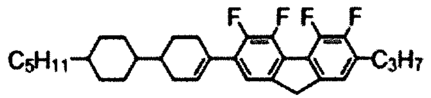 | 1-4-15 | 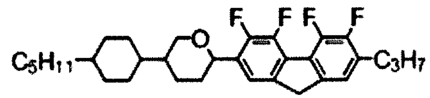 |
| 1-4-6 | 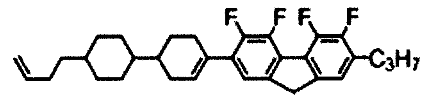 | 1-4-16 | 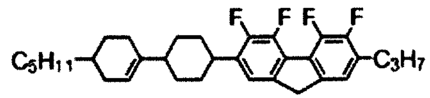 |
| 1-4-7 | 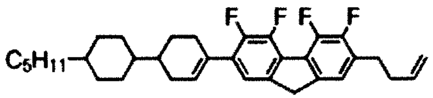 | 1-4-17 | 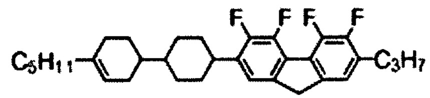 |
| 1-4-8 | 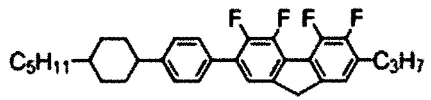 | 1-4-18 | 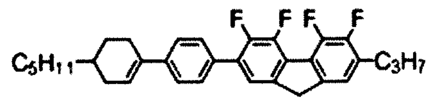 |
| 1-4-9 | 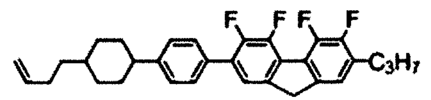 | 1-4-19 | 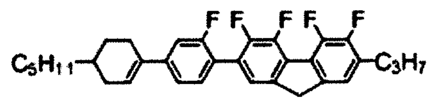 |
| 1-4-10 | 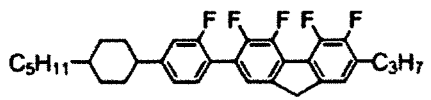 | 1-4-20 | 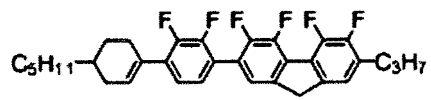 |
| 1-4-21 | 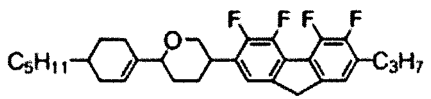 | 1-4-31 | 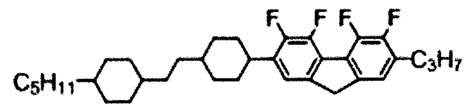 |
| 1-4-22 | 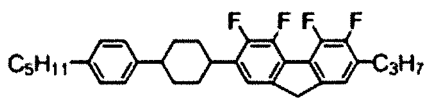 | 1-4-32 | 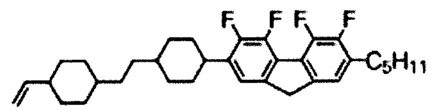 |
| 1-4-23 | 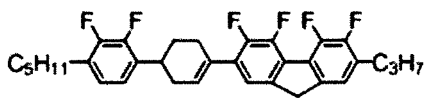 | 1-4-33 | 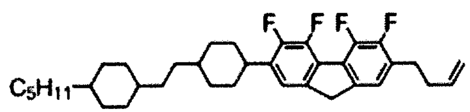 |

(continued)

| No. | | No. | |
|---|---|---|---|
| 1-4-24 | 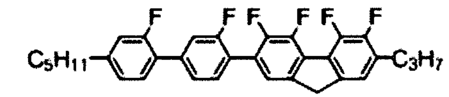 | 1-4-34 | 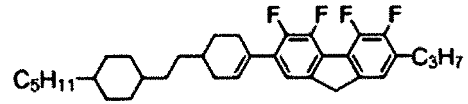 |
| 1-4-25 | 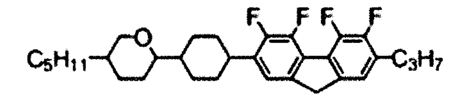 | 1-4-35 | 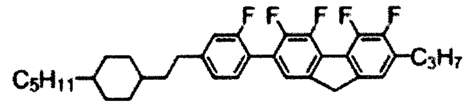 |
| 1-4-26 | 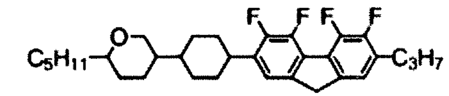 | 1-4-36 | 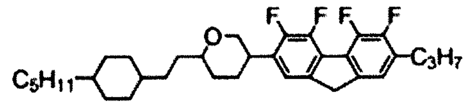 |
| 1-4-27 | 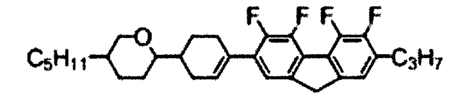 | 1-4-37 | 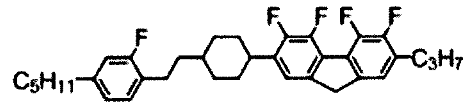 |
| 1-4-28 | 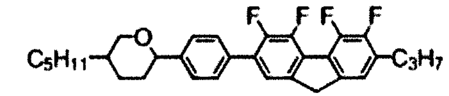 | 1-4-38 | 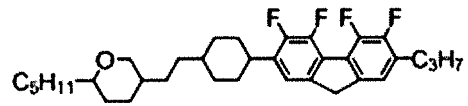 |
| 1-4-29 | 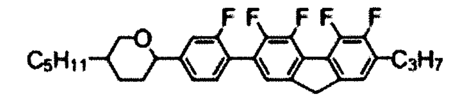 | 1-4-39 | 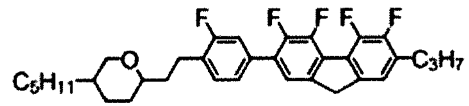 |
| 1-4-30 | 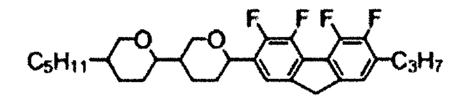 | 1-4-40 | 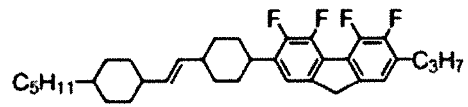 |
| 1-4-41 | 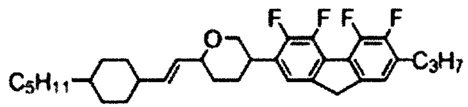 | 1-4-51 | 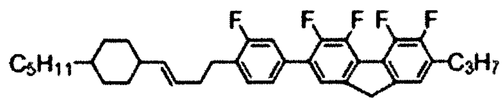 |
| 1-4-42 | 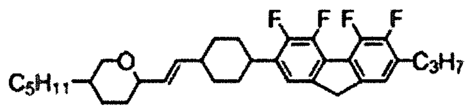 | 1-4-52 | 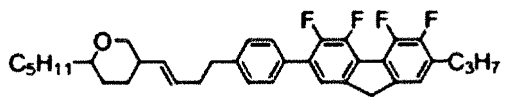 |

(continued)

| No. | | No. | |
|---|---|---|---|
| 1-4-43 | 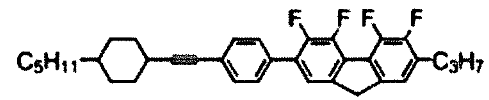 | 1-4-53 | 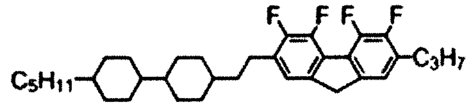 |
| 1-4-44 | 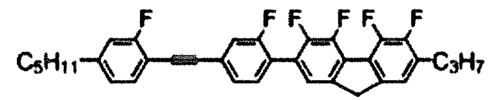 | 1-4-54 | 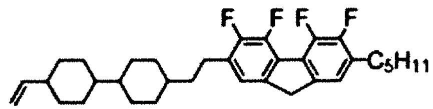 |
| 1-4-45 | 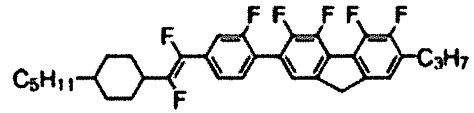 | 1-4-55 | 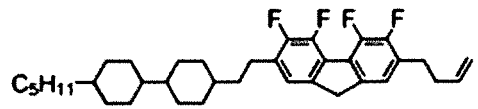 |
| 1-4-46 | 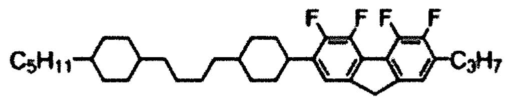 | 1-4-56 | 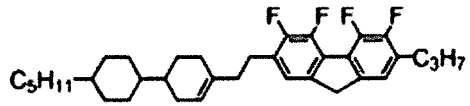 |
| 1-4-47 | 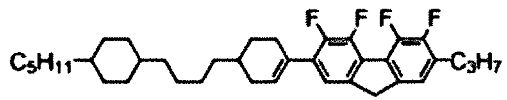 | 1-4-57 | 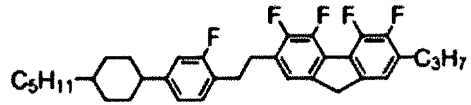 |
| 1-4-48 | 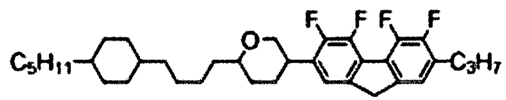 | 1-4-58 | 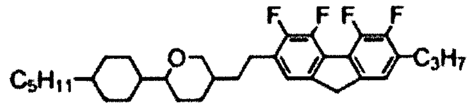 |
| 1-4-49 | 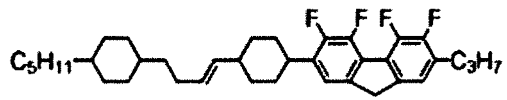 | 1-4-59 | 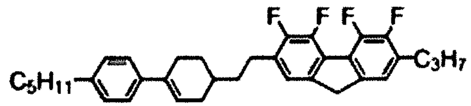 |
| 1-4-50 | 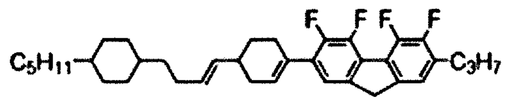 | 1-4-60 | 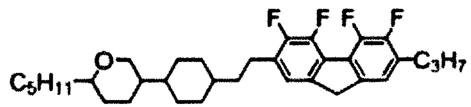 |
| 1-4-61 | 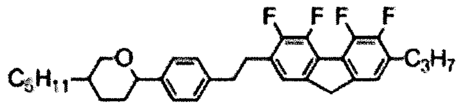 | 1-4-71 | 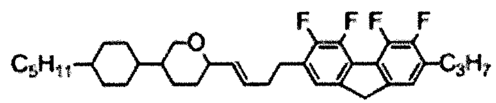 |

(continued)

| No. | | No. | |
|---|---|---|---|
| 1-4-62 | 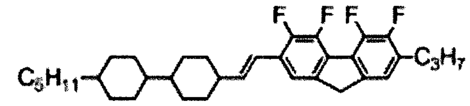 | 1-4-72 | 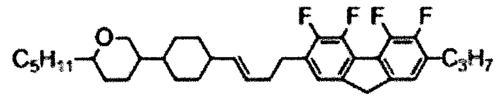 |
| 1-4-63 | 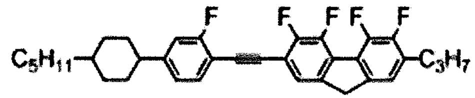 | 1-4-73 | 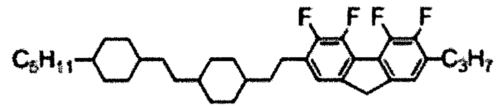 |
| 1-4-64 | 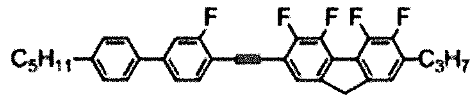 | 1-4-74 | 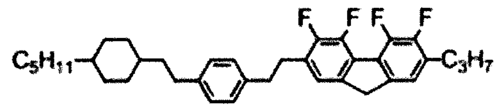 |
| 1-4-65 | 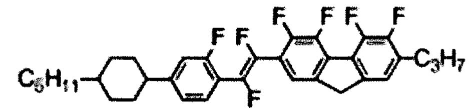 | 1-4-75 | 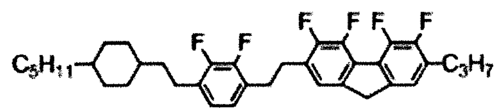 |
| 1-4-66 | 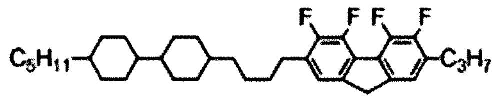 | 1-4-76 | 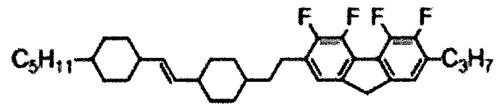 |
| 1-4-67 | 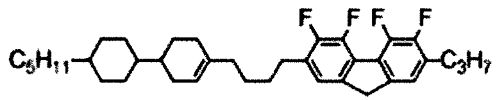 | | |
| 1-4-68 | 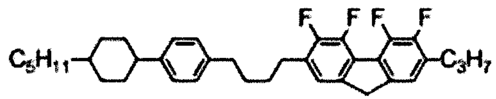 | | |
| 1-4-69 | 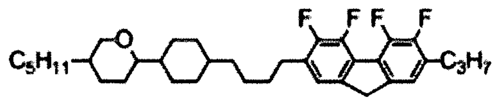 | | |
| 1-4-70 | 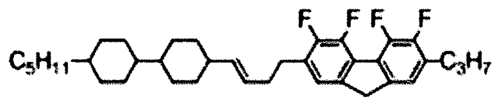 | | |

**Comparative Example 1**

In order to clarify an effect of the compound in which a bonding group has no oxygen atom according to the invention, compound (S-1-1) having a $-CH_2O-$ bonding group was synthesized as a comparative compound. The reason is that the compound is described in JP H10-236992 A. In addition, although a symmetric compound in which both alkyl terminals are $-C_3H_7$ is described in JP H10-236992 A, an asymmetrical compound in which alkyl terminal are $-C_5H_{11}$ and $-C_3H_7$ was synthesized herein because an object is to compare melting points or the like.

Synthesis of comparative compound (S-1-1)

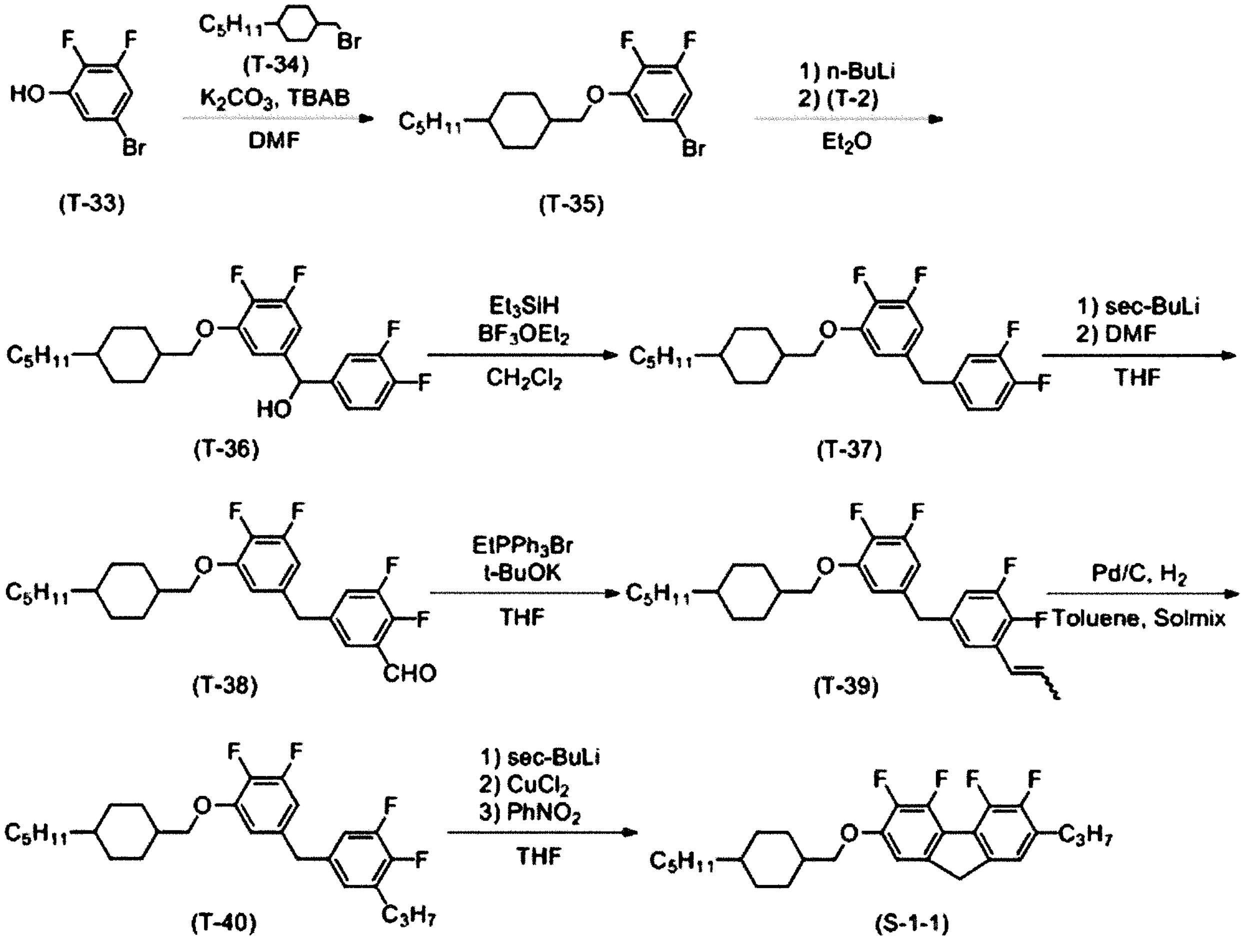


(T-33) (T-35) (T-36) (T-37) (T-38) (T-39) (T-40) (S-1-1)

First step

Under a nitrogen atmosphere, compound (T-33) (10.0 g), potassium carbonate (19.8 g), TBAB (3.09 g) and DMF (150 mL) were put in a reaction vessel, and the resulting mixture was stirred for 30 minutes at 80°C. Next, a DMF (50.0 mL) solution of compound (T-34) (14.2 g) was slowly added thereto, and the resulting mixture was stirred for 2 hours. The reaction mixture was poured into water, and the aqueous layer was subjected to extraction with toluene. The combined organic layer was washed with brine, and dried over anhydrous magnesium sulfate. The solution was concentrated under reduced pressure, and the residue was purified by silica gel chromatography (toluene) to obtain compound (T-35) (17.8 g; 99%).

Second step

Under a nitrogen atmosphere, compound (T-35) (17.8 g) and diethyl ether (300 mL) were put in a reaction vessel, and the resulting mixture was cooled to -70°C. Then, n-butyllithium (1.57 M; n-hexane solution; 27.6 mL) was slowly added thereto, and the resulting mixture was stirred for 1 hour. Next, a diethyl ether (50.0 mL) solution of compound

(T-2) (5.62 g) was slowly added thereto, and the resulting mixture was stirred for 12 hours while returning to room temperature. The reaction mixture was poured into a saturated aqueous solution of ammonium chloride and the aqueous layer was subjected to extraction with ethyl acetate. The combined organic layer was washed with brine, and dried over anhydrous magnesium sulfate. The solution was concentrated under reduced pressure, and the residue was purified by silica gel chromatography (toluene: ethyl acetate = 10 : 1 in a volume ratio) to obtain compound (T-36) (16.1 g; 93%).

Third step

Compound (T-37) (13.7 g; 88%) was obtained by using compound (T-36) (16.1 g) as a raw material in a manner similar to the technique in the second step in Example 1.

Fourth step

Compound (T-38) (11.0 g; 75%) was obtained by using compound (T-37) (13.7 g) as a raw material in a manner similar to the technique in the third step in Example 1.

Fifth step

Compound (T-39) (6.09 g; 99%) was obtained by using compound (T-38) (6.00 g) as a raw material in a manner similar to the technique in the fourth step in Example 1.

Sixth step

Compound (T-40) (5.48 g; 90%) was obtained by using compound (T-39) (6.09 g) as a raw material in a manner similar to the technique in the fifth step in Example 1.

Seventh step

Compound (S-1-1) (1.03 g; 19%) was obtained by using compound (T-40) (5.48 g) as a raw material in a manner similar to the technique in the ninth step in Example 1.

Chemical shift $\delta$ (ppm; $CDCl_3$): 7.03 (d, J = 5.6 Hz, 1H), 6.89 (d, J = 6.2 Hz, 1H), 3.87 (d, J = 6.4 Hz, 2H), 3.84 (s, 2H), 2.67 (t, J = 7.3 Hz, 2H), 1.97 - 1.90 (m, 2H), 1.88 - 1.77 (m, 3H), 1.72 - 1.63 (m, 2H), 1.36 - 1.16 (m, 9H), 1.15 - 1.02 (m, 2H), 1.01 - 0.86 (m, 8H).

Physical properties of compound (No.S-1-1) were described as follows. In addition, a mixture of the compound (3% by weight) and the base liquid crystal (97% by weight) was used as the sample in measuring a maximum temperature ($T_{NI}$), optical anisotropy ($\Delta n$), dielectric anisotropy ($\Delta\varepsilon$) and viscosity.

Transition temperature: C 157 I.

Maximum temperature ($T_{NI}$) = 97.9°C; optical anisotropy ($\Delta n$) = 0.147; dielectric anisotropy ($\Delta\varepsilon$) = -13.1; viscosity ($\eta$) = 98.9 mPa·s.

In comparison of phase transition temperatures between comparative compound (S-1-1) and compounds (1-2-5), (1-2-6), (1-2-15), (1-2-41), (1-2-22) and (1-2-52) that were obtained in Examples 1 to 6, the melting points of compounds (1-2-5), (1-2-6), (1-2-15), (1-2-41), (1-2-22) and (1-2-52) are lower than the melting point of the comparative compound. Moreover, while liquid crystal phases were developed in a temperature drop process in compounds (1-2-5), (1-2-6), (1-2-15), (1-2-22) and (1-2-52), no liquid crystal phase was developed in comparative compound (S-1-1) under influence of high crystallinity.

Further, in comparison of compatibility with other compounds between comparative compound (S-1-1) and compounds (1-2-5), (1-2-6), (1-2-15), (1-2-41), (1-2-22) and (1-2-52), while the base liquid crystal can be added in 10% to compounds (1-2-5), (1-2-6), (1-2-15) and (1-2-22), the base liquid crystal can be added only in 3% to comparative compound (S-1-1). The above results show that compounds (1-2-5), (1-2-6), (1-2-15), (1-2-41), (1-2-22) and (1-2-52) are excellent compounds that have lower melting points, higher compatibility and can be used in wider temperature range.

1-2. Example of Composition (1)

Composition (1) of the invention will be described in greater detail by way of Examples. Compounds in Examples are described using symbols according to definitions in Table 2 below. In Table 1, a configuration of 1,4-cyclohexylene is trans. A parenthesized number next to a symbolized compound in Examples corresponds to the number of the compound. A symbol (-) means any other liquid crystal compound. A proportion (percentage) of the liquid crystal compound is expressed in terms of weight percent (% by weight) based on the weight of the liquid crystal composition.

Values of physical properties of the composition are summarized in a last part. Physical properties were measured according to the methods described above, and measured values are directly described without extrapolation.

Table 1 Method for Description of Compounds using Symbols

| $R-(A_1)-Z_1-\cdots\cdots-Z_n-(A_n)-R'$ | | | |
|---|---|---|---|
| 1) Left-terminal Group R- | Symbol | 4) Ring Structure -An- | Symbol |
| $C_nH_{2n+1}$- | n- | | |
| $C_nH_{2n+1}O$- | nO- | 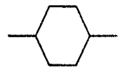 | H |
| $C_mH_{2m+1}OC_nH_{2n}$- | mOn- | | |
| $CH_2$=CH- | V- | 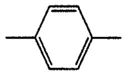 | B |
| $C_nH_{2n+1}$-CH=CH- | nV- | | |
| $CH_2$=CH-$C_nH_{2n}$- | Vn- | | |
| $C_mH_{2m+1}$-CH=CH-$C_nH_{2n}$- | mVn- | 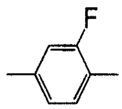 | B(F) |
| $CF_2$=CH- | VFF- | | |
| $CF_2$=CH-$C_nH_{2n}$- | VFFn- | | |
| 2) Right-terminal Group -R' | Symbol | 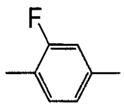 | B(2F) |
| -$C_nH_{2n+1}$ | -n | | |
| -$OC_nH_{2n+1}$ | -On | | |
| -$COOCH_3$ | -EMe | 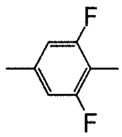 | B(F,F) |
| -CH=$CH_2$ | -V | | |
| -CH=CH-$C_nH_{2n+1}$ | -Vn | | |
| -$C_nH_{2n}$-CH=$CH_2$ | -nV | 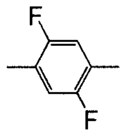 | B(2F,5F) |
| -$C_mH_{2m}$-CH=CH-$C_nH_{2n+1}$ | -mVn | | |
| -CH=$CF_2$ | -VFF | | |
| -F | -F | 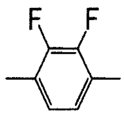 | B(2F,3F) |
| -Cl | -CL | | |
| -$OCF_3$ | -OCF3 | | |
| -$OCF_2H$ | -OCF2H | 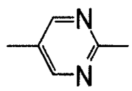 | Py |
| -$CF_3$ | -CF3 | | |
| -OCH=CH-$CF_3$ | -OVCF3 | | |
| -C≡N | -C | 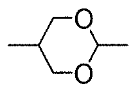 | G |
| 3) Bonding Group -Zn- | Symbol | | |
| -$C_nH_{2n}$- | n | | |
| -COO- | E | 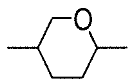 | dh |
| -CH=CH- | V | | |
| -$CH_2O$- | 1O | 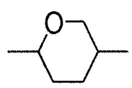 | Dh |
| -$OCH_2$- | O1 | | |
| -$CF_2O$- | X | 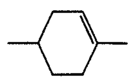 | ch |
| -C≡C- | T | 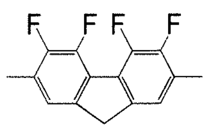 | FLF4 |

(continued)

| 5) Examples of Description | |
|---|---|
| Example 1 5-HFLF4-3 | Example 2 3-HBB(F,F)-F |
| 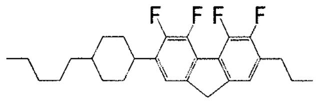 | 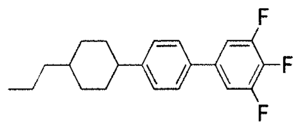 |

**Example 7**

| | | |
|---|---|---|
| 5-HFLF4-3 | (1-2-5) | 3% |
| 3-HB-O2 | (13-5) | 10% |
| 5-HB-CL | (2-2) | 13% |
| 3-HBB(F,F)-F | (3-24) | 7% |
| 3-PyB(F)-F | (2-15) | 10% |
| 5-PyB(F)-F | (2-15) | 10% |
| 3-PyBB-F | (3-80) | 10% |
| 4-PyBB-F | (3-80) | 10% |
| 5-PyBB-F | (3-80) | 10% |
| 5-HBB(F)B-2 | (15-5) | 10% |
| 5-HBB(F)B-3 | (15-5) | 7% |

NI = 94.9°C; Δn = 0.188; Δε = 7.6; η = 41.1 mPa·s.

**Example 8**

| | | |
|---|---|---|
| 5-HFLF4-4 | (1-2-6) | 3% |
| 2-HB-C | (5-1) | 5% |
| 3-HB-C | (5-1) | 12% |
| 3-HB-O2 | (13-5) | 15% |
| 2-BTB-1 | (13-10) | 3% |
| 3-HHB-F | (3-1) | 4% |
| 3-HHB-1 | (14-1) | 8% |
| 3-HHB-O1 | (14-1) | 5% |
| 3-HHB-3 | (14-1) | 14% |
| 3-HHEB-F | (3-10) | 4% |
| 5-HHEB-F | (3-10) | 4% |
| 2-HHB(F)-F | (3-2) | 4% |
| 3-HHB(F)-F | (3-2) | 7% |
| 5-HHB(F)-F | (3-2) | 7% |

(continued)

| | | |
|---|---|---|
| 3-HHB(F,F)-F | (3-3) | 5% |

NI = 100.6°C; Δn = 0.102; Δε = 4.1; η = 20.6 mPa·s.

**Example 9**

| | | |
|---|---|---|
| 5-HFLF4-2V | (1-2-15) | 3% |
| 5-HB-CL | (2-2) | 16% |
| 3-HH-4 | (13-1) | 12% |
| 3-HH-5 | (13-1) | 4% |
| 3-HHB-F | (3-1) | 4% |
| 3-HHB-CL | (3-1) | 3% |
| 4-HHB-CL | (3-1) | 4% |
| 3-HHB(F)-F | (3-2) | 10% |
| 4-HHB(F)-F | (3-2) | 9% |
| 5-HHB(F)-F | (3-2) | 9% |
| 7-HHB(F)-F | (3-2) | 8% |
| 5-HBB(F)-F | (3-23) | 4% |
| 1O1-HBBH-5 | (15-1) | 3% |
| 3-HHBB(F, F) -F | (4-6) | 2% |
| 5-HHBB(F,F)-F | (4-6) | 3% |
| 3-HH2BB(F,F)-F | (4-15) | 3% |
| 4-HH2BB(F,F)-F | (4-15) | 3% |

NI = 112.1°C; Δn = 0.091; Δε = 3.2; η = 20.0 mPa·s.

**Example 10**

| | | |
|---|---|---|
| 3-DhFLF4-5 | (1-2-41) | 3% |
| 3-HHB(F,F)-F | (3-3) | 9% |
| 3-H2HB(F,F)-F | (3-15) | 8% |
| 4-H2HB(F,F)-F | (3-15) | 8% |
| 5-H2HB(F,F)-F | (3-15) | 8% |
| 3-HBB(F,F)-F | (3-24) | 21% |
| 5-HBB(F,F)-F | (3-24) | 20% |
| 3-H2BB(F,F)-F | (3-27) | 10% |
| 5-HHEBB-F | (4-17) | 2% |
| 3-HH2BB(F,F)-F | (4-15) | 3% |
| 1O1-HBBH-4 | (15-1) | 4% |

(continued)

| | | |
|---|---|---|
| 1O1-HBBH-5 | (15-1) | 4% |

NI = 93.8°C; $\Delta n$ = 0.115; $\Delta\varepsilon$ = 8.3; $\eta$ = 36.7 mPa·s.

A pitch was 64.3 micrometers when 0.3 part by weight of compound (a-17) was added to 100 parts by weight of the composition described above

**Example 11**

| | | |
|---|---|---|
| 5-chFLF4-3 | (1-2-22) | 3% |
| 5-HB-F | (2-2) | 12% |
| 6-HB-F | (2-2) | 9% |
| 7-HB-F | (2-2) | 7% |
| 2-HHB-OCF3 | (3-1) | 7% |
| 3-HHB-OCF3 | (3-1) | 7% |
| 4-HHB-OCF3 | (3-1) | 7% |
| 5-HHB-OCF3 | (3-1) | 5% |
| 3-HH2B-OCF3 | (3-4) | 4% |
| 5-HH2B-OCF3 | (3-4) | 4% |
| 3-HHB(F,F)-OCF2H | (3-3) | 4% |
| 3-HHB(F,F)-OCF3 | (3-3) | 5% |
| 3-HH2B(F)-F | (3-5) | 3% |
| 3-HBB(F)-F | (3-23) | 8% |
| 5-HBB(F)-F | (3-23) | 9% |
| 5-HBBH-3 | (15-1) | 3% |
| 3-HB(F)BH-3 | (15-2) | 3% |

NI = 85.7°C; $\Delta n$ = 0.094; $\Delta\varepsilon$ = 3.9; $\eta$ = 16.9 mPa·s.

**Example 12**

| | | |
|---|---|---|
| 5-H2FLF4-3 | (1-2-52) | 3% |
| 5-HB-CL | (2-2) | 11% |
| 3-HH-4 | (13-1) | 8% |
| 3-HHB-1 | (14-1) | 5% |
| 3-HHB(F,F)-F | (3-3) | 8% |
| 3-HBB(F,F)-F | (3-24) | 20% |
| 5-HBB(F,F)-F | (3-24) | 15% |
| 3-HHEB(F,F)-F | (3-12) | 10% |
| 4-HHEB(F,F)-F | (3-12) | 3% |

(continued)

| | | |
|---|---|---|
| 5-HHEB(F,F)-F | (3-12) | 3% |
| 2-HBEB(F,F)-F | (3-39) | 3% |
| 3-HBEB(F,F)-F | (3-39) | 5% |
| 5-HBEB(F,F)-F | (3-39) | 3% |
| 3-HHBB(F,F)-F | (4-6) | 3% |

NI = 77.4°C; Δn = 0.104; Δε = 8.0; η = 23.3 mPa·s.

**Example 13**

| | | |
|---|---|---|
| 5-dhFLF4-3 | (1-2-48) | 3% |
| 3-HB-CL | (2-2) | 6% |
| 5-HB-CL | (2-2) | 4% |
| 3-HHB-OCF3 | (3-1) | 5% |
| 3-H2HB-OCF3 | (3-13) | 5% |
| 5-H4HB-OCF3 | (3-19) | 15% |
| V-HHB(F)-F | (3-2) | 5% |
| 3-HHB(F)-F | (3-2) | 5% |
| 5-HHB(F)-F | (3-2) | 5% |
| 3-H4HB(F,F)-CF3 | (3-21) | 5% |
| 5-H4HB(F,F)-CF3 | (3-21) | 10% |
| 5-H2HB(F,F)-F | (3-15) | 5% |
| 5-H4HB(F,F)-F | (3-21) | 7% |
| 2-H2BB(F)-F | (3-26) | 5% |
| 3-H2BB(F)-F | (3-26) | 10% |
| 3-HBEB(F,F)-F | (3-39) | 5% |

**Example 14**

| | | |
|---|---|---|
| 5-B(F)FLF4-3 | (1-2-37) | 3% |
| 5-HB-CL | (2-2) | 3% |
| 7-HB(F)-F | (2-3) | 7% |
| 3-HH-4 | (13-1) | 9% |
| 3-HH-EMe | (13-2) | 20% |
| 3-HHEB-F | (3-10) | 8% |
| 5-HHEB-F | (3-10) | 8% |
| 3-HHEB(F,F)-F | (3-12) | 10% |
| 4-HHEB(F,F)-F | (3-12) | 5% |

(continued)

| | | |
|---|---|---|
| 4-HGB(F,F)-F | (3-103) | 5% |
| 5-HGB(F,F)-F | (3-103) | 6% |
| 2-H2GB(F,F)-F | (3-106) | 4% |
| 3-H2GB(F,F)-F | (3-106) | 5% |
| 5-GHB(F,F)-F | (3-109) | 7% |

**Example 15**

| | | |
|---|---|---|
| 4-FLF4-3 | (1-1-1) | 3% |
| 1V2-BEB(F,F)-C | (5-15) | 6% |
| 3-HB-C | (5-1) | 18% |
| 2-BTB-1 | (13-10) | 10% |
| 5-HH-VFF | (13-1) | 30% |
| 3-HHB-1 | (14-1) | 4% |
| VFF-HHB-1 | (14-1) | 8% |
| VFF2-HHB-1 | (14-1) | 8% |
| 3-H2BTB-2 | (14-17) | 5% |
| 3-H2BTB-3 | (14-17) | 4% |
| 3-H2BTB-4 | (14-17) | 4% |

**Example 16**

| | | |
|---|---|---|
| 5-HFLF4H-3 | (1-3-1) | 3% |
| 5-HB(F)B(F,F)XB(F,F)-F | (4-41) | 5% |
| 3-BB(F)B(F,F)XB(F,F)-F | (4-47) | 3% |
| 4-BB(F)B(F,F)XB(F,F)-F | (4-47) | 7% |
| 5-BB(F)B(F,F)XB(F,F)-F | (4-47) | 3% |
| 3-HH-V | (13-1) | 41% |
| 3-HH-V1 | (13-1) | 7% |
| 3-HHB-1 | (14-1) | 4% |
| V-HHB-1 | (14-1) | 5% |
| V2-BB(F)B-1 | (14-6) | 5% |
| 1V2-BB-F | (2-1) | 3% |
| 3-BB(F,F)XB(F,F)-F | (3-97) | 11% |
| 3-HHBB(F,F)-F | (4-6) | 3% |

**Example 17**

| | | |
|---|---|---|
| 5-HHFLF4-3 | (1-4-1) | 3% |
| 3-GB(F)B(F,F)XB(F,F)-F | (4-57) | 5% |
| 3-BB(F)B(F,F)XB(F,F)-F | (4-47) | 3% |
| 4-BB(F)B(F,F)XB(F,F)-F | (4-47) | 7% |
| 5-BB(F)B(F,F)XB(F,F)-F | (4-47) | 3% |
| 3-HH-V | (13-1) | 41% |
| 3-HH-V1 | (13-1) | 7% |
| 3-HHB-1 | (14-1) | 4% |
| V-HHB-1 | (14-1) | 5% |
| V2-BB(F)B-1 | (14-6) | 5% |
| 1V2-BB-F | (2-1) | 3% |
| 3-BB(F,F)XB(F,F)-F | (3-97) | 6% |
| 3-GB(F,F)XB(F,F)-F | (3-113) | 5% |
| 3-HHBB(F,F)-F | (4-6) | 3% |

## Industrial Applicability

A liquid crystal compound of the invention has high stability to heat, light and so forth, a high clearing point, a low minimum temperature of a liquid crystal phase, small viscosity, suitable optical anisotropy, large negative dielectric anisotropy, a suitable elastic constant and excellent compatibility with other liquid crystal compounds. A composition of the invention contains the compound, and has a high maximum temperature of a nematic phase, a low minimum temperature of the nematic phase, small viscosity, suitable optical anisotropy, large negative dielectric anisotropy, and a suitable elastic constant. The composition has a suitable balance regarding at least two of physical properties. A liquid crystal display device of the invention includes the composition, and has a wide temperature range in which the device can be used, a short response time, a large voltage holding ratio, a low threshold voltage, a large contrast ratio and a long service life. Accordingly, the device can be widely utilized in a display such as a personal computer and a television.

## Claims

1. A compound represented by formula (1):

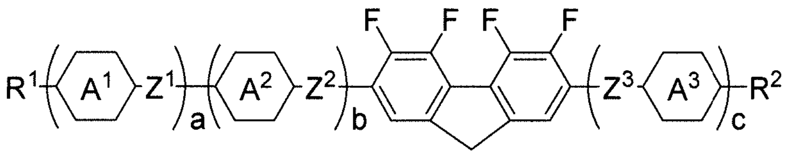

(1)

wherein, in formula (1),

$R^1$ and $R^2$ are independently alkyl having 1 to 15 carbons or alkenyl having 2 to 15 carbons, and in the groups, at least one piece of hydrogen may be replaced by halogen;
ring $A^1$, ring $A^2$ and ring $A^3$ are independently 1,4-cyclohexylene, 1,4-cyclohexenylene, 1,4-phenylene, 1,4-phenylene in which at least one piece of hydrogen is replaced by halogen, or tetrahydropyran-2,5-diyl;
$Z^1$, $Z^2$ and $Z^3$ are independently a single bond, $-(CH_2)_2-$, -CH=CH-, -C≡C-, -CF=CF-, - $(CH_2)_4-$, $-CH=CH-(CH_2)_2-$ or $-(CH_2)_2-CH=CH-$; and
a, b and c are independently 0 or 1, and a sum of a, b and c is 0, 1 or 2.

**2.** The compound according to claim 1, wherein, in the formula (1), $R^1$ and $R^2$ are independently alkyl having 1 to 10 carbons, alkenyl having 2 to 10 carbons, alkyl having 1 to 10 carbons in which at least one piece of hydrogen is replaced by fluorine, or alkenyl having 2 to 10 carbons in which at least one piece of hydrogen is replaced by fluorine, and $Z^1$, $Z^2$ and $Z^3$ are independently a single bond, $-(CH_2)_2-$, $-CH=CH-$, $-(CH_2)_4-$, $-CH=CH-(CH_2)_2-$ or $-(CH_2)_2-CH=CH-$.

**3.** The compound according to claim 1, represented by any one of formulas (1-1) to (1-4):

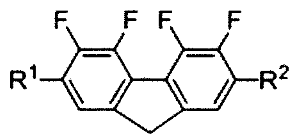

(1-1)

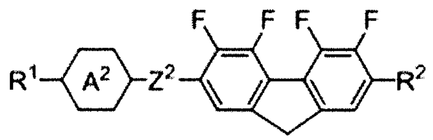

(1-2)

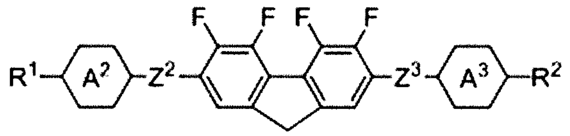

(1-3)

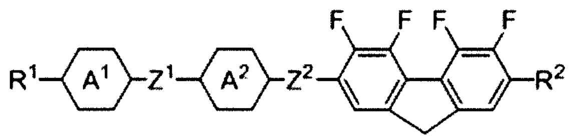

(1-4)

wherein, formulas (1-1) to (1-4),

$R^1$ and $R^2$ are independently alkyl having 1 to 10 carbons, alkenyl having 2 to 10 carbons or alkyl having 1 to 10 carbons in which at least one piece of hydrogen is replaced by fluorine;
ring $A^1$, ring $A^2$ and ring $A^3$ are independently 1,4-cyclohexylene, 1,4-cyclohexenylene, 1,4-phenylene, 1,4-phenylene in which at least one piece of hydrogen is replaced by fluorine, or tetrahydropyran-2,5-diyl;
$Z^1$ is a single bond, $-(CH_2)_2-$ or $-CH=CH-$;
$Z^2$ is a single bond, $-(CH_2)_2-$, $-(CH_2)_4-$ or $-CH=CH-(CH_2)_2-$; and
$Z^3$ is a single bond, $-(CH_2)_2-$, $-(CH_2)_4-$ or $-(CH_2)_2-CH=CH-$.

**4.** The compound according to claim 1, represented by any one of formulas (1-5) to (1-12):

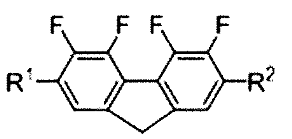

(1-5)

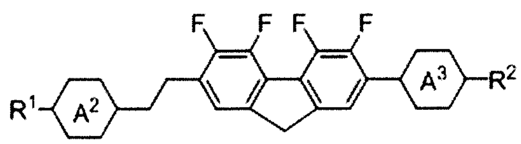

(1-9)

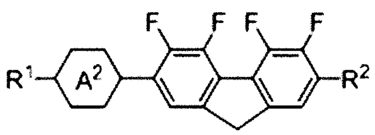

(1-6)

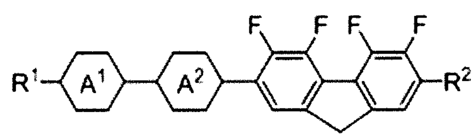

(1-10)

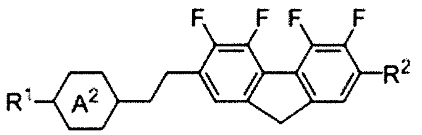 (1-7) 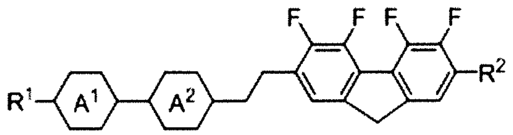 (1-11)

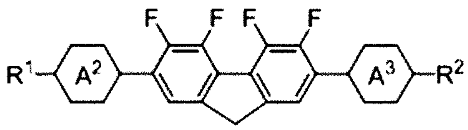 (1-8) 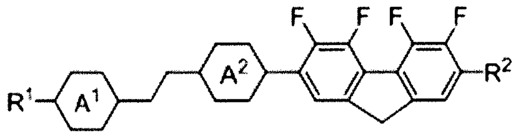 (1-12)

wherein, in formulas (1-5) to (1-12),

$R^1$ and $R^2$ are independently alkyl having 1 to 10 carbons or alkenyl having 2 to 10 carbons; and
ring $A^1$, ring $A^2$ and ring $A^3$ are independently 1,4-cyclohexylene, 1,4-cyclohexenylene, 1,4-phenylene, 1,4-phenylene in which at least one piece of hydrogen is replaced by fluorine, or tetrahydropyran-2,5-diyl.

**5.** The compound according to claim 1, represented by any one of formulas (1-13) to (1-22):

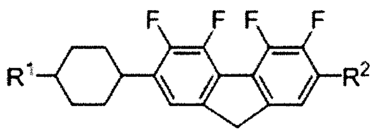 (1-13) 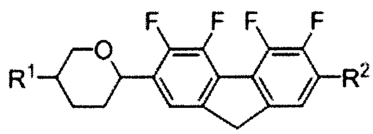 (1-18)

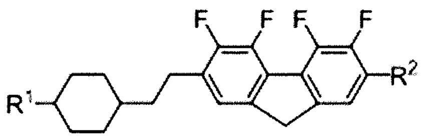 (1-14) 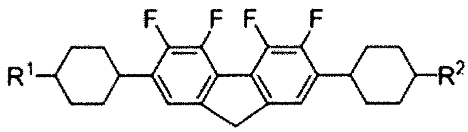 (1-19)

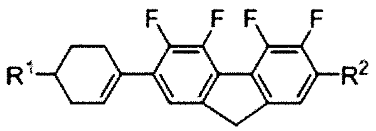 (1-15) 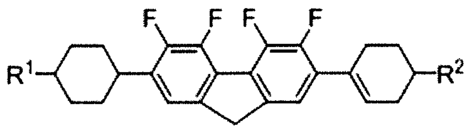 (1-20)

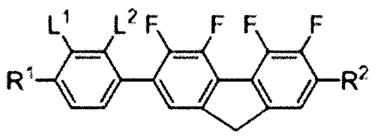 (1-16) 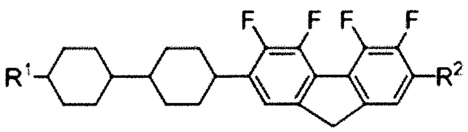 (1-21)

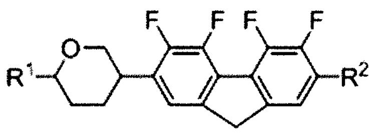 (1-17) 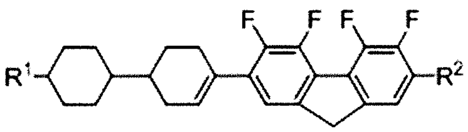 (1-22)

wherein, in formulas (1-13) to (1-22), $R^1$ and $R^2$ are independently alkyl having 1 to 10 carbons or alkenyl having 2 to 10 carbons; and $L^1$ and $L^2$ are independently hydrogen or fluorine.

**6.** The compound according to claim 1, represented by any one of formulas (1-23) to (1-25):

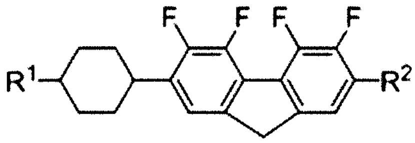
(1-23)

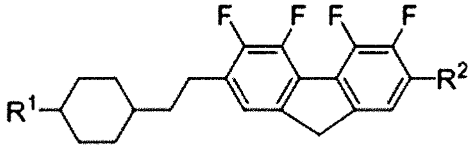
(1-24)

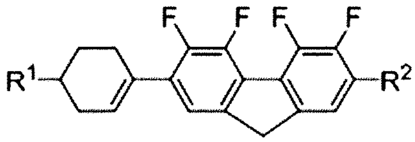
(1-25)

wherein, in formulas (1-23) to (1-25), $R^1$ and $R^2$ are independently alkyl having 1 to 7 carbons or alkenyl having 2 to 7 carbons.

**7.** A liquid crystal composition, containing at least one of compounds described in claim 1.

**8.** The liquid crystal composition according to claim 7, further containing at least one compound selected from the group of compounds represented by formulas (2) to (4):

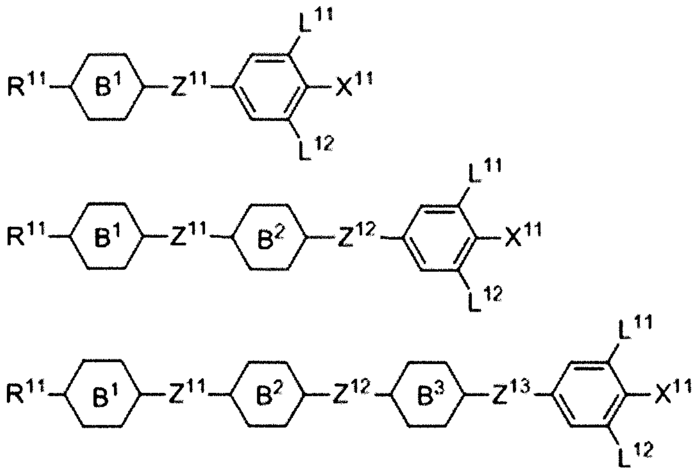
(2)

(3)

(4)

wherein, in formulas (2) to (4),

$R^{11}$ is alkyl having 1 to 10 carbons or alkenyl having 2 to 10 carbons, and in the alkyl and the alkenyl, at least one piece of hydrogen may be replaced by fluorine, and at least one piece of $-CH_2-$ may be replaced by -O-;
$X^{11}$ is fluorine, chlorine, $-OCF_3$, $-OCHF_2$, $-CF_3$, $-CHF_2$, $-CH_2F$, $-OCF_2CHF_2$ or $-OCF_2CHFCF_3$ ;
ring $B^1$, ring $B^2$ and ring $B^3$ are independently 1,4-cyclohexylene, 1,4-phenylene, 2-fluoro-1,4-phenylene, 2,5-difluoro-1,4-phenylene, tetrahydropyran-2,5-diyl, 1,3-dioxane-2,5-diyl or pyrimidine-2,5-diyl; and
$Z^{11}$, $Z^{12}$ and $Z^{13}$ are independently a single bond, $-CH_2CH_2-$, -CH=CH-, -C=C-, -COO-, $-CF_2O-$, $-OCF_2-$, $-CH_2O-$ or $-(CH_2)_4-$; and $L^{11}$ and $L^{12}$ are independently hydrogen or fluorine.

**9.** The liquid crystal composition according to claim 7, further containing at least one compound selected from the group of compounds represented by formula (5):

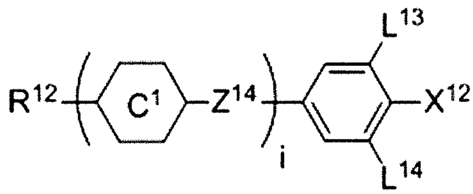

(5)

wherein, in the formula (5),

$R^{12}$ is alkyl having 1 to 10 carbons or alkenyl having 2 to 10 carbons, and in the alkyl and the alkenyl, at least one piece of hydrogen may be replaced by fluorine, and at least one piece of $-CH_2-$ may be replaced by -O-;
$X^{12}$ is -C=N or -C≡C-C≡N;
ring $C^1$ is 1,4-cyclohexylene, 1,4-phenylene, 2-fluoro-1,4-phenylene, 2,5-difluoro-1,4-phenylene, tetrahydropyran-2,5-diyl, 1,3-dioxane-2,5-diyl or pyrimidine-2,5-diyl;
$Z^{14}$ is a single bond, $-CH_2CH_2-$, -C=C-, -COO-, $-CF_2O-$, $-OCF_2-$ or $-CH_2O-$;
$L^{13}$ and $L^{14}$ are independently hydrogen or fluorine; and
i is 1, 2, 3 or 4.

**10.** The liquid crystal composition according to claim 7, further containing at least one compound selected from the group of compounds represented by formulas (6) to (12):

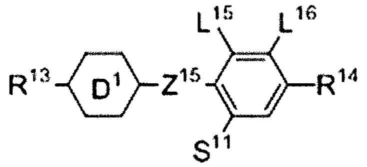

(6)

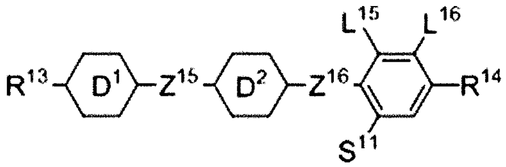

(7)

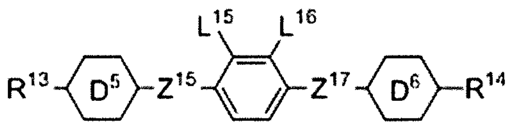

(8)

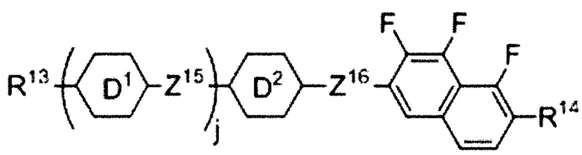

(9)

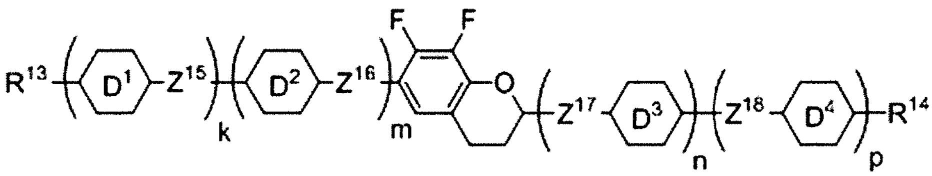

(10)

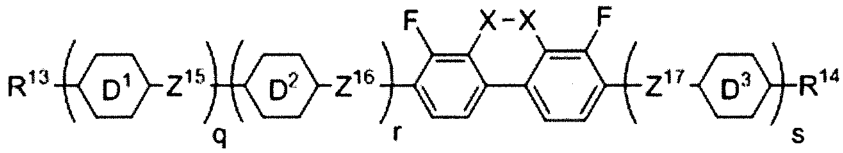

(11)

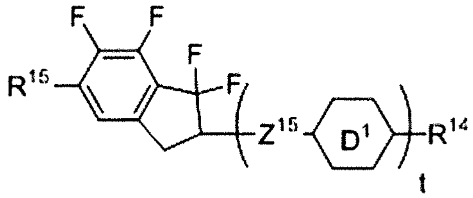

(12)

wherein, in formulas (6) to (12),

$R^{13}$ and $R^{14}$ are independently alkyl having 1 to 10 carbons or alkenyl having 2 to 10 carbons, and in the alkyl and the alkenyl, at least one piece of $-CH_2-$ may be replaced by -O-;
$R^{15}$ is hydrogen, fluorine, alkyl having 1 to 10 carbons or alkenyl having 2 to 10 carbons, and in the alkyl and the alkenyl, at least one piece of $-CH_2-$ may be replaced by -O-, and at least one piece of hydrogen may be replaced by fluorine;
$S^{11}$ is hydrogen or methyl;
X is $-CF_2-$, -O- or -CHF-;
ring $D^1$, ring $D^2$, ring $D^3$ and ring $D^4$ are independently 1,4-cyclohexylene, 1,4-cyclohexenylene, 1,4-phenylene in which at least one piece of hydrogen may be replaced by fluorine, tetrahydropyran-2,5-diyl or decahydronaphthalene-2,6-diyl;
ring $D^5$ and ring $D^6$ are independently 1,4-cyclohexylene, 1,4-cyclohexenylene, 1,4-phenylene, tetrahydropyran-2,5-diyl or decahydronaphthalene-2,6-diyl;
$Z^{15}$, $Z^{16}$, $Z^{17}$ and $Z^{18}$ are independently a single bond, $-CH_2CH_2-$, -COO-, $-CH_2O-$, $-OCF_2-$ or $-OCF_2CH_2CH_2-$;
$L^{15}$ and $L^{16}$ are independently fluorine or chlorine; and
j, k, m, n, p, q, r and s are independently 0 or 1, a sum of k, m, n and p is 1 or 2, a sum of q, r and s is 0, 1, 2 or 3, and t is 1, 2 or 3.

**11.** The liquid crystal composition according to claim 7, further containing at least one compound selected from the group of compounds represented by formulas (13) to (15):

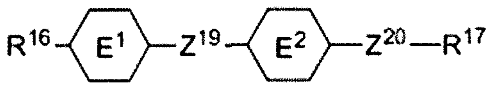

(13)

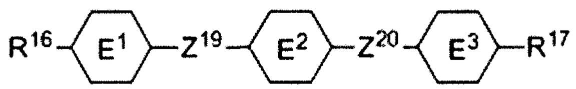

(14)

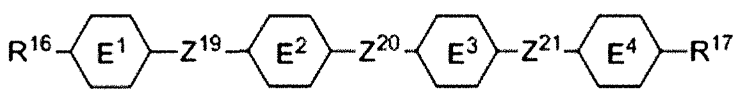

(15)

wherein, in formulas (13) to (15),

$R^{16}$ and $R^{17}$ are independently alkyl having 1 to 10 carbons or alkenyl having 2 to 10 carbons, and in the alkyl or the alkenyl, at least one piece of $-CH_2-$ may be replaced by -O-, and at least one piece of hydrogen may be replaced by fluorine;
ring $E^1$, ring $E^2$, ring $E^3$ and ring $E^4$ are independently 1,4-cyclohexylene, 1,4-phenylene, 2-fluoro-1,4-phenylene, 2,5-difluoro-1,4-phenylene or pyrimidine-2,5-diyl; and
$Z^{19}$, $Z^{20}$ and $Z^{21}$ are independently a single bond, $-CH_2CH_2-$, -CH=CH-, -C=C- or -COO-.

**12.** The liquid crystal composition according to claim 7, further containing at least one of a polymerizable compound,

an optically active compound, an antioxidant, an ultraviolet light absorber, a light stabilizer, a heat stabilizer, and an antifoaming agent.

**13.** A liquid crystal display device, including the liquid crystal composition according to claim 7.

**Patentansprüche**

**1.** Verbindung mit der Formel (1):

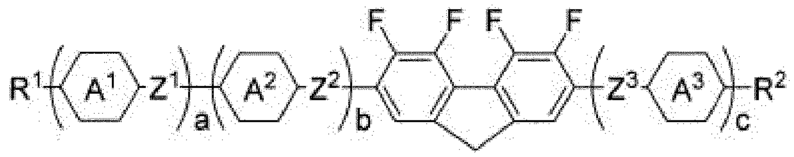

(1)

worin in der Formel (1)

$R^1$ und $R^2$ unabhängig Alkyl mit 1 bis 15 Kohlenstoffatomen oder Alkenyl mit 2 bis 15 Kohlenstoffatomen sind und in den Gruppen zumindest ein Wasserstoff durch Halogen ersetzt sein kann,
Ring $A^1$, Ring $A^2$ und Ring $A^3$ unabhängig 1,4-Cyclohexylen, 1,4-Cyclohexenylen, 1,4-Phenylen, 1,4-Phenylen, worin zumindest ein Wasserstoff durch Halogen ersetzt ist, oder Tetrahydropyran-2,5-diyl sind,
$Z^1$, $Z^2$ und $Z^3$ unabhängig eine Einfachbindung, $-(CH_2)_2-$, -CH=CH-, -C≡C-, -CF=CF-, $-(CH_2)_4-$, $-CH=CH-(CH_2)_2-$ oder $-(CH_2)_2-CH=CH-$ sind; und
a, b und c unabhängig 0 oder 1 sind und eine Summe von a, b und c 0, 1 oder 2 ist.

**2.** Verbindung gemäß Anspruch 1, worin in der Formel (1) $R^1$ und $R^2$ unabhängig Alkyl mit 1 bis 10 Kohlenstoffatomen, Alkenyl mit 2 bis 10 Kohlenstoffatomen, Alkyl mit 1 bis 10 Kohlenstoffatomen, worin zumindest ein Wasserstoff durch Fluor ersetzt ist, oder Alkenyl mit 2 bis 10 Kohlenstoffatomen, worin zumindest ein Wasserstoff durch Fluor ersetzt ist, sind und $Z^1$, $Z^2$ und $Z^3$ unabhängig eine Einfachbindung, $-(CH_2)_2-$, -CH=CH-, $-(CH_2)_4-$, $-CH=CH-(CH_2)_2-$ oder $-(CH_2)_2-CH=CH-$ sind.

**3.** Verbindung gemäß Anspruch 1, dargestellt durch eine der Formeln (1-1) bis (1-4) :

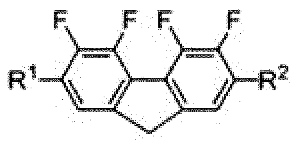

(1-1)

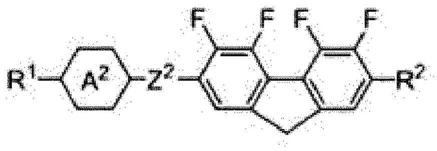

(1-2)

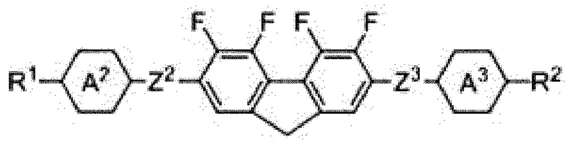

(1-3)

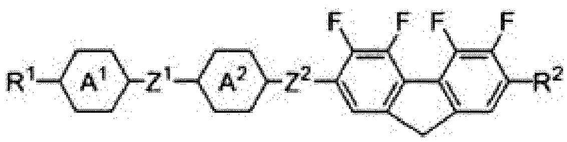

(1-4)

worin in den Formeln (1-1) bis (1-4)

$R^1$ und $R^2$ jeweils unabhängig Alkyl mit 1 bis 10 Kohlenstoffatomen, Alkenyl mit 2 bis 10 Kohlenstoffatomen oder Alkyl mit 1 bis 10 Kohlenstoffatomen sind, worin zumindest ein Wasserstoff durch Fluor ersetzt ist;
Ring $A^1$, Ring $A^2$ und Ring $A^3$ unabhängig 1,4-Cyclohexylen, 1,4-Cyclohexenylen, 1,4-Phenylen, 1,4-Phenylen, worin zumindest ein Wasserstoff durch Fluor ersetzt ist, oder Tetrahydropyran-2,5-diyl sind;
$Z^1$ eine Einfachbindung, $-(CH_2)_2-$ oder -CH=CH- ist;
$Z^2$ eine Einfachbindung $-(CH_2)_2-$, $-(CH_2)_4-$ oder $-CH=CH-(CH_2)_2-$ ist und
$Z^3$ eine Einfachbindung, $-(CH_2)_2-$, $-(CH_2)_4-$ oder $-(CH_2)_2-CH=CH-$ ist.

**4.** Verbindung gemäß Anspruch 1, dargestellt durch eine der Formeln (1-5) bis (1-12):

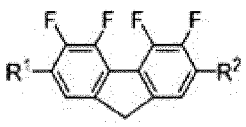
(1-5)

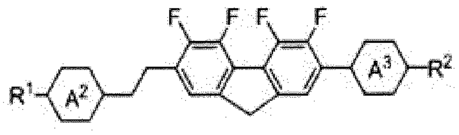
(1-9)

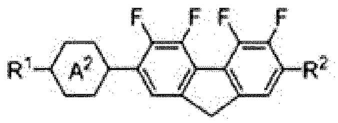
(1-6)

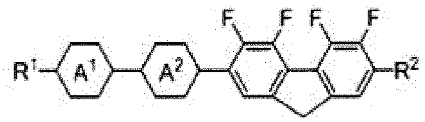
(1-10)

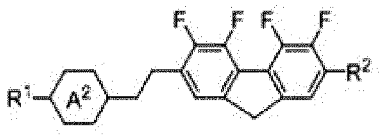
(1-7)

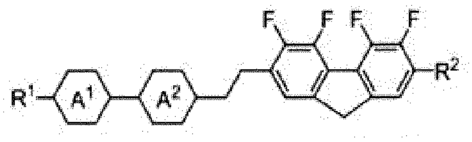
(1-11)

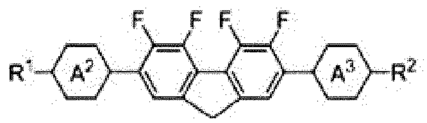
(1-8)

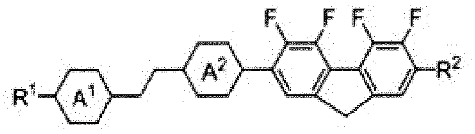
(1-12)

worin in den Formeln (1-5) bis (1-12)

$R^1$ und $R^2$ jeweils unabhängig Alkyl mit 1 bis 10 Kohlenstoffatomenen oder Alkenyl mit 2 bis 10 Kohlenstoffatomen sind, und
Ring $A^1$, Ring $A^2$ und Ring $A^3$ unabhängig 1,4-Cyclohexylen, 1,4-Cyclohexenylen, 1,4-Phenylen, 1,4-Phenylen, worin zumindest ein Wasserstoff durch Fluor ersetzt ist, oder Tetrahydropyran-2,5-diyl sind.

**5.** Verbindung gemäß Anspruch 1, dargestellt durch eine der Formeln (1-13) bis (1-22):

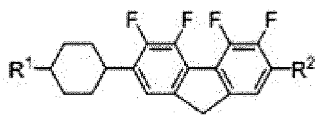
(1-13)

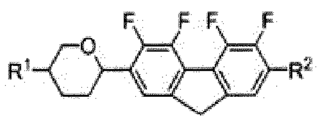
(1-18)

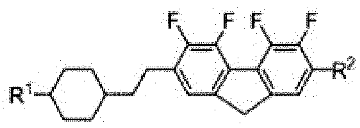
(1-14)

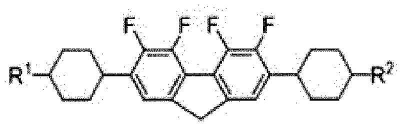
(1-19)

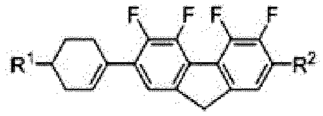 (1-15)

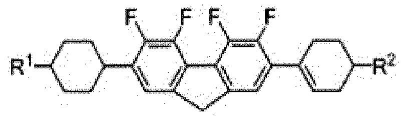 (1-20)

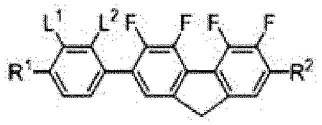 (1-16)

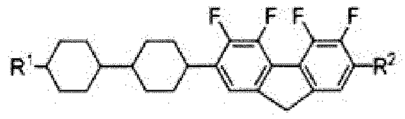 (1-21)

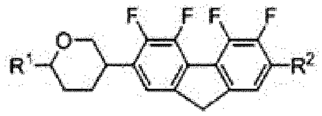 (1-17)

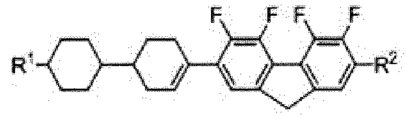 (1-22)

worin in den Formeln (1-13) bis (1-22) $R^1$ und $R^2$ unabhängig Alkyl mit 1 bis 10 Kohlenstoffatomen oder Alkenyl mit 2 bis 10 Kohlenstoffatomen sind und $L^1$ und $L^2$ unabhängig Wasserstoff oder Fluor sind.

**6.** Verbindung gemäß Anspruch 1, dargestellt durch eine der Formeln (1-23) bis (1-25):

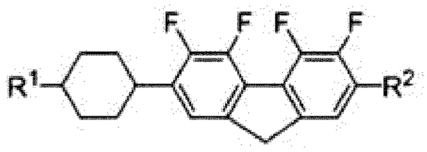 (1-23)

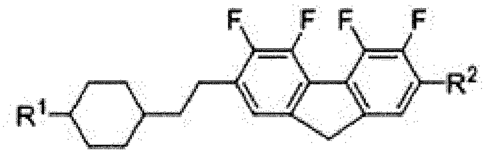 (1-24)

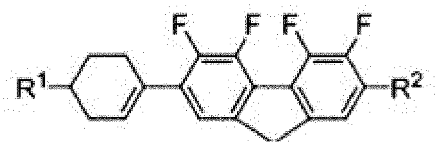 (1-25)

worin in den Formeln (1-23) bis (1-25) $R^1$ und $R^2$ unabhängig Alkyl mit 1 bis 7 Kohlenstoffatomen oder Alkenyl mit 2 bis 7 Kohlenstoffatomen sind.

**7.** Flüssigkristallzusammensetzung, enthaltend zumindest eine der Verbindungen, die in Anspruch 1 beschrieben sind.

**8.** Flüssigkristallzusammensetzung gemäß Anspruch 7, weiterhin enthaltend zumindest eine Verbindung, ausgewählt aus der Gruppe von Verbindungen, dargestellt durch die Formeln (2) bis (4):

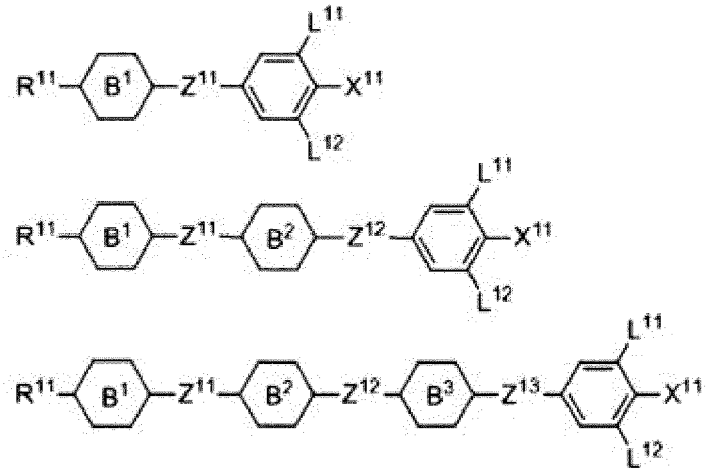

(2)

(3)

(4)

worin in den Formeln (2) bis (4)

$R^{11}$ Alkyl mit 1 bis 10 Kohlenstoffatomen oder Alkenyl mit 2 bis 10 Kohlenstoffatomen ist und in dem Alkyl und Alkenyl zumindest ein Wasserstoff durch Fluor ersetzt sein kann und zumindest ein $-CH_2-$ durch -O- ersetzt sein kann;
$X^{11}$ Fluor, Chlor, $-OCF_3$, $-OCHF_2$, $-CF_3$, $-CHF_2$, $-CH_2F$, $-OCF_2CHF_2$ oder $-OCF_2CHFCF_3$ ist;
Ring $B^1$, Ring $B^2$ und Ring $B^3$ unabhängig 1,4-Cyclohexylen, 1,4-Phenylen, 2-Fluor-1,4-phenylen, 2,5-Difluor-1,4-phenylen, Tetrahydropyran-2,5-diyl, 1,3-Dioxan-2,5-diyl oder Pyrimidin-2,5-diyl sind; und
$Z^{11}$, $Z^{12}$ und $Z^{13}$ unabhängig eine Einfachbindung, $-CH_2CH_2-$, -CH=CH-, -C≡C-, -COO-, $-CF_2O-$, $-OCF_2-$, $-CH_2O-$ oder $-(CH_2)_4-$ und $L^{11}$ und $L^{12}$ unabhängig Wasserstoff oder Fluor sind.

**9.** Flüssigkristallzusammensetzung gemäß Anspruch 7, weiterhin enthaltend zumindest eine Verbindung, ausgewählt aus der Gruppe von Verbindungen mit der Formel (5):

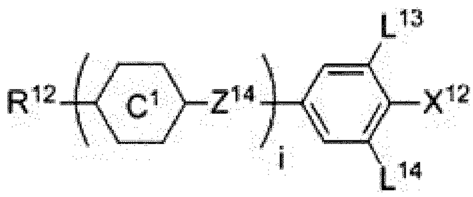

(5)

worin in der Formel (5)

$R^{12}$ Alkyl mit 1 bis 10 Kohlenstoffatomen oder Alkenyl mit 2 bis 10 Kohlenstoffatomen ist und im Alkyl und Alkenyl zumindest ein Wasserstoff durch Fluor ersetzt sein kann und zumindest ein $-CH_2-$ durch -O- ersetzt sein kann,
$X^{12}$ -C≡N oder -C≡C-C≡N ist,
Ring $C^1$ 1,4-Cyclohexylen, 1,4-Phenylen, 2-Fluor-1,4-phenylen, 2,5-Difluor-1,4-phenylen, Tetrahydropyran-2,5-diyl, 1,3-Dioxan-2,5-diyl oder Pyrimidin-2,5-diyl ist,
$Z^{14}$ eine Einfachbindung, $-CH_2CH_2-$, -C≡C-, -COO-, $-CF_2O-$, $-OCF_2-$ oder $-CH_2O-$ ist,
$L^{13}$ und $L^{14}$ unabhängig Wasserstoff oder Fluor sind; und
i 1, 2, 3 oder 4 ist.

**10.** Flüssigkristallzusammensetzung gemäß Anspruch 7, weiterhin enthaltend zumindest eine Verbindung, ausgewählt aus der Gruppe von Verbindungen mit den Formeln (6) bis (12):

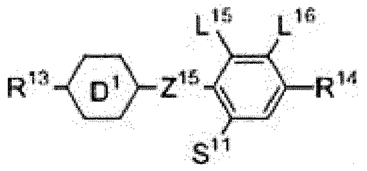

(6)

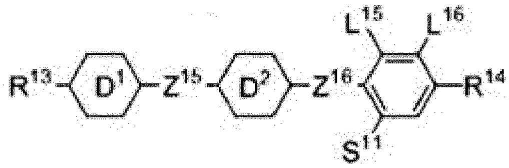

(7)

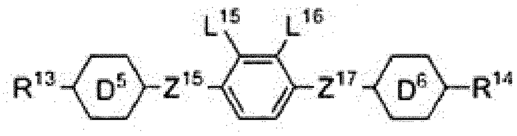

(8)

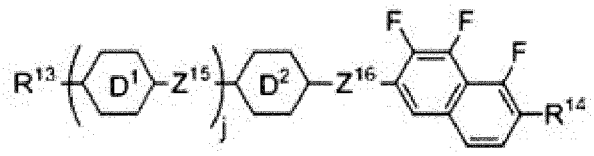

(9)

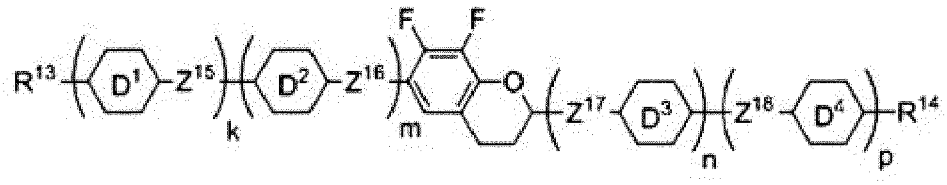

(10)

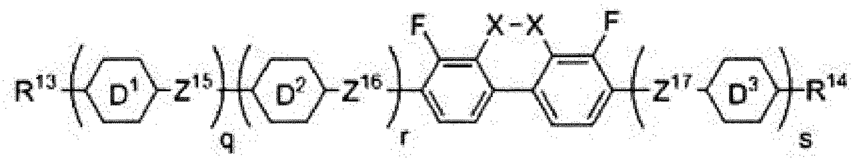

(11)

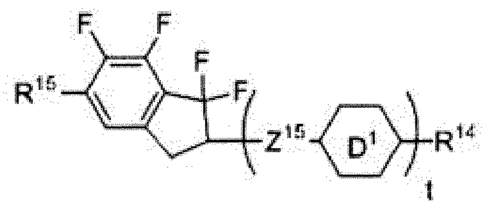

(12)

worin in den Formeln (6) bis (12)

$R^{13}$ und $R^{14}$ unabhängig Alkyl mit 1 bis 10 Kohlenstoffatomen oder Alkenyl mit 2 bis 10 Kohlenstoffatomen sind und im Alkyl und Alkenyl zumindest ein $-CH_2-$ durch -O- ersetzt sein kann,
$R^{15}$ Wasserstoff, Fluor, Alkyl mit 1 bis 10 Kohlenstoffatomen oder Alkenyl mit 2 bis 10 Kohlenstoffatomen ist und im Alkyl und Alkenyl zumindest ein $-CH_2-$ durch -O- ersetzt sein kann und zumindest ein Wasserstoff durch Fluor ersetzt sein kann;
$s^{11}$ Wasserstoff oder Methyl ist;
X $-CF_2-$, -O- oder -CHF- ist;
Ring $D^1$, Ring $D^2$, Ring $D^3$ und Ring $D^4$ unabhängig 1,4-Cyclohexylen, 1,4-Cyclohexenylen, 1,4-Phenylen, worin

zumindest ein Wasserstoff durch Fluor ersetzt sein kann, Tetrahydropyran-2,5-diyl oder Decahydronaphthalin-2,6-diyl sind,
Ring $D^5$ und Ring $D^6$ unabhängig 1,4-Cyclohexylen, 1,4-Cyclohexenylen, 1,4-Phenylen, Tetrahydropyran-2,5-diyl oder Decahydronaphthalin sind,
$Z^{15}$, $Z^{16}$, $Z^{17}$ und $Z^{18}$ unabhängig eine Einfachbindung, $-CH_2CH_2-$, -COO-, $-CH_2O-$, $-OCF_2-$ oder $-OCF_2CH_2CH_2-$ sind,
$L^{15}$ und $L^{16}$ unabhängig Fluor oder Chlor sind, und
j, k, m, n, p, q, r und s unabhängig 0 oder 1 sind, eine Summe von k, m, n und p 1 oder 2 ist, eine Summe von q, r und s 0, 1, 2 oder 3 ist und t 1, 2 oder 3 ist.

**11.** Flüssigkristallzusammensetzung gemäß Anspruch 7, weiterhin enthaltend zumindest eine Verbindung, ausgewählt aus der Gruppe von Verbindungen mit den Formeln (13) bis (15):

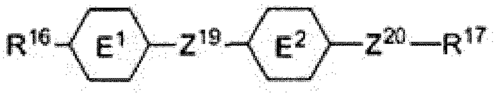

(13)

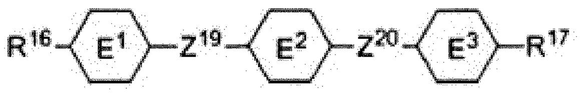

(14)

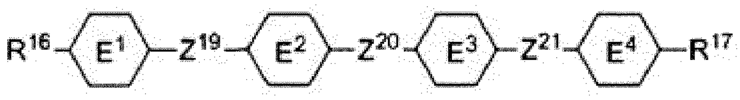

(15)

worin in den Formeln (13) bis (15)

$R^{16}$ und $R^{17}$ unabhängig Alkyl mit 1 bis 10 Kohlenstoffatomen oder Alkenyl mit 2 bis 10 Kohlenstoffatomen sind und im Alkyl und Alkenyl zumindest ein $-CH_2-$ durch -O- ersetzt sein kann und zumindest ein Wasserstoff durch Fluor ersetzt sein kann;
Ring $E^1$, Ring $E^2$, Ring $E^3$ und Ring $E^4$ unabhängig 1,4-Cyclohexylen, 1,4-Phenylen, 2-Fluor-1,4-phenylen, 2,5-Difluor-1,4-phenylen oder Pyrimidin-2,5-diyl sind und
$Z^{19}$, $Z^{20}$ und $Z^{21}$ unabhängig eine Einfachbindung, $-CH_2CH_2-$, -CH=CH-, -C≡C- oder -COO- sind.

**12.** Flüssigkristallzusammensetzung gemäß Anspruch 7, weiterhin enthaltend zumindest eine von einer polymerisierbaren Verbindung, einer opotisch aktiven Verbindung, einem Antioxidans, Ultraviolettlicht-Absorber, Lichtstabilisator, Wärmestabilisator und Antischäummittel.

**13.** Flüssigkristall-Anzeigevorrichtung, enthaltend die Flüssigkristallzusammensetzung gemäß Anspruch 7.

## Revendications

**1.** Composé représenté par la formule (1) :

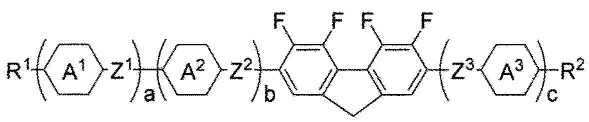

(1)

dans lequel, dans la formule (1),

$R^1$ et $R^2$ sont indépendamment un alkyle ayant 1 à 15 atomes de carbone ou un alcényle ayant 2 à 15 atomes de carbone et, dans les groupements, au moins un hydrogène peut être remplacé par un halogène ;
le noyau $A^1$, le noyau $A^2$ et le noyau $A^3$ sont indépendamment un 1,4-cyclohexylène, un 1,4-cyclohexénylène, un 1,4-phénylène, un 1,4-phénylène dans lequel au moins un hydrogène est remplacé par un halogène, ou un

tétrahydropyran-2,5-diyle ;
$Z^1$, $Z^2$ et $Z^3$ sont indépendamment une liaison simple, $-(CH_2)_2-$, -CH=CH-, -C≡C-, -CF=CF-, $-(CH_2)_4-$, $-CH=CH-(CH_2)_2-$ ou $-(CH_2)_2-CH=CH-$ ; et
a, b et c sont indépendamment 0 ou 1 et une somme de a, b et c est de 0, 1 ou 2.

**2.** Composé selon la revendication 1, dans lequel, dans la formule (1), $R^1$ et $R^2$ sont indépendamment un alkyle ayant 1 à 10 atomes de carbone, un alcényle ayant 2 à 10 atomes de carbone, un alkyle ayant 1 à 10 atomes de carbone dans lequel au moins un hydrogène est remplacé par du fluor ou un alcényle ayant 2 à 10 atomes de carbone dans lequel au moins un hydrogène est remplacé par du fluor, et $Z^1$, $Z^2$ et $Z^3$ sont indépendamment une liaison simple, $-(CH_2)_2-$, -CH=CH-, $-(CH_2)_4-$, $-CH=CH-(CH_2)_2-$ou $-(CH_2)_2-CH=CH-$.

**3.** Composé selon la revendication 1, représenté par l'une quelconque des formules (1-1) à (1-4) :

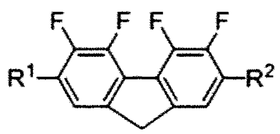
**(1-1)**

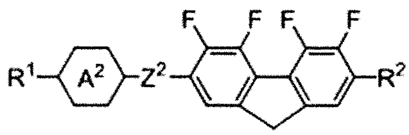
**(1-2)**

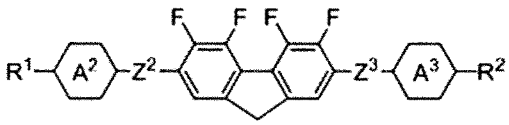
**(1-3)**

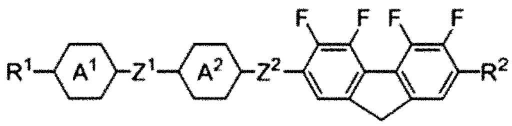
**(1-4)**

dans lequel, dans les formules (1-1) à (1-4),

$R^1$ et $R^2$ sont indépendamment un alkyle ayant 1 à 10 atomes de carbone, un alcényle ayant 2 à 10 atomes de carbone ou un alkyle ayant 1 à 10 atomes de carbone dans lequel au moins un hydrogène est remplacé par du fluor,
le noyau $A^1$, le noyau $A^2$ et le noyau $A^3$ sont indépendamment un 1,4-cyclohexylène, un 1,4-cyclohéxénylène, un 1,4-phénylène, un 1,4-phénylène dans lequel au moins un hydrogène est remplacé par du fluor ou un tétrahydropyran-2,5-diyle ;
$Z^1$ est une liaison simple, $-(CH_2)_2-$ ou -CH=CH- ;
$Z^2$ est une liaison simple, $-(CH_2)_2-$, $-(CH_2)_4-$ ou $-CH=CH-(CH_2)_2-$ ; et
$Z^3$ est une liaison simple, $-(CH_2)_2-$, $-(CH_2)_4-$ ou $-(CH_2)_2-CH=CH-$.

**4.** Composé selon la revendication 1, représenté par l'une quelconque des formules (1-5) à (1-12) :

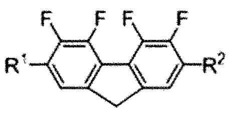
(1-5)

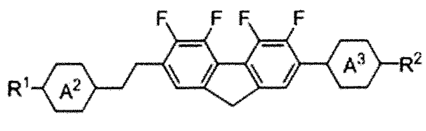
(1-9)

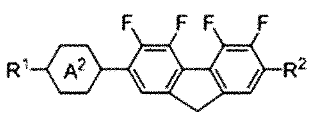
(1-6)

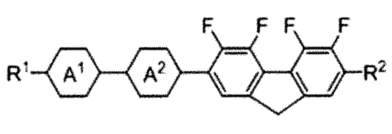
(1-10)

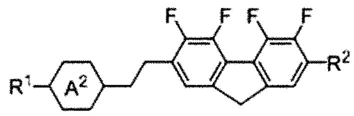 (1-7) 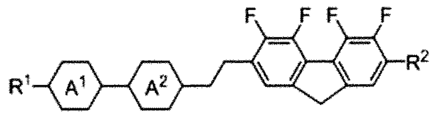 (1-11)

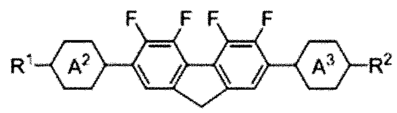 (1-8) 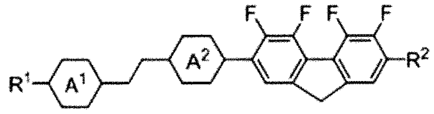 (1-12)

dans lequel, dans les formules (1-5) à (1-12),

$R^1$ et $R^2$ sont indépendamment un alkyle ayant 1 à 10 atomes de carbone ou un alcényle ayant 2 à 10 atomes de carbone ; et
le noyau $A^1$, le noyau $A^2$ et le noyau $A^3$ sont indépendamment un 1,4-cyclohexylène, un 1,4-cyclohexénylène, un 1,4-phénylène, un 1,4-phénylène dans lequel au moins un hydrogène est remplacé par du fluor ou un tétrahydropyran-2,5-diyle.

**5.** Composé selon la revendication 1, représenté par l'une quelconque des formules (1-13) à (1-22) :

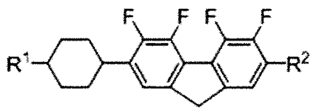 (1-13) 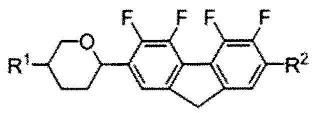 (1-18)

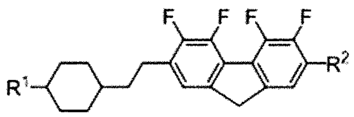 (1-14) 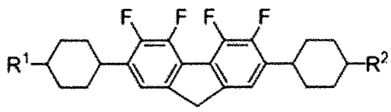 (1-19)

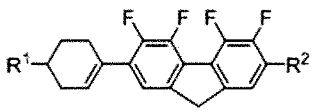 (1-15) 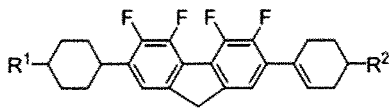 (1-20)

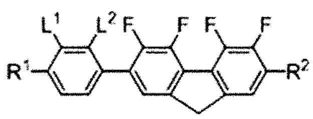 (1-16) 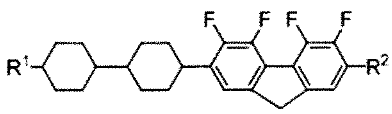 (1-21)

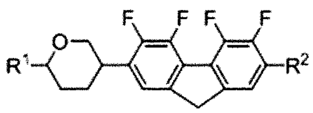 (1-17) 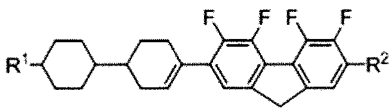 (1-22)

dans lequel, dans les formules (1-13) à (1-22), $R^1$ et $R^2$ sont indépendamment un alkyle ayant 1 à 10 atomes de carbone ou un alcényle ayant 2 à 10 atomes de carbone ; et $L^1$ et $L^2$ sont indépendamment de l'hydrogène ou du fluor.

**6.** Composé selon la revendication 1, représenté par l'une quelconque des formules (1-23) à (1-25) :

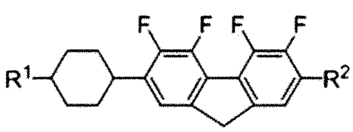 (1-23)

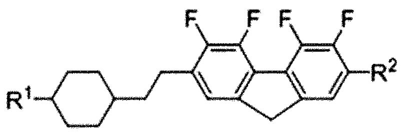

(1-24)

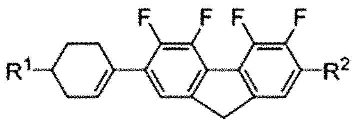

(1-25)

dans lequel, dans les formules (1-23) à (1-25), $R^1$ et $R^2$ sont indépendamment un alkyle ayant 1 à 7 atomes de carbone ou un alcényle ayant 2 à 7 atomes de carbone.

**7.** Composition de cristal liquide contenant au moins l'un des composés décrits dans la revendication 1.

**8.** Composition de cristal liquide selon la revendication 7, contenant en outre au moins un composé choisi dans le groupe de composés représentés par les formules (2) à (4) :

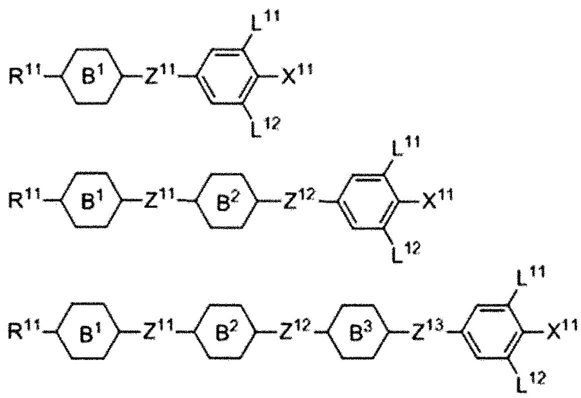

(2)

(3)

(4)

dans laquelle, dans les formules (2) à (4),

$R^{11}$ est un alkyle ayant 1 à 10 atomes de carbone ou un alcényle ayant 2 à 10 atomes de carbone et, dans les groupements alkyle et alcényle, au moins un hydrogène peut être remplacé par du fluor et au moins un $-CH_2-$ peut être remplacé par -O- ;
$X^{11}$ est du fluor, du chlore, $-OCF_3$, $-OCHF_2$, $-CF_3$, $-CHF_2$, $-CH_2F$, $-OCF_2CHF_2$ ou $-OCF_2CHFCF_3$;
le noyau $B^1$, le noyau $B^2$ et le noyau $B^3$ sont indépendamment un 1,4-cyclohexylène, un 1,4-phénylène, un 2-fluoro-1,4-phénylène, un 2,5-difluoro-1,4-phénylène, un tétrahydropyran-2,5-diyle, un 1,3-dioxane-2,5-diyle ou un pyrimidine-2,5-diyle ; et
$Z^{11}$, $Z^{12}$ et $Z^{13}$ sont indépendamment une liaison simple, $-CH_2CH_2-$, -CH=CH-, -C≡C-, -COO-, $-CF_2O-$, $-OCF_2-$, $-CH_2O-$ ou $-(CH_2)_4-$ ; et
$L^{11}$ et $L^{12}$ sont indépendamment de l'hydrogène ou du fluor.

**9.** Composition de cristal liquide selon la revendication 7, contenant en outre au moins un composé choisi dans le groupe de composés représentés par la formule (5) :

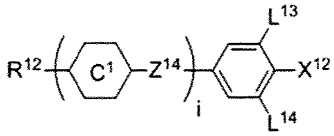

(5)

dans laquelle, dans la formule (5),

$R^{12}$ est un alkyle ayant 1 à 10 atomes de carbone ou un alcényle ayant 2 à 10 atomes de carbone et, dans les

groupements alkyle et alcényle, au moins un hydrogène peut être remplacé par du fluor et au moins un $-CH_2-$ peut être remplacé par -O- ;
$X^{12}$ est -C≡N ou -C≡C-C≡N ;
le noyau $C^1$ est un 1,4-cyclohexylène, un 1,4-phénylène, un 2-fluoro-1,4-phénylène, un 2,5-difluoro-1,4-phénylène, un tétrahydropyran-2,5-diyle, un 1,3-dioxane-2,5-diyle ou un pyrimidine-2,5-diyle ;
$Z^{14}$ est une liaison simple, $-CH_2CH_2-$, -C≡C-, -COO-, $-CF_2O-$, $-OCF_2-$ ou $-CH_2O-$ ;
$L^{13}$ et $L^{14}$ sont indépendamment de l'hydrogène ou du fluor ; et
i est 1, 2, 3 ou 4.

**10.** Composition de cristal liquide selon la revendication 7, contenant en outre au moins un composé choisi dans le groupe de composés représentés par les formules (6) à (12):

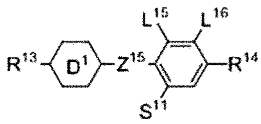
(6)

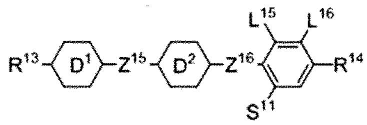
(7)

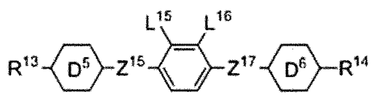
(8)

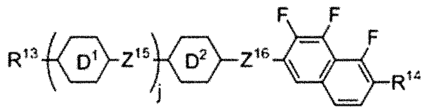
(9)

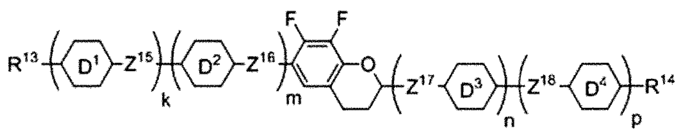
(10)

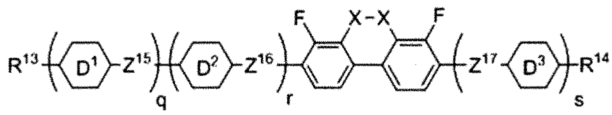
(11)

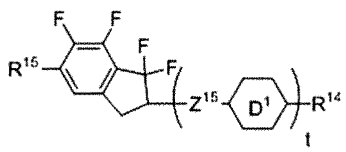
(12)

dans laquelle, dans les formules (6) à (12),

$R^{13}$ et $R^{14}$ sont indépendamment un alkyle ayant 1 à 10 atomes de carbone ou un alcényle ayant 2 à 10 atomes de carbone et, dans les groupements alkyle et alcényle, au moins un $-CH_2-$ peut être remplacé par -O- ;
$R^{15}$ est de l'hydrogène, du fluor, un alkyle ayant 1 à 10 atomes de carbone ou un alcényle ayant 2 à 10 atomes de carbone et, dans les groupements alkyle et alcényle, au moins un $-CH_2-$ peut être remplacé par -O- et au moins un hydrogène peut être remplacé par du fluor ;
$S^{11}$ est de l'hydrogène ou un méthyle ;
X est $-CF_2-$, -O- ou -CHF-;
le noyau $D^1$, le noyau $D^2$, le noyau $D^3$ et le noyau $D^4$ sont indépendamment un 1,4-cyclohexylène, un 1,4-cyclohexénylène, un 1,4-phénylène dans lequel au moins un hydrogène peut être remplacé par du fluor, un tétrahydropyan-2,5-diyle ou un décahydronaphtalène-2,6-diyle ;
le noyau $D^5$ et le noyau $D^6$ sont indépendamment un 1,4-cyclohexylène, un 1,4-cyclohexénylène, un 1,4-phénylène, un tétrahydropiran-2,5-diyle ou un décahydronaphtalène-2,6-diyle ;
$Z^{15}$, $Z^{16}$, $Z^{17}$ et $Z^{18}$ sont indépendamment une liaison simple, $-CH_2CH_2-$, -COO-, $-CH_2O-$, $OCF_2-$ ou $-OCF_2CH_2CH_2-$ ;
$L^{15}$ et $L^{16}$ sont indépendamment du fluor ou du chlore ; et
j, k, m, n, p, q, r et s sont indépendamment 0 ou 1, une somme de k, m, n et p est de 1 ou 2, une somme de q, r et s est de 0, 1, 2 ou 3 et t est 1, 2 ou 3.

**11.** Composition de cristal liquide selon la revendication 7, contenant en outre un composé choisi dans le groupe de composés représentés par les formules (13) à (15) :

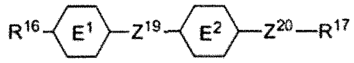
(13)

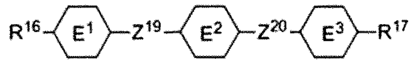
(14)

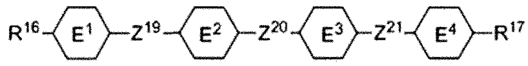
(15)

dans laquelle, dans les formules (13) à (15),

$R^{16}$ et $R^{17}$ sont indépendamment un alkyle ayant 1 à 10 atomes de carbone et un alcényle ayant 2 à 10 atomes de carbone et, dans les groupements alkyle ou alcényle, au moins un $-CH_2-$ peut être remplacé par -O- et au moins un hydrogène peut être remplacé par du fluor ;
le noyau $E^1$, le noyau $E^2$, le noyau $E^3$ et le noyau $E^4$ sont indépendamment un 1,4-cyclohexylène, un 1,4-phénylène, un 2-fluoro-1,4-phénylène, un 2,5-difluoro-1,4-phénylène ou un pyrimidine-2,5-diyle ; et
$Z^{19}$, $Z^{20}$ et $Z^{21}$ sont indépendamment une liaison simple, $-CH_2CH_2-$, -CH=CH, -C≡C- ou -COO-.

**12.** Composition de cristal liquide selon la revendication 7, contenant en outre au moins l'un d'un composé polymérisable, d'un composé optiquement actif, d'un antioxydant, d'un absorbeur de lumière ultraviolette, d'un stabilisateur à la lumière, d'un stabilisateur à la chaleur et d'un agent antimousse.

**13.** Dispositif d'affichage de cristal liquide comprenant la composition de cristal liquide selon la revendication 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H10236992 B
- WO 02055463 A
- JP 2000297051 A
- JP 2000178211 A
- EP 1223210 A
- JP H10236992 A
- WO 9110936 A
- US 5576867 B


### Non-patent literature cited in the description


- *Mol. Cryst. Liq. Cryst.,* 1985, vol. 131, 109
- *Mol. Cryst. Liq. Cryst.,* 1985, vol. 131, 327
- Organic Syntheses. John Wiley & Sons, Inc,
- Organic Reactions. John Wiley & Sons, Inc,
- Comprehensive Organic Synthesis. Pergamon Press
- New Experimental Chemistry Course (Shin Jikken Kagaku Koza in Japanese). Maruzen Co., Ltd,
- **K. OHMURO ; S. KATAOKA ; T. SASAKI ; Y. KOIKE.** *SID '97 Digest of Technical Papers,* 1997, vol. 28, 845
- **M. IMAI et al.** *Molecular Crystals and Liquid Crystals,* 1995, vol. 259, 37